# EUROPÄISCHE PATENTSCHRIFT

(11) **EP 1 963 278 B1**
(45) Veröffentlichungstag und Bekanntmachung des Hinweises auf die Patenterteilung: **18.11.2009**
(21) Anmeldenummer: 06841028.1
(22) Anmeldetag: 19.12.2006
(51) Int. Cl.: C07D 233/28, A61K 31/4164, A61P 25/04

(54) **SUBSTITUIERTE IMIDAZOLIN-DERIVATE**
SUBSTITUTED IMIDAZOLINE DERIVATIVES
DERIVES IMIDAZOLINE SUBSTITUES

(30) Priorität: 22.12.2005 DE 102005061430
(43) Veröffentlichungstag der Anmeldung: 03.09.2008
(73) Patentinhaber: Grünenthal GmbH, 52078 Aachen (DE)
(72) Erfinder: MERLA, Beatrix, 52078 Aachen (DE); OBERBÖRSCH, Stefan, 52074 Aachen (DE); SUNDERMANN, Bernd, 61381 Friedrichsdorf (DE); ENGLBERGER, Werner, 52223 Stolberg (DE); HENNIES, Hagen-Heinrich, 52152 Simmerath (DE); GRAUBAUM, Heinz, 12557 Berlin (DE)
(86) Internationale Anmeldenummer: PCT/EP2006/012221
(87) Internationale Veröffentlichungsnummer: WO 2007/079927

(56) Entgegenhaltungen:
- WO-A-01/49654
- WO-A-99/09829
- WO-A-99/09979
- WO-A-03/101969

## Beschreibung

Die vorliegende Erfindung betrifft substituierte lmidazolin-Derivate, Verfahren zu deren Herstellung, Arzneimittel enthaltend diese Verbindungen und die Verwendung von substituierten Imidazolin-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

In Org. Lett. 2003, 5, 3759-3762 werden ebenfalls mit einer Carbonsäure/Carbonsäureesterfunktion substituierte Imidazoline offenbart, die jedoch nicht mit einem aminomethyl-substituierten Cyclohexanring verknüpft sind.

Eine der Erfindung zugrundeliegende Aufgabe bestand darin, neue analgetisch wirksame Substanzen zur Verfügung zu stellen, die sich zur Schmerztherapie - insbesondere auch chronischer und neuropathischer Schmerzen - eignen.

Gegenstand der Erfindung sind daher substituierte Imidazolin-Derivate der allgemeinen Formel I, 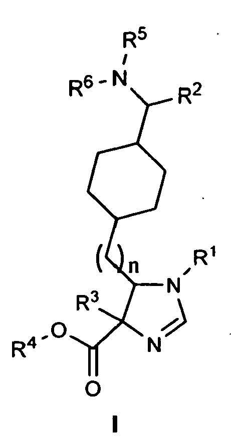 , worin
n 0, 1 oder 2 bedeutet;
R¹ C₁₋₈-Alkyl, gesättigt oder ungesättigt, verzweigt oder unverzweigt, unsubstituiert oder einfach oder mehrfach substituiert; C₃₋₈-Cycloalkyl, gesättigt oder ungesättigt, unsubstituiert oder einfach oder mehrfach substituiert; einen über eine C₁₋₃-Alkylkette gebundenen Aryl- oder Heteroarylrest, jeweils unsubstituiert oder einfach oder mehrfach substituiert, bedeutet;
R² Aryl oder Heteroaryl, jeweils unsubstituiert oder einfach oder mehrfach substituiert; einen über eine C₁₋₃-Alkylkette gebundenen Arylrest, jeweils unsubstituiert oder einfach oder mehrfach substituiert, bedeutet;
R³ C₁₋₈-Alkyl, gesättigt oder ungesättigt, verzweigt oder unverzweigt, unsubstituiert oder einfach oder mehrfach substituiert; einen über eine C₁₋₃-Alkylkette gebundenen Arylrest, jeweils unsubstituiert oder einfach oder mehrfach substituiert, bedeutet;
R⁴ H, C₁₋₄-Alkyl oder einen über eine C₁₋₃-Alkylkette gebundenen Arylrest bedeutet;
R⁵ und R⁶ unabhängig voneinander H; C₁₋₆-Alkyl, jeweils gesättigt oder ungesättigt, verzweigt oder unverzweigt, bedeutet, wobei R⁵ und R⁶ nicht gleichzeitig H bedeuten,
oder die Reste R⁵ und R⁶ zusammen CH₂CH₂OCH₂CH₂, oder (CH₂)₃₋₆ bedeuten,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Verbindungen weisen eine Affinität zum µ-Opioid-Rezeptor auf.

Die Ausdrücke "C₁₋₈-Alkyl", "C₁₋₃-Alkyl", "C₁₋₄-Alkyl" und "C₁₋₆-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 8 bzw. 1 bis 3 C-Atomen bzw. 1 bis 4 C-Atomen bzw. 1-6 C-Atomen, d.h. C₁₋₈-Alkanyle, C₂₋₈-Alkenyle und C₂₋₈-Alkinyle bzw. C₁₋₃-Alkanyle, C₂₋₃-Alkenyle und C₂₋₃-Alkinyle bzw C₁₋₄-Alkanyle, C₂₋₄-Alkenyle und C₂₋₄-Alkinyle bzw. C₁₋₆-Alkanyle, C₂₋₆-Alkenyle und C₂₋₆-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-hexyl, n-Heptyl, n-Octyl, 1,1,3,3-Tetramethylbutyl; Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl, Hexinyl, Heptenyl, Heptinyl, Octenyl und Octinyl umfasst. Besonders vorteihaft sind Methyl, Ethyl, n-Propyl, n-Butyl, sec-Butyl, iso-Butyl.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können, oder Cycloalkyle, in denen ein oder zwei Kohlenstoffatome durch ein Heteroatom S, N oder O. ersetzt sind (Heterocyclen). Vorteilhaft ist C₃₋₈-Cycloalkyl aus der Gruppe, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält, aber auch Tetrahydropyranyl, Dioxanyl, Dioxolanyl, Morpholinyl, Piperidinyl, Piperazinyl, Pyrazolinonyl und Pyrrolidinyl.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält. Besonders vorteilhaft ist der Phenyl-Rest.

Der Ausdruch "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl, Furyl, Thienyl und Indolyl.

Der Ausdruck "über C₁₋₃-Alkyl gebundenes Aryl oder Heteroaryl" bedeuten für die Zwecke der vorliegenden Erfindung, daß C₁₋₃-Alkyl und Aryl bzw. Heteroaryl die oben definierten Bedeutungen haben und der Aryl- bzw. Heteroaryl-Rest über eine C₁₋₃-Alkyl-Gruppe an die Verbindung der allgemeinen Struktur I gebunden ist. Besonders vorteilhaft im Sinne dieser Erfindung sind Benzyl und Phenethyl.

Im Zusammenhang mit "Alkyl" oder "Cycloalkyl" versteht man unter dem Begriff "substituiert" im Sinne dieser Erfindung die Substitution eines Wasserstoffrestes durch F, Cl, Br, I, -CN, NH₂, NH-C₁₋₆-Alkyl, NH-C₁₋₆-Alkyl-OH, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, C₁₋₆-Alkyl, Benzyl, O-Benzyl, C(=O)C₁₋₆-Alkyl, CO₂H, CO₂-C₁₋₆-Alkyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Besonders bevorzugt für die Zwecke der vorliegenden Erfindung bedeutet "einfach oder mehrfach substituiert" im Zusammenhang mit Alkyl S-CH₃, S-Benzyl oder COOCH₃.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" und "Heteroaryl" sind dabei bevorzugte Substituenten -F, -Cl, -CF₃, -O-CH₃, Methyl, Ethyl, n-Propyl, Nitro, tert.-Butyl, und -CN. Besonders bevorzugt sind-F und -Cl.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure und/oder Asparaginsäure. Besonders bevorzugt ist die Salzsäure.

Unter dem Begriff (CH₂)₃₋₆ bzw (CH₂)₄-₅ ist -CH₂-CH₂-CH₂-, -CH₂-CH₂-CH₂-CH₂-, - CH₂-CH₂-CH₂-CH₂CH₂- und CH₂-CH₂-CH₂-CH₂-CH₂-CH₂- bzw. -CH₂-CH₂-CH₂-CH₂- und -CH₂-CH₂-CH₂-CH₂-CH₂-zu verstehen.

Bevorzugt im Sinne dieser Erfindung sind substituierte Imidazolin-Derivate, worin

R¹ C₁₋₈-Alkyl, verzweigt oder unverzweigt, gesättigt oder ungesättigt, unsubstituiert oder einfach oder mehrfach substituiert mit F, Cl, -CN, NH-C₁₋₆-Alkyl, NH-C₁₋₆-Alkyl-OH, N(C₁₋₆-Alkyl)₂, N(C₁₋₆-Alkyl-OH)₂, NO₂, SH, S-C₁₋₆-Alkyl, S-Benzyl, OH, O-C₁₋₆-Alkyl-OH, O-C₁₋₆-Alkyl, O-Benzyl, Benzyl, C(=O)C₁₋₆-Alkyl, CO₂H, CO₂-C₁₋₆-Alkyl; C₃₋₈-Cycloalkyl, jeweils unsubstituiert oder einfach oder mehrfach substituiert mit F, Cl,-CN, NH-C₁₋₆-Alkyl. NH-C₁₋₆-Alkyl-OH, N(C₁₋₆-Alkyl)₂, N(C₁₋₆-Alkyl-OH)₂, NO₂. SH, S-C₁₋₆-Alkyl, S-Benzyl, Benzyl, C₁₋₆-Alkyl, OH, O-C₁₋₆-Alkyl-OH, O-Benzyl, C(=O)C₁₋₆-Alkyl, CO₂H, CO₂-C₁₋₆-Alkyl; einen über eine C₁₋₃-Alkylkette gebundenen Phenyl-, Furyl-, Thienyl- oder Indolylrest, jeweils unsubstituiert oder einfach oder mehrfach substituiert mit F, Cl, CN, NH-C₁₋₆-Alkyl, NH-C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl; bedeutet;
vorzugsweise
R¹ C₁₋₈-Alkyl, verzweigt oder unverzweigt, unsubstituiert oder einfach oder mehrfach substituiert mit F, Cl, -CN, SH, S-C₁₋₆-Alkyl, S-Benzyl, OH, O-Benzyl, O-C₁₋₆-Alkyl, CO₂H, CO₂-C₁₋₆-Alkyl; C₃₋₈-Cycloalkyl, jeweils unsubstituiert oder einfach oder mehrfach substituiert mit F, Cl, -CN, SH, S-C₁₋₆-Alkyl, C₁₋₆-Alkyl, S-Benzyl, OH, O-Benzyl, O-C₁₋₆-Alkyl, CO₂H, CO₂-C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; bedeutet,
insbesondere
R¹ C₁₋₈-Alkyl, verzweigt oder unverzweigt; C₃₋₈-Cycloalkyl; einen über eine C₁₋₃-Alkylkette gebundenen Phenylrest, bedeutet.
Besonders bevorzugt sind Imidazolin-Derivate, bei denen R¹ Cyclohexyl, n-Propyl, n-Butyl, Phenethyl oder Benzyl bedeutet.
Bevorzugt im Sinne dieser Erfindung sind auch substituierte Imidazolin-Derivate, worin
R² Phenyl, Thienyl oder Pyridyl, jeweils unsubstituiert oder einfach oder mehrfach substituiert mit F, Cl, CN, NH-C₁₋₆-Alkyl, NH-C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl. O-C₁₋₆Alkyl-OH, C(=O)C₁₋₆-Alkyl, CO₂H, CO₂-C₁₋₆-Alkyl, CF₃, C₁₋₆-Alkyl; einen über eine C₁₋₃-Alkylkette gebundenen Arylrest, jeweils unsubstituiert oder einfach oder mehrfach substituiert mit F, Cl, CN, NH-C₁₋₆-Alkyl, NH-C₁₋₆-Alkyl-OH, N(C₁₋₆-Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl, bedeutet;
vorzugsweise
R² Phenyl oder Thienyl, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl, bedeutet;
insbesondere
R² Phenyl, unsubstituiert oder einfach oder mehrfach substituiert mit F, Cl, OH, OCH₃, CF₃ oder CH₃; Thienyl; oder einen über eine C₁₋₃-Alkylkette gebundenen Phenylrest, unsubstituiert oder einfach oder mehrfach substituiert mit F, Cl, CN, OH, OCH₃, CF₃ oder CH₃; bedeutet.

Ganz besonders bevorzugt sind Imidazolin Derivate, worin R² Phenyl, unsubstituiert oder einfach substituiert mit Cl oder F, Thienyl oder Phenethyl bedeutet.

Weiterhin bevorzugt im Sinne dieser Erfindung sind substituierte Imidazolin-Derivate, worin
R³ C₁₋₈-Alkyl, gesättigt oder ungesättigt, verzweigt oder unverzweigt, unsubstituiert oder einfach oder mehrfach substituiert mit F, Cl, -CN, SH, S-C₁₋₆-Alkyl, S-Benzyl, Benzyl, OH, O-Benzyl, O-C₁₋₆-Alkyl, CO₂H, CO₂-C₁₋₆-Alkyl; einen über eine C₁₋₃-Alkylkette gebundenen Phenylrest, jeweils unsubstituiert oder einfach oder mehrfach substituiert mit F, Cl, CN, NH-C₁₋₆-Alkyl, NH-C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl, bedeutet,
vorzugsweise
R³ C₁₋₈-Alkyl, verzweigt oder unverzweigt, unsubstituiert oder einfach oder mehrfach substituiert mit F, Cl, -CN, SH, S-C₁₋₆-Alkyl, S-Benzyl, OH, O-Benzyl, O-C₁₋₆-Alkyl, CO₂H, CO₂-C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; bedeutet,
insbesondere
R³ C₁₋₈-Alkyl, verzweigt oder unverzweigt, unsubstituiert oder einfach oder mehrfach substituiert mit S-CH₃, S-Benzyl oder COOCH₃; einen über eine C₁₋₃-Alkylkette gebundenen Phenylrest, jeweils unsubstituiert oder einfach oder mehrfach substituiert mit F, Cl, CN, OH, O-CH3, CF₃, CH₃; bedeutet.

Besonders bevorzugt sind Imidazolin-Derivate, worin R³ sec-Butyl, iso-Butyl, n-Butyl, n-Propyl, CH₃, CH₂CH₂COOCH₃, CH₂-S-Benzyl, CH₂CH₂-S-CH₃, CH₂-S-CH₃ oder 4-CI-Benzyl bedeutet.

Bevorzugt im Sinne dieser Erfindung sind auch Imidazolin-Derivate, worin R⁴ C₁₋₄-Alkyl bedeutet.

Besonders bevorzugt sind Imidazolin-Derivate, worin R⁴ CH₃ bedeutet.

Außerdem bevorzugt im Sinne dieser Erfindung sind Imidazolin-Derivate, worin R⁵ und R⁶ unabhängig voneinander H oder C₁₋₆-Alkyl bedeuten, wobei R⁵ und R⁶ nicht gleichzeitig H bedeuten.

Weiterhin bevorzugt sind Imidazolin-Derivate, worin die Reste R⁵ und R⁶ zusammen CH₂CH₂OCH₂CH₂, oder (CH₂)₄₋₅ bedeuten,

Es ist bevorzugt, dass n für 0 oder 1 steht.

Besonders bevorzugt sind Imidazolin-Derivate, worin R⁵ und R⁶CH₃ bedeuten.

Ganz besonders bevorzugt sind substituierte Imidazolin-Derivate aus der Gruppe
1-Cyclohexyl-5-{4-[dimethylamino-(3-fluor-phenyl)-methyl]-cyclohexyl}-4-isobutyl-4,5-dihydro-1 H-imidazol-4-carbonsäuremethylester
4-(4-Chlor-benzyl)-1-cyclohexyl-5-{4-[dimethylamino-(3-fluor-phenyl)-methyl]-cyclohexyl}-4,5-dihydro-1 H-imidazol-4-carbonsäuremethylester
4-Butyl-1-cyclohexyl-5-{4-[dimethylamino-(3-fluor-phenyl)-methyl]-cyclohexyl}-4,5-dihydro-1 H-imidazol-4-carbonsäuremethylester
5-{4-[(4-Chlor-phenyl)-dimethylamino-methyl]-cyclohexyl}-1-cyclohexyl-4-isobutyl-4,5-dihydro-1 H-imidazol-4- carbonsäuremethylester
4-(4-Chlor-benzyl)-5-{4-[(4-chlor-phenyl)-dimethylamino-methyl]-cyclohexyl}-1-cyclohexyl-4,5-dihydro-1 H-imidazol-4-carbonsäuremethylester
4-Butyl-5-(4-[(4-chlor-phenyl)-dimethylamino-methyl]-cyclohexyl)-1-cyclohexyl-4,5-dihydro-1H-imidazol-4-carboxylic carbonsäuremethylester
5-{4-[(4-Chlor-phenyl)-dimethylamino-methyl]-cyclohexylmethyl}-1-cyclohexyl-4-isobutyl-4,5-dihydro-1 H-imidazol-4-carbonsäuremethylester
4-(4-Chlor-benzyl)-5-{4-[(4-chlor-phenyl)-dimethylamino-methyl]-cyclohexylmethyl}-1-cyclohexyl-4,5-dihydro-1 H-imidazol-4-carbonsäuremethylester
4-Butyl-5-{4-[(4-chlor-phenyl)-dimethylamino-methyl]-cyclohexylmethyl}-1-cyclohexyl-4,5-dihydro-1 H-imidazol-4-carbonsäuremethylester
4-(4-Chlor-benzyl)-1-cyclohexyl-5-[4-(dimethylamino-thiophen-2-yl-methyl)-cyclohexylmethyl]-4,5-dihydro-1 H-imidazol-4-carbonsäuremethylester
5-{4-[Dimethylamino-(3-fluor-phenyl)-methyl]-cyclohexyl}-4-isobutyl-1-propyl-4,5-dihydro-1 H-imidazol-4-carbonsäuremethylester
4-(4-Chlor-benzyl)-5-{4-[dimethylamino-(3-fluor-phenyl)-methyl]-cyclohexyl}-1-propyl-4,5-dihydro-1 H-imidazol-4carbonsäuremethylester
4-Butyl-5-{4-[dimethylamino-(3-fluor-phenyl)-methyl]-cyclohexyl}-1-propyl-4,5-dihydro-1 H-imidazol-4-carbonsäuremethylester
5-{4-[(4-Chlor-phenyl)-dimethylamino-methyl]-cyclohexyl}-4-isobutyl-1-propyl-4,5-dihydro-1 H-imidazol-4-carbonsäuremethylester
4-(4-Chlor-benzyl)-5-{4-[(4-chlor-phenyl)-dimethylamino-methyl]-cyclohexyl}-1-propyl-4,5-dihydro-1 H-imidazol-4-carbonsäuremethylester
5-{4-[(4-Chlor-phenyl)-dimethylamino-methyl]-cyclohexylmethyl}-4-isobutyl-1-propyl-4,5-dihydro-1 H-imidazol-4-carbonsäuremethylester
4-(4-Chlor-benzyl)-5-{4-[(4-chlor-phenyl)-dimethylamino-methyl]-cyclohexylmethyl}-1-propyl-4,5-dihydro-1 H-imidazol-4-carbonsäuremethylester
4-Butyl-5-{4-[(4-chlor-phenyl)-dimethylamino-methyl]-cyclohexylmethyl}-1-propyl-4,5-dihydro-1 H-imidazol-4-carbonsäuremethylester
1-Butyl-5-{4-[dimethylamino-(3-fluor-phenyl)-methyl]-cyclohexyl}-4-isobutyl-4,5-dihydro-1 H-imidazol-4-carbonsäuremethylester
1-Butyl-4-(4-chlor-benzyl)-5-{4-[dimethylamino-(3-fluor-phenyl)-methyl]-cyclohexyl}-4,5-dihydro-1H-imidazol-4-carbonsäuremethylester
1-Butyl-5-{4-[(4-chlor-phenyl)-dimethylamino-methyl]-cyclohexyl}-4-isobutyl-4,5-dihydro-1H-imidazol-4-carbonsäuremethylester
1-Butyl-4-(4-chlor-benzyl)-5-{4-[(4-chlor-phenyl)-dimethylamino-methyl]-cyclohexyl}-4,5-dihydro-1H-imidazol-4-carbonsäuremethylester
1,4-Dibutyl-5-{4-[(4-chlor-phenyl)-dimethylamino-methyl]-cyclohexyl}-4,5-dihydro-1 H-imidazol-4-carbonsäuremethylester
1-Butyl-5-{4-[(4-chlor-phenyl)-dimethylamino-methyl]-cyclohexylmethyl}-4-isobutyl-4,5-dihydro-1 H-imidazol-4-carbonsäuremethylester
1-Butyl-4-(4-chlor-benzyl)-5-{4-[(4-chlor-phenyl)-dimethylamino-methyl]-cyclohexylmethyl}-4,5-dihydro-1H-imidazol-4-carbonsäuremethylester
1,4-Dibutyl-5-{4-[(4-chlor-phenyl)-dimethylamino-methyl]-cyclohexylmethyl}-4,5-dihydro-1H-imidazol-4-carbonsäuremethylester
5-{4-[Dimethylamino-(3-fluor-phenyl)-methyl]-cyclohexyl}-4-isobutyl-1-phenethyl-4,5-dihydro-1H-imidazol-4-carbonsäuremethylester
4-(4-Chlor-benzyl)-5-{4-[dimethylamino-(3-fluor-phenyl)-methyl]-cyclohexyl}-1-phenethyl-4,5-dihydro-1H-imidazol-4-carbonsäuremethylester
4-Butyl-5-{4-[dimethylamino-(3-fluor-phenyl)-methyl]-cyclohexyl}-1-phenethyl-4,5-dihydro-1H-imidazol-4-carbonsäuremethylester
5-{4-[(4-Chlor-phenyl)-dimethylamino-methyl]-cyclohexyl}-4-isobutyl-1-phenethyl-4,5-dihydro-1H-imidazol-4-carbonsäuremethylester
4-(4-Chlor-benzyl)-5-{4-[(4-chlor-phenyl)-dimethylamino-methyl]-cyclohexyl}-1-phenethyl-4,5-dihydro-1 H-imidazol-4-carbonsäuremethylester
4-Butyl-5-{4-[(4-chlor-phenyl)-dimethylamino-methyl]-cyclohexyl}-1-phenethyl-4,5-dihydro-1H-imidazol-4-carbonsäuremethylester
5-{4-[(4-Chlor-phenyl)-dimethylamino-methyl]-cyclohexylmethyl}-4-isobutyl-1-phenethyl-4,5-dihydro-1H-imidazol-4carbonsäuremethylester
4-(4-Chlor-benzyl)-5-{4-[(4-chlor-phenyl)-dimethylamino-methyl]-cyclohexylmethyl}-1-phenethyl-4,5-dihydro-1H-imidazol-4-carbonsäuremethylester
4-Butyl-5-{4-[(4-chlor-phenyl)-dimethylamino-methyl]-cyclohexylmethyl}-1-phenethyl-4,5-dihydro-1 H-imidazol-4-carbonsäuremethylester
1-Benzyl-5-{4-[dimethylamino-(3-fluor-phenyl)-methyl]-cyclohexyl}-4-isobutyl-4,5-dihydro-1H-imidazol-4-carbonsäuremethylester
1-Benzyl-4-(4-chlor-benzyl)-5-{4-[dimethylamino-(3-fluor-phenyl)-methyl]-cyclohexyl}-4,5-dihydro-1 H-imidazol-4-carbonsäuremethylester
1-Benzyl-5-{4-[(4-chlor-phenyl)-dimethylamino-methyl]-cyclohexyl}-4-isobutyl-4,5-dihydro-1 H-imidazol-4-carbonsäuremethylester
1-Benzyl-4-(4-chlor-benzyl)-5-{4-[(4-chlor-phenyl)-dimethylamino-methyl]-cyclohexyl}-4,5-dihydro-1 H-imidazol-4-carbonsäuremethylester
1-Benzyl-5-{4-[(4-chlor-phenyl)-dimethylamino-methyl]-cyclohexylmethyl}-4-isobutyl-4,5-dihydro-1H-imidazol-4-carbonsäuremethylester
5-{4-[(4-Chlor-phenyl)-dimethylamino-methyl]-cyclohexyl}-1-cyclohexyl-4-(2-methoxycarbonyl-ethyl)-4,5-dihydro-1 H-imidazol-4-carbonsäuremethylester
4-Benzylsulfanylmethyl-5-{4-[(4-chlor-phenyl)-dimethylamino-methyl]-cyclohexyl}-1-cyclohexyl-4,5-dihydro-1 H-imidazol-4-carbonsäuremethylester
5-{4-[(4-Chlor-phenyl)-dimethylamino-methyl]-cyclohexylmethyl}-1-cyclohexyl-4-(2-methoxycarbonyl-ethyl)-4,5-dihydro-1 H-imidazol-4-carbonsäuremethylester
1-Benzyl-5-{4-[dimethylamino-(3-fluor-phenyl)-methyl]-cyclohexylmethyl}-4-propyl-4,5-dihydro-1 H-imidazol-4-carbonsäuremethylester
5-{4-[(4-Chlor-phenyl)-dimethylamino-methyl]-cyclohexylmethyl}-1-cyclohexyl-4-methylsulfanylmethyl-4,5-dihydro-1 H-imidazol-4-carbonsäuremethylester
4-Benzylsulfanylmethyl-5-{4-[(4-chlor-phenyl)-dimethylamino-methyl]-cyclohexylmethyl}-1-cyclohexyl-4,5-dihydro-1 H-imidazol-4-carbonsäuremethylester
5-{4-[Dimethylamino-(3-fluor-phenyl)-methyl]-cyclohexyl}-4-methylsulfanylmethyl-1-propyl-4,5-dihydro-1 H-imidazol-4-carbonsäuremethylester
4-Benzylsulfanylmethyl-5-{4-[dimethylamino-(3-fluor-phenyl)-methyl]-cyclohexyl}-1-propyl-4,5-dihydro-1 H-imidazol-4-carbonsäuremethylester
5-{4-[(4-Chlor-phenyl)-dimethylamino-methyl]-cyclohexyl}-4-(2-methoxycarbonylethyl)-1-propyl-4,5-dihydro-1 H-imidazol-4-carbonsäuremethylester
4-Benzylsulfanylmethyl-5-{4-[(4-chlor-phenyl)-dimethylamino-methyl]-cyclohexyl}-1-propyl-4,5-dihydro-1 H-imidazol-4-carbonsäuremethylester
5-{4-[(4-Chlor-phenyl)-dimethylamino-methyl]-cyclohexylmethyl}-4-(2-methoxycarbonyl-ethyl)-1-propyl-4,5-dihydro-1 H-imidazol-4-carbonsäuremethylester
4-sec-Butyl-5-{4-[(4-chlor-phenyl)-dimethylamino-methyl]-cyclohexylmethyl}-1-propyl-4,5-dihydro-1H-imidazol-4-carbonsäuremethylester
5-{4-[(4-Chlor-phenyl)-dimethylamino-methyl]-cyclohexylmethyl}-4-methylsulfanylmethyl-1-propyl-4,5-dihydro-1 H-imidazol-4-carbonsäuremethylester
4-Benzylsulfanylmethyl-5-{4-[(4-chlor-phenyl)-dimethylamino-methyl]-cyclohexylmethyl}-1-propyl-4,5-dihydro-1 H-imidazol-4-carbonsäuremethylester
1-Butyl-5-{4-[dimethylamino-(3-fluor-phenyl)-methyl]-cyclohexyl}-4-(2-methoxycarbonyl-ethyl)-4,5-dihydro-1 H-imidazol-4-carbonsäuremethylester
1-Butyl-5-{4-[dimethylamino-(3-fluor-phenyl)-methyl]-cyclohexyl}-4-methylsulfanylmethyl-4,5-dihydro-1 H-imidazol-4-carbonsäuremethylester
4-Benzylsulfanylmethyl-1-butyl-5-{4-[dimethylamino-(3-fluor-phenyl)-methyl]-cyclohexyl}-4,5-dihydro-1 H-imidazol-4-carbonsäuremethylester
1-Butyl-5-{4-[(4-chlor-phenyl)-dimethylamino-methyl]-cyclohexyl}-4-(2-methoxycarbonyl-ethyl)-4,5-dihydro-1 H-imidazol-4-carbonsäuremethylester
4-Benzylsulfanylmethyl-1-butyl-5-{4-[(4-chlor-phenyl)-dimethylamino-methyl]-cyclohexyl}-4,5-dihydro-1H-imidazol-4-carbonsäuremethylester
1-Butyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester
5-[4-(1-Dimethylamino-3-phenyl-propyl)-cyclohexylmethyl]-4-(2-methoxycarbonylethyl)-1-propyl-4,5-dihydro-1 H-imidazol-4-carbonsäuremethylester
1-Butyl-5-{4-[dimethylamino-(3-fluor-phenyl)-methyl]-cyclohexylmethyl}-4-(2-methoxycarbonyl-ethyl)-4,5-dihydro-1 H-imidazol-4-carbonsäuremethylester
1-Butyl-4-sec-butyl-5-{4-[dimethylamino-(3-fluor-phenyl)-methyl]-cyclohexylmethyl}-4,5-dihydro-1 H-imidazol-4-carbonsäuremethylester
1-Butyl-5-{4-[dimethylamino-(3-fluor-phenyl)-methyl]-cyclohexylmethyl}-4-methylsulfanylmethyl-4,5-dihydro-1 H-imidazol-4-carbonsäuremethylester
4-Benzylsulfanylmethyl-1-butyl-5-{4-[dimethylamino-(3-fluor-phenyl)-methyl]-cyclohexylmethyl}-4,5-dihydro-1H-imidazol-4-carbonsäuremethylester
1-Butyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
1-Butyl-5-{4-[dimethylamino-(4-fluor-phenyl)-methyl]-cyclohexylmethyl}-4-methylsulfanylmethyl-4,5-dihydro-1H-imidazol-4-carbonsäuremethylester
1-Butyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester
4-Benzylsulfanylmethyl-1-butyl-5-[4-(1-dimethylamino-3-phenyl-propyl)-cyclohexylmethyl]-4,5-dihydro-1H-imidazol-4-carbonsäuremethylester
5-{4-[Dimethylamino-(3-fluor-phenyl)-methyl]-cyclohexylmethyl}-4-(2-methoxycarbonyl-ethyl)-1-phenethyl-4,5-dihydro-1 H-imidazol-4-carbonsäuremethylester
4-sec-Butyl-5-{4-[dimethylamino-(3-fluor-phenyl)-methyl]-cyclohexylmethyl}-1-phenethyl-4,5-dihydro-1H-imidazol-4-carbonsäuremethylester
4-Benzylsulfanylmethyl-5-{4-[dimethylamino-(3-fluor-phenyl)-methyl]-cyclohexylmethyl}-1-phenethyl-4,5-dihydro-1H-imidazol-4-carbonsäuremethylester
5-{4-[Dimethylamino-(4-fluor-phenyl)-methyl]-cyclohexylmethyl}-4-(2-methoxycarbonyl-ethyl)-1-phenethyl-4,5-dihydro-1 H-imidazol-4-carbonsäuremethylester
5-[4-(1-Dimethylamino-3-phenyl-propyl)-cyclohexylmethyl]-4-(2-methoxycarbonylethyl)-1-phenethyl-4,5-dihydro-1H-imidazol-4-carbonsäuremethylester
1-Benzyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
1-Benzyl-4-benzylsulfanylmethyl-5-{4-[dimethylamino-(3-fluor-phenyl)-methyl]-cyclohexylmethyl}-4,5-dihydro-1 H-imidazol-4-carbonsäuremethylester
1-Benzyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
1-Butyl-4-sec-butyl-5-{4-[(4-chlor-phenyl)-dimethylamino-methyl]-cyclohexylmethyl}-4,5-dihydro-1 H-imidazol-4-carbonsäuremethylester
1-Butyl-5-{4-[(4-chlor-phenyl)-dimethylamino-methyl]-cyclohexylmethyl}-4-methylsulfanylmethyl-4,5-dihydro-1 H-imidazol-4-carbonsäuremethylester
4-Benzylsulfanylmethyl-1-butyl-5-{4-[(4-chtor-phenyl)-dimethylamino-methyl]-cyclohexylmethyl}-4,5-dihydro-1 H-imidazol-4-carbonsäuremethylester
5-{4-[Dimethylamino-(3-fluor-phenyl)-methyl]-cyclohexyl}-4-(2-methoxycarbonylethyl)-1-phenethyl-4,5-dihydro-1H-imidazol-4-carbonsäuremethylester
4-Benzylsulfanylmethyl-5-{4-[dimethylamino-(3-fluor-phenyl)-methyl]-cyclohexyl}-1-phenethyl-4,5-dihydro-1 H-imidazol-4-carbonsäuremethylester
4-Benzylsulfanylmethyl-5-{4-[(4-chlor-phenyl)-dimethylamino-methyl]-cyclohexyl}-1-phenethyl-4,5-dihydro-1 H-imidazol-4-carbonsäuremethylester
5-{4-[(4-Chlor-phenyl)-dimethylamino-methyl]-cyclohexylmethyl}-4-(2-methoxycarbonyl-ethyl)-1-phenethyl-4,5-dihydro-1H-imidazol-4-carbonsäuremethylester
1-Benzyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
1-Benzyl-5-{4-[dimethylamino-(3-fluor-phenyl)-methyl]-cyclohexyl}-4-methylsulfanylmethyl-4,5-dihydro-1 H-imidazol-4-carbonsäuremethylester
1-Benzyl-4-benzylsulfanylmethyl-5-{4-[dimethylamino-(3-fluor-phenyl)-methyl]-cyclohexyl}-4,5-dihydro-1H-imidazol-4-carbonsäuremethylester
1-Benzyl-4-benzylsulfanylmethyl-5-{4-[(4-chlor-phenyl)-dimethylamino-methyl]-cyclohexyl}-4,5-dihydro-1 H-imidazol-4-carbonsäuremethylester
1-Benzyl-5-{4-[(4-chlor-phenyl)-dimethylamino-methyl]-cyclohexylmethyl}-4-(2-methoxycarbonyl-ethyl)-4,5-dihydro-1 H-imidazol-4-carbonsäuremethylester
1-Benzyl-5-{4-[(4-chlor-phenyl)-dimethylamino-methyl]-cyclohexylmethyl}-4-methylsulfanylmethyl-4,5-dihydro-1 H-imidazol-4-carbonsäuremethylester
1-Benzyl-4-benzylsulfanylmethyl-5-{4-[(4-chlor-phenyl)-dimethylamino-methyl]-cyclohexylmethyl}-4,5-dihydro-1 H-imidazol-4-carbonsäuremethylester
1-Cyclohexyl-S-{4-[dimethylamino-(3-fluor-phenyl)-methyl]-cyclohexylmethyl}-4-propyl-4,5-dihydro-1 H-imidazol-4-carbonsäuremethylester
1-Cyclohexyl-5-{4-[dimethylamino-(3-fluor-phenyl)-methyl]-cyclohexylmethyl}-4-(2-methylsulfanyl-ethyl)-4,5-dihydro-1 H-imidazol-4-carbonsäuremethylester
1-Cyclohexyl-5-{4-[dimethylamino-(3-fluor-phenyl)-methyl]-cyclohexylmethyl}-4-methyl-4,5-dihydro-1H-imidazol-4-carbonsäuremethylester
1-Cyclohexyl-5-{4-[dimethylamino-(4-fluor-phenyl)-methyl]-cyclohexylmethyl}-4-propyl-4,5-dihydro-1 H-imidazol-4-carbonsäuremethylester
1-Cyclohexyl-5-{4-[dimethylamino-(4-fluor-phenyl)-methyl]-cyclohexylmethyl}-4-(2-methylsulfanyl-ethyl)-4,5-dihydro-1 H-imidazol-4-carbonsäuremethylester
1-Cyclohexyl-5-{4-[dimethylamino-(4-fluor-phenyl)-methyl]-cyclohexylmethyl}-4-methyl-4,5-dihydro-1 H-imidazol-4-carbonsäuremethylester
1-Cyclohexyl-5-[4-(1-dimethylamino-3-phenyl-propyl)-cyclohexylmethyl]-4-propyl-4,5-dihydro-1 H-imidazol-4-carbonsäuremethylester
1-Cyclohexyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
1-Cyclohexyl-5-[4-(1-dimethylamino-3-phenyl-propyl)-cyclohexylmethyl]-4-methyl-4,5-dihydro-1H-imidazol-4-carbonsäuremethylester
5-{4-[Dimethylamino-(3-fluor-phenyl)-methyl]-cyclohexylmethyl}-1,4-dipropyl-4,5-dihydro-1 H-imidazol-4-carbonsäuremethylester
5-{4-[Dimethylamino-(3-fluor-phenyl)-methyl]-cyclohexylmethyl}-4-(2-methylsulfanyl-ethyl)-1-propyl-4,5-dihydro-1H-imidazol-4-carbonsäuremethylester
1-Benzyl-4-(4-chlor-benzyl)-5-{4-[(4-chlor-phenyl)-dimethylamino-methyl]-cyclohexylmethyl}-4,5-dihydro-1 H-imidazol-4-carbonsäuremethylester
1-Benzyl-4-butyl-5-{4-[(4-chlor-phenyl)-dimethylamino-methyl]-cyclohexylmethyl}-4,5-dihydro-1 H-imidazol-4-carbonsäuremethylester
1-Cyclohexyl-5-{4-[dimethylamino-(3-fluor-phenyl)-methyl]-cyclohexyl}-4-(2-methoxycarbonyl-ethyl)-4,5-dihydro-1 H-imidazol-4-carbonsäuremethylester
4-Benzylsulfanylmethyl-1-cyclohexyl-5-{4-[dimethylamino-(3-fluor-phenyl)-methyl]-cyclohexyl}-4,5-dihydro-1H-imidazol-4-carbonsäuremethylester
5-{4-[Dimethylamino-(3-fluor-phenyl)-methyl]-cyclohexylmethyl}-4-methyl-1-propyl-4,5-dihydro-1 H-imidazol-4-carbonsäuremethylester
5-{4-[Dimethylamino-(4-fluor-phenyl)-methyl]-cyclohexylmethyl}-1,4-dipropyl-4,5-dihydro-1 H-imidazol-4-carbonsäuremethylester
5-{4-[Dimethylamino-(4-fluor-phenyl)-methyl]-cyclohexylmethyl}-4-(2-methylsulfanyl-ethyl)-1-propyl-4,5-dihydro-1H-imidazol-4-carbonsäuremethylester
5-[4-(1-Dimethylamino-3-phenyl-propyl)-cyclohexylmethyl]-1,4-dipropyl-4,5-dihydro-1 H-imidazol-4-carbonsäuremethylester
5-[4-(1-Dimethylamino-3-phenyl-propyl)-cyclohexylmethyl]-4-(2-methylsulfanyl-ethyl)-1-propyl-4,5-dihydro-1H-imidazol-4-carbonsäuremethylester
1-Butyl-5-{4-[dimethylamino-(3-fluor-phenyl)-methyl]-cyclohexylmethyl}-4-propyl-4,5-dihydro-1 H-imidazol-4-carbonsäuremethylester
1-Butyl-5-{4-[dimethylamino-(3-fluor-phenyl)-methyl]-cyclohexylmethyl}-4-(2-methylsulfanyl-ethyl)-4,5-dihydro-1 H-imidazol-4-carbonsäuremethylester
1-Butyl-5-{4-[dimethylamino-(3-fluor-phenyl)-methyl]-cyclohexylmethyl}-4-methyl-4,5-dihydro-1 H-imidazol-4-carbonsäuremethylester
1-Butyl-5-{4-[dimethylamino-(4-fluor-phenyl)-methyl]-cyclohexylmethyl}-4-propyl-4,5-dihydro-1H-imidazol-4-carbonsäuremethylester
1-Butyl-5-{4-[dimethylamino-(4-fluor-phenyl)-methyl]-cyclohexylmethyl}-4-(2-methylsulfanyl-ethyl)-4,5-dihydro-1 H-imidazol-4-carbonsäuremethylester
1-Butyl-5-[4-(1-dimethylamino-3-phenyl-propyl)-cyclohexylmethyl]-4-propyl-4,5-dihydro-1 H-imidazol-4-carbonsäuremethylester
1-Butyl-5-[4-(1-dimethylamino-3-phenyl-propyl)-cyclohexylmethyl]-4-(2-methylsulfanyl-ethyl)-4,5-dihydro-1 H-imidazol-4-carbonsäuremethylester
5-{4-[Dimethylamino-(3-fluor-phenyl)-methyl]-cyclohexylmethyl}-1-phenethyl-4-propyl-4,5-dihydro-1 H-imidazol-4-carbonsäuremethylester
5-{4-[Dimethylamino-(3-fluor-phenyl)-methyl]-cyclohexylmethyl}-4-(2-methylsulfanyl-ethyl)-1-phenethyl-4,5-dihydro-1 H-imidazol-4-carbonsäuremethylester
5-{4-[Dimethylamino-(3-fluor-phenyl)-methyl]-cyclohexylmethyl}-4-methyl-1-phenethyl-4,5-dihydro-1 H-imidazol-4-carbonsäuremethylester
5-{4-[Dimethylamino-(4-fluor-phenyl)-methyl]-cyclohexylmethyl}-1-phenethyl-4-propyl-4,5-dihydro-1 H-imidazol-4-carbonsäuremethylester
5-{4-[Dimethylamino-(4-fluor-phenyl)-methyl]-cyclohexylmethyl}-4-(2-methylsulfanyl-ethyl)-1-phenethyl-4,5-dihydro-1 H-imidazol-4-carbonsäuremethylester
5-{4-[Dimethylamino-(4-fluor-phenyl)-methyl]-cyclohexylmethyl}-4-methyl-1-phenethyl-4,5-dihydro-1H-imidazol-4-carbonsäuremethylester
5-[4-(1-Dimethylamino-3-phenyl-propyl)-cyclohexylmethyl]-1-phenethyl-4-propyl-4,5-dihydro-1 H-imidazol-4-carbonsäuremethylester
5-[4-(1-Dimethylamino-3-phenyl-propyl)-cyclohexylmethyl]-4-(2-methylsulfanyl-ethyl)-1-phenethyl-4,5-dihydro-1H-imidazol-4-carbonsäuremethylester
5-[4-(1-Dimethylamino-3-phenyl-propyl)-cyclohexylmethyl]-4-methyl-1-phenethyl-4,5-dihydro-1 H-imidazol-4-carbonsäuremethylester
1-Benzyl-5-{4-[dimethylamino-(3-fluor-phenyl)-methyl]-cyclohexylmethyl}-4-propyl-4,5-dihydro-1 H-imidazol-4-carbonsäuremethylester
1-Benzyl-5-{4-[dimethylamino-(3-fluor-phenyl)-methyl]-cyclohexylmethyl}-4-(2-methylsulfanyl-ethyl)-4,5-dihydro-1 H-imidazol-4-carbonsäuremethylester
1-Benzyl-5-{4-[dimethylamino-(3-fluor-phenyl)-methyl]-cyclohexylmethyl}-4-methyl-4,5-dihydro-1H-imidazol-4-carbonsäuremethylester
1-Benzyl-5-{4-[dimethylamino-(4-fluor-phenyl)-methyl]-cyclohexylmethyl}-4-propyl-4,5-dihydro-1 H-imidazol-4-carbonsäuremethylester
1-Benzyl-5-{4-[dimethylamino-(4-fluor-phenyl)-methyl]-cyclohexylmethyl}-4-(2-methylsulfanyl-ethyl)-4,5-dihydro-1 H-imidazol-4-carbonsäuremethylester
1-Benzyl-5-{4-[dimethylamino-(4-fluor-phenyl)-methyl]-cyclohexylmethyl}-4-methyl-4,5-dihydro-1 H-imidazol-4-carbonsäuremethylester
1-Benzyl-5-[4-(1-dimethylamino-3-phenyl-propyl)-cyclohexylmethyl]-4-propyl-4,5-dihydro-1H-imidazol-4-carbonsäuremethytester
1-Benzyl-5-[4-(1-dimethylamino-3-phenyl-propyl)-cyclohexylmethyl]-4-(2-methylsulfanyl-ethyl)-4,5-dihydro-1 H-imidazol-4-carbonsäuremethylester
1-Benzyl-5-[4-(1-dimethylamino-3-phenyl-propyl)-cyclohexylmethyl]-4-methyl-4,5-dihydro-1H-imidazol-4-carbonsäuremethylester
1-Cyclohexyl-5-{4-[dimethylamino-(3-fluor-phenyl)-methyl]-cyclohexylmethyl}-4-(2-methoxycarbonyl-ethyl)-4,5-dihydro-1 H-imidazol-4-carbonsäuremethylester
4-*sec-*Butyl-1-cyclohexyl-5-{4-[dimethylamino-(3-fluor-phenyl)-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexylmethyl}-4-methylsulfanylmethyl-4,5-dihydro-1H-imidazol-4-carbonsäuremethylester
4-Benzylsulfanylmethyl-1-cyclohexyl-5-{4-[dimethylamino-(3-fluor-phenyl)-methyl]-cyclohexylmethyl}-4,5-dihydro-1H-imidazol-4-carbonsäuremethylester
1-Cyclohexyl-5-{4-[dimethylamino-(4-fluor-phenyl)-methyl]-cyclohexylmethyl}-4-(2-methoxycarbonyl-ethyl)-4,5-dihydro-1 H-imidazol-4-carbonsäuremethylester
4-sec-Butyl-1-cyclohexyl-5-{4-[dimethylamino-(4-fluor-phenyl)-methyl]-cyclohexylmethyl}-4,5-dihydro-1 H-imidazol-4-carbonsäuremethylester
1-Cyclohexyl-5-{4-[dimethylamino-(4-fluor-phenyl)-methyl]-cyclohexylmethyl}-4-methylsulfanylmethyl-4,5-dihydro-1 H-imidazol-4-carbonsäuremethylester
1-Cyclohexyl-5-[4-(1-dimethylamino-3-phenyl-propyl)-cyclohexylmethyl]-4-(2-methoxycarbonyl-ethyl)-4,5-dihydro-1 H-imidazol-4-carbonsäuremethylester
1-Cyclohexyl-5-[4-(1-dimethylamino-3-phenyl-propyl)-cyclohexylmethyl]-4-methylsulfanylmethyl-4,5-dihydro-1 H-imidazol-4-carbonsäuremethylester
4-Benzylsulfanylmethyl-1-cyclohexyl-5-[4-(1-dimethylamino-3-phenyl-propyl)-cyclohexylmethyl]-4,5-dihydro-1 H-imidazol-4-carbonsäuremethylester
5-{4-[Dimethylamino-(3-fluor-phenyl)-methyl]-cyclohexylmethyl}-4-(2-methoxycarbonyl-ethyl)-1-propyl-4,5-dihydro-1 H-imidazol-4-carbonsäuremethylester
5-{4-[Dimethylamino-(3-fluor-phenyl)-methyl]-cyclohexylmethyl}-4-methylsulfanylmethyl-1-propyl-4,5-dihydro-1 H-imidazol-4-carbonsäuremethylester
5-{4-[Dimethylamino-(4-fluor-phenyl)-methyl]-cyclohexylmethyl}-4-(2-methoxycarbonyl-ethyl)-1-propyl-4,5-dihydro-1 Himidazol-4-carbonsäuremethylester
5-{4-[Dimethylamino-(4-fluor-phenyl)-methyl]-cyclohexylmethyl}-4-methylsulfanylmethyl-1-propyl-4,5-dihydro-1 H-imidazol-4-carbonsäuremethylester
4-Benzylsulfanylmethyl-5-{4-[dimethylamino-(4-fluor-phenyl)-methyl]-cyclohexylmethyl}-1-propyl-4,5-dihydro-1H-imidazol-4-carbonsäuremethylester
Methyl-4-sec-butyl-5-((4-((4-chlorphenyl)(dimethylamino)methyl)cyclohexyl)methyl)-1-cyclohexyl-4,5-dihydro-1 H-imidazol-4-carboxylat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Ein weiterer Gegenstand der Erfindung ist ein Verfahren zur Herstellung eines erfindungsgemäßen Imidazolin-Derivates. Dabei schützt man die Ketofunktion von 4-Oxo-cyclohexancarbonsäureester, 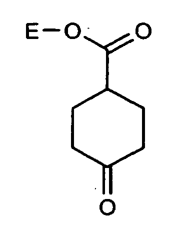 wobei E für einen C₁₋₆-Alkylrest, vorzugsweise Ethyl, steht nach dem Fachmann bekannten Methoden,

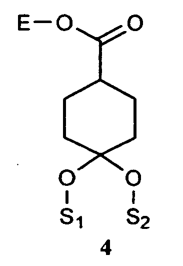

Wobei S1 und S2 jeweils für eine Schutzgruppe stehen, vorzugsweise einen Ring bilden und zusammen für -CH₂-CH₂- stehen. Der Ester **4** wird mit einem Reduktionsmittel, beispielsweise Diisobutylaluminiumhydrid zum Aldehyd **5** 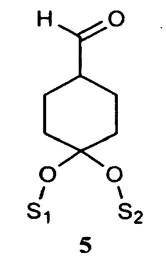 reduziert. Durch Zugabe eines Amins der allgemeinen Formel R⁵R⁶NH und eines Cyanids, beispielsweise KCN oder NaCN, wird der Aldehyd **5** unter Zugabe einer Säure, beispielsweise Salzsäure, in einem organischen Lösungsmittel, beispielsweise Methanol oder Ethanol, zum Nitril **6** umgesetzt.

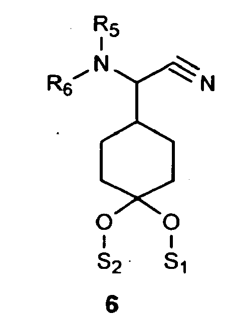

Das Nitril **6** wird mit einem Grignard-Reagenz der allgemeinen Formel R²MgHal, wobei Hal für Br, Cl oder I steht, oder einer metallorganischen Verbindung der allgemeinen Formel R²Li in einem organischen Lösungsmittel, beispielsweise Diethylether, Dioxan oder Tetrahydrofuran, zu einer Verbindung der allgemeinen Formel **7** umgesetzt.

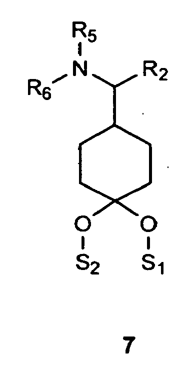

Die Schutzgruppen werden nach den üblichen Methoden abgespalten, wobei man das Keton **8** erhält.

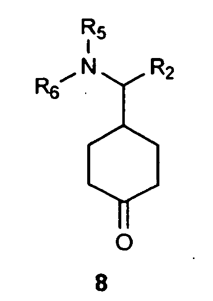

Der Aldehyd **9** 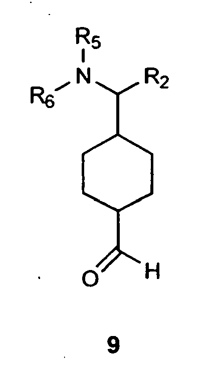 wird durch Umsetzung des Ketons **8** mit (Methoxymethyl)triphenylphosphonium-chlorid und einer starken Base, beispielsweise Kalium-tert-butylat, bei einer Temperatur von -20°C und +30°C, erhalten. Durch Umsetzung von Aldehyd **9** mit (Methoxymethyl)triphenylphosphoniumchlorid und einer starken Base, beispielsweise Kalium-tert-butylat, bei einer Temperatur von -20°C und +30°C, wird ein Aldehyd der allgemeinen Formel **10** erhalten.

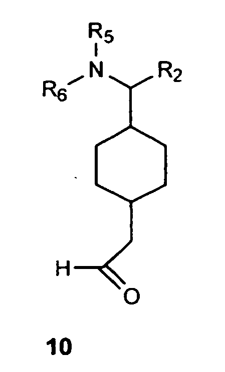

Über eine Wiederholung des letzten Reaktionsschrittes können Aldehyde erhalten werden, bei denen n für 2 steht.

Die Aldehyde der allgemeinen Formel **B** werden mit Aminen der allgemeinen Formel **A** und Isonitrilestern der allgemeinen Formel **C** in einem organischen Lösungsmittel, beispielsweise Methanol oder Ethanol, bei einer Temperatur von 20-100°C nach dem folgenden Syntheseschema zu substituierten Imidazolin-Derivaten der allgemeinen Formel I umsetzt.

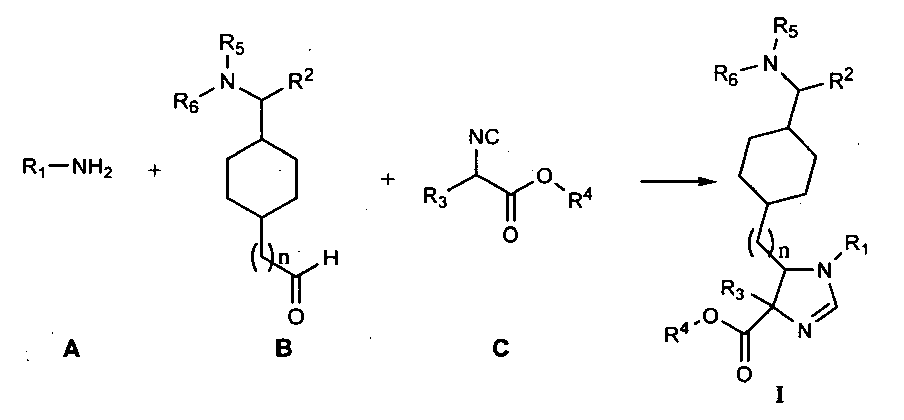

Die Amine der allgemeinen Formel **A** sind kommerziell erhältlich oder werden nach dem Fachmann bekannten Methoden hergestellt. Die Isonitrilester der allgemeinen Formel **C** sind entweder kommerziell erhältlich oder werden nach der Methode von R. S. Bon et al, Org. Lett. 2003, 5, 20, 3759-3762 hergestellt.

Die Trennung von Diastereomeren und/oder Enantiomeren erfolgt nach dem Fachmann bekannten Methoden, beispielsweise durch Umkristallisation, Chromatographie oder insbesondere HPLC-Chromatographie bzw. Kristallisation mit einer gegebenenfalls chiralen Säure oder Base und Trennung der Salze oder chirale HPLC-Chromatographie (Fogassy et al., Optical resolution methods, Org. Biomol. Chem 2006, 4, 3011-3030).

Bei Verbindungen, bei denen R⁴ C₁₋₄-Alkyl oder einen über eine C₁₋₃-Alkylkette gebundenen Arylrest bedeutet kann R⁴ nach der folgenden Methode abgespalten werden:

Der Ester wird in wenig Dioxan oder THF gelöst oder suspendiert und mit 1 N Natron- oder Kalilauge (10 ml / g) unter Rückfluss erhitzt, bis sich alles gelöst hat. Zur Aufarbeitung wird zunächst zur Trockne eingeengt und anschließend soviel Wasser zugegeben, bis sich der Rückstand gerade löst. Man säuert mit konz. HCI vorsichtig an (pH 1) und extrahiert die Säure mehrfach mit Diethylether oder Dichlormethan. Die vereinigten organischen Phasen werden mit wenig gesättigter Kochsalzlösung gewaschen, getrocknet und eingeengt.

Die Aldehyde der allgemeinen Formel **B** sind neu und weisen überaschenderweise eine gute Bindung an den µ-Opioid-Rezeptor sowie eine Hemmung der Noradrenalinsowie der Serotonin-Wiederaufnahme auf. Daher ist ein weiterer Gegenstand der Erfindung ein Aldehyd der allgemeinen Formel **B** 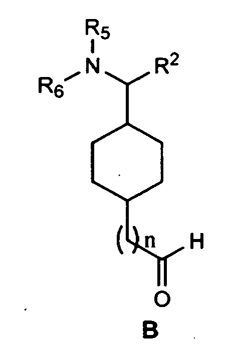 worin die Reste R², R⁵, R⁶ sowie n die oben angegebene Bedeutung haben.

Bevorzugt sind erfindungsgemäße Aldehyde der allgemeinen Formel B aus der Gruppe
4-[Dimethylamino-(4-fluor-phenyl)-methyl]-cyclohexanecarbaldehyd
4-(1-Dimethylamino-3-phenyl-propyl)-cyclohexanecarbaldehyd
4-[Dimethylamino-(3-fluor-phenyl)-methyl]-cyclohexanecarbaldehyd
4-[(4-Chlor-phenyl)-dimethylamino-methyl]-cyclohexanecarbaldehyd
{4-[(4-Chlor-phenyl)-dimethylamino-methyl]-cyclohexyl}-acetaldehyd
{4-[Dimethylamino-(3-fluor-phenyl)-methyl]-cyclohexyl}-acetaldehyd
4-(Dimethylamino-thiophen-2-yl-methyl)-cyclohexanecarbaldehyd
{4-[Dimethylamino-(4-fluor-phenyl)-methyl]-cyclohexyl}acetaldehyd
[4-(Dimethylamino-thiophen-2-yl-methyl)-cyclohexyl]-acetaldehyd
[4-(1 -Dimethylamino-3-phenyl-propyl)-cydohexyl]-acetaldehyd
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes substituiertes Imidazolin-Derivat der allgemeinen Formel I bzw. ein erfindungsgemäßer substituierter Aldehyd der allgemeinen Formel **B**, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Imidazolin-Derivat oder einem erfindungsgemäßen substituierten Aldehyd gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Imidazolin-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Imidazolin-Derivate oder Aldehyde verzögert freisetzen. Prinzipiell können den erfindungsgemäßen Arzneimitteln andere dem Fachmann bekannte weitere Wirkstoffe zugesetzt werden. Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens eines erfindungsgemäßen Imidazolin-Derivats oder Aldehyds appliziert.

In dem Arzneimittel kann ein enthaltenes erfindungsgemäßes Imidazolin-Derivat oder enthaltener erfindungsgemäßer Aldehyd als reines Diastereomer und/oder Enantiomer, als Razemat oder als nicht-äquimolare oder äquimolare Mischung der Diastereomere und/oder Enantiomere vorliegen.

Ein weiterer Gegenstand der Erfindung ist die Verwendung eines erfindungsgemäßen Imidazolin-Derivats oder substituierten Aldehyds zur Herstellung eines Arzneimittels zur Behandlung von Schmerz, insbesondere von akutem, neuropathischem oder chronischem Schmerz.

Ein weiterer Gegenstand der Erfindung ist die Verwendung eines erfindungsgemäßen Imidazolin-Derivats oder substituierten Aldehyds zur Herstellung eines Arzneimittels zur Behandlung von Depressionen und/oder zur Anxiolyse.

Die substituierten Imidazolin-Derivate oder substituierten Aldehyde der allgemeinen Formel bzw. der allgemeinen Formel B eignen sich auch zur Behandlung von Harninkontinenz, Diarrhöe, Pruritus, Alkohol- und Drogenmißbrauch, Medikamentenabhängigkeit und Antriebslosigkeit.

Gegenstand der Erfindung ist daher auch die Verwendung eines substituierten Imidazolin-Derivates der allgemeinen Formel I bzw eines substituierten Aldehyds der allgemeinen Formel B zur Herstellung eines Arzneimittels zur Behandlung von von Harninkontinenz, Diarrhöe, Pruritus, Alkohol- und Drogenmißbrauch, Medikamentenabhängigkeit und Antriebslosigkeit.

Besonders bevorzugt werden die erfindungsgemäßen substituierten Imidazolin-Derivate oder substituierten Aldehyde, die zur Herstellung eines Arzneimittels zur Behandlung von Schmerz, insbesondere von akutem, neuropathischem oder chronischem Schmerz, von Depressionen und/oder zur Anxiolyse, zur Behandlung von Harninkontinenz, Diarrhöe, Pruritus, Alkohol- und Drogenmißbrauch, Medikamentenabhängigkeit und Antriebslosigkeit verwendet werden, ausgewählt aus folgender Gruppe:
4-[Dimethylamino-(4-fluor-phenyl)-methyl]-cyclohexanecarbaldehyd
4-(1-Dimethylamino-3-phenyl-propyl)-cyclohexanecarbaldehyd
4-[Dimethylamino-(3-fluor-phenyl)-methyl]-cyclohexanecarbaldehyd
4-[(4-Chlor-phenyl)-dimethylamino-methyl]-cyclohexanecarbaldehyd
{4-[(4-Chlor-phenyl)-dimethylamino-methyl]-cyclohexyl}-acetaldehyd
{4-[Dimethylamino-(3-fluor-phenyl)-methyl]-cyclohexyl}-acetaldehyd
4-(Dimethylamino-thiophen-2-yl-methyl)-cyclohexanecarbaldehyd
{4-[Dimethylamino-(4-fluor-phenyl)-methyl]-cyclohexyl}-acetaldehyd
[4-(Dimethylamino-thiophen-2-yl-methyl)-cyclohexyl]-acetaldehyd
[4-(1-Dimethylamino-3-phenyl-propyl)-cyclohexyl]-acetaldehyd
1-Cyclohexyl-5-{4-[dimethylamino-(3-fluor-phenyl)-methyl]-cyclohexyl}-4-isobutyl-4,5-dihydro-1 H-imidazol-4-carbonsäuremethylester
4-(4-Chlor-benzyl)-1-cyclohexyl-5-{4-[dimethylamino-(3-fluor-phenyl)-methyl]-cyclohexyl}-4,5-dihydro-1H-imidazol-4-carbonsäuremethylester
4-Butyl-1-cyclohexyl-5-{4-[dimethylamino-(3-fluor-phenyl)-methyl]-cyclohexyl}-4,5-dihydro-1 H-imidazol-4-carbonsäuremethylester
5-{4-[(4-Chlor-phenyl)-dimethylamino-methyl]-cyclohexyl}-1-cyclohexyl-4-isobutyl-4,5-dihydro-1 H-imidazol-4-carbonsäuremethylester
4-(4-Chlor-benzyl)-5-{4-[(4-chlor-phenyl)-dimethylamino-methyl]-cyclohexyl}-1-cyclohexyl-4,5-dihydro-1 H-imidazol-4-carbonsäuremethylester
4-Butyl-5-{4-[(4-chlor-phenyl)-dimethylamino-methyl]-cyclohexyl}-1-cyclohexyl-4,5-dihydro-1 H-imidazol-4-carbonsäuremethylester
5-{4-[(4-Chlor-phenyl)-dimethylamino-methyl]-cyclohexylmethyl}-1-cyclohexyl-4-isobutyl-4,5-dihydro-1H-imidazol-4-carbonsäuremethylester
4-(4-Chlor-benzyl)-5-{4-[(4-chlor-phenyl)-dimethylamino-methyl]-cyclohexylmethyl}-1-cyclohexyl-4,5-dihydro-1 H-imidazol-4-carbonsäuremethylester
4-Butyl-5-{4-[(4-chlor-phenyl)-dimethylamino-methyl]-cyclohexylmethyl}-1-cyclohexyl-4,5-dihydro-1H-imidazol-4-carbonsäuremethylester
4-(4-Chlor-benzyl)-1-cyclohexyl-5-[4-(dimethylamino-thiophen-2-yl-methyl)-cyclohexylmethyl]-4,5-dihydro-1 H-imidazol-4-carbonsäuremethylester
5-{4-[Dimethylamino-(3-fluor-phenyl)-methyl]-cyclohexyl}-4-isobutyl-1-propyl-4,5-dihydro-1 H-imidazol-4-carbonsäuremethylester
4-(4-Chlor-benzyl)-5-{4-[dimethylamino-(3-fluor-phenyl)-methyl]-cyclohexyl}-1-propyl-4,5-dihydro-1 H-imidazol-4carbonsäuremethylester
4-Butyl-5-{4-[dimethylamino-(3-fluor-phenyl)-methyl]-cyclohexyl}-1-propyl-4,5-dihydro-1 H-imidazol-4-carbonsäuremethylester
5-{4-[(4-Chlor-phenyl)-dimethylamino-methyl]-cyclohexyl}-4-isobutyl-1-propyl-4,5-dihydro-1 H-imidazol-4-carbonsäuremethylester
4-(4-Chlor-benzyl)-5-{4-[(4-chlor-phenyl)-dimethylamino-methyl]-cyclohexyl}-1-propyl-4,5-dihydro-1 H-imidazol-4-carbonsäuremethylester
5-{4-[(4-Chlor-phenyl)-dimethylamino-methyl]-cyclohexylmethyl}-4-isobutyl-1-propyl-4,5-dihydro-1 H-imidazol-4-carbonsäuremethylester
4-(4-Chlor-benzyl)-5-{4-[(4-chlor-phenyl)-dimethylamino-methyl]-cyclohexylmethyl}-1-propyl-4,5-dihydro-1H-imidazol-4-carbonsäuremethylester
4-Butyl-5-{4-[(4-chlor-phenyl)-dimethylamino-methyl]-cyclohexylmethyl}-1-propyl-4,5-dihydro-1 H-imidazol-4-carbonsäuremethylester
1-Butyl-5-{4-[dimethylamino-(3-fluor-phenyl)-methyl]-cyclohexyl}-4-isobutyl-4,5-dihydro-1 H-imidazol-4-carbonsäuremethylester
1-Butyl-4-(4-chlor-benzyl)-5-{4-[dimethylamino-(3-fluor-phenyl)-methyl]-cyclohexyl}-4,5-dihydro-1 H-imidazol-4-carbonsäuremethylester
1-Butyl-5-{4-[(4-chlor-phenyl)-dimethylamino-methyl]-cyclohexyl}-4-isobutyl-4,5-dihydro-1 H-imidazol-4-carbonsäuremethylester
1-Butyl-4-(4-chlor-benzyl)-5-{4-[(4-chlor-phenyl)-dimethylamino-methyl]-cyclohexyl}-4,5-dihydro-1H-imidazol-4-carbonsäuremethylester
1,4-Dibutyl-5-{4-[(4-chlor-phenyl)-dimethylamino-methyl]-cyclohexyl}-4,5-dihydro-1H-imidazol-4-carbonsäuremethylester
1-Butyl-5-{4-[(4-chlor-phenyl)-dimethylamino-methyl]-cyclohexylmethyl}-4-isobutyl-4,5-dihydro-1 H-imidazol-4-carbonsäuremethylester
1-Butyl-4-(4-chlor-benzyl)-5-{4-[(4-chlor-phenyl)-dimethylamino-methyl]-cyclohexylmethyl}-4,5-dihydro-1 H-imidazol-4-carbonsäuremethylester
1,4-Dibutyl-5-{4-[(4-chlor-phenyl)-dimethylamino-methyl]-cyclohexylmethyl}-4,5-dihydro-1 H-imidazol-4-carbonsäuremethylester
5-{4-[Dimethylamino-(3-fluor-phenyl)-methyl]-cyclohexyl}-4-isobutyl-1-phenethyl-4,5-dihydro-1H-imidazol-4-carbonsäuremethylester
4-(4-Chlor-benzyl)-5-{4-[dimethylamino-(3-fluor-phenyl)-methyl]-cyclohexyl}-1-phenethyl-4,5-dihydro-1 H-imidazol-4-carbonsäuremethylester
4-Butyl-5-{4-[dimethylamino-(3-fluor-phenyl)-methyl]-cyclohexyl}-1-phenethyl-4,5-dihydro-1 H-imidazol-4-carbonsäuremethylester
5-{4-[(4-Chlor-phenyl)-dimethylamino-methyl]-cyclohexyl}-4-isobutyl-1-phenethyl-4,5-dihydro-1 H-imidazol-4-carbonsäuremethylester
4-(4-Chlor-benzyl)-5-{4-[(4-chlor-phenyl)-dimethylamino-methyl]-cyclohexyl}-1-phenethyl-4,5-dihydro-1H-imidazol-4-carbonsäuremethylester
4-Butyl-5-{4-[(4-chlor-phenyl)-dimethylamino-methyl]-cyclohexyl}-1-phenethyl-4,5-dihydro-1H-imidazol-4-carbonsäuremethylester
5-{4-[(4-Chlor-phenyl)-dimethylamino-methyl]-cyclohexyimethyl}-4-isobutyl-1-phenethyl-4,5-dihydro-1H-imidazol-4carbonsäuremethylester
4-(4-Chlor-benzyl)-5-{4-[(4-chlor-phenyl)-dimethylamino-methyl]-cyclohexylmethyl}-1-phenethyl-4,5-dihydro-1H-imidazol-4-carbonsäuremethylester
4-Butyl-5-{4-[(4-chlor-phenyl)-dimethylamino-methyl]-cyclohexylmethyl}-1-phenethyl-4,5-dihydro-1H-imidazol-4-carbonsäuremethylester
1-Benzyl-5-{4-[dimethylamino-(3-fluor-phenyl)-methyl]-cyclohexyl}-4-isobutyl-4,5-dihydro-1H-imidazol-4-carbonsäuremethylester
1-Benzyl-4-(4-chlor-benzyl)-5-{4-[dimethylamino-(3-fluor-phenyl)-methyl]-cyclohexyl}-4,5-dihydro-1H-imidazol-4-carbonsäuremethylester
1-Benzyl-5-{4-[(4-chlor-phenyl)-dimethylamino-methyl]-cyclohexyl}-4-isobutyl-4,5-dihydro-1H-imidazol-4-carbonsäuremethylester
1-Benzyl-4-(4-chlor-benzyl)-5-{4-[(4-chlor-phenyl)-dimethylamino-methyl]-cyclohexyl}-4,5-dihydro-1H-imidazol-4-carbonsäuremethylester
1-Benzyl-5-{4-[(4-chlor-phenyl)-dimethylamino-methyl]-cyclohexylmethyl}-4-isobutyl-4,5-dihydro-1H-imidazol-4-carbonsäuremethylester
5-{4-[(4-Chlor-phenyl)-dimethylamino-methyl]-cyclohexyl}-1-cyclohexyl-4-(2-methoxycarbonyl-ethyl)-4,5-dihydro-1H-imidazol-4-carbonsäuremethylester
4-Benzylsulfanylmethyl-5-{4-[(4-chlor-phenyl)-dimethylamino-methyl]-cyclohexyl}-1-cyclohexyl-4,5-dihydro-1H-imidazol-4-carbonsäuremethylester
5-{4-[(4-Chlor-phenyl)-dimethylamino-methyl]-cyclohexylmethyl}-1-cyclohexyl-4-(2-methoxycarbonyl-ethyl)-4,5-dihydro-1H-imidazol-4-carbonsäuremethylester
1-Benzyl-5-{4-[dimethylamino-(3-fluor-phenyl)-methyl]-cyclohexylmethyl}-4-propyl-4,5-dihydro-1H-imidazol-4-carbonsäuremethylester
5-{4-[(4-Chlor-phenyl)-dimethylamino-methyl]-cyclohexylmethyl}-1-cyclohexyl-4-methylsulfanylmethyl-4,5-dihydro-1H-imidazol-4-carbonsäuremethylester
4-Benzylsulfanylmethyl-5-{4-[(4-chlor-phenyl)-dimethylamino-methyl]-cyclohexylmethyl}-1-cyclohexyl-4,5-dihydro-1H-imidazol-4-carbonsäuremethylester
5-{4-[Dimethylamino-(3-fluor-phenyl)-methyl]-cyclohexyl}-4-methylsulfanylmethyl-1-propyl-4,5-dihydro-1H-imidazol-4-carbonsäuremethylester
4-Benzylsulfanylmethyl-5-{4-[dimethylamino-(3-fluor-phenyl)-methyl]-cyclohexyl}-1-propyl-4,5-dihydro-1H-imidazol-4-carbonsäuremethylester
5-{4-[(4-Chlor-phenyl)-dimethylamino-methyl]-cyclohexyl}-4-(2-methoxycarbonyl-ethyl)-1-propyl-4,5-dihydro-1H-imidazol-4-carbonsäuremethylester
4-Benzylsulfanylmethyl-5-{4-[(4-chlor-phenyl)-dimethylamino-methyl]-cyclohexyl}-1-propyl-4,5-dihydro-1H-imidazol-4-carbonsäuremethylester
5-{4-[(4-Chlor-phenyl)-dimethylamino-methyl]-cyclohexylmethyl}-4-(2-methoxycarbonyl-ethyl)-1-propyl-4,5-dihydro-1H-imidazol-4-carbonsäuremethylester
4-sec-Butyl-5-{4-[(4-chlor-phenyl)-dimethylamino-methyl]-cyclohexylmethyl}-1-propyl-4,5-dihydro-1H-imidazol-4-carbonsäuremethylester
5-{4-[(4-Chlor-phenyl)-dimethylamino-methyl]-cyclohexylmethyl}-4-methylsulfanylmethyl-1-propyl-4,5-dihydro-1H-imidazol-4-carbonsäuremethylester
4-Benzylsulfanylmethyl-5-{4-[(4-chlor-phenyl)-dimethylamino-methyl]-cyclohexylmethyl}-1-propyl-4,5-dihydro-1H-imidazol-4-carbonsäuremethylester
1-Butyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
1-Butyl-{4-[dimethylamino-(3-fluor-phenyl)-methyl]-cyclohexyl}-4-methylsulfanylmethyl-4,5-dihydro-1H-imidazol-4-carbonsäuremethylester
4-Benzylsulfanylmethyl-1-butyl-5-{4-[dimethylamino-(3-fluor-phenyl)-methyl]-cyclohexyl}-4,5-dihydro-1H-imidazol-4-carbonsäuremethylester
1-Butyl-5-{4-[(4-chlor-phenyl)-dimethylamino-methyl]-cyclohexyl}-4-(2-methoxycarbonyl-ethyl)-4,5-dihydro-1H-imidazol-4-carbonsäuremethylester
4-Benzylsulfanylmethyl-1-butyl-5-{4-[(4-chlor-phenyl)-dimethylamino-methyl]-cyclohexyl}-4,5-dihydro-1H-imidazol-4-carbonsäuremethylester
1-Butyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester
5-[4-(1-Dimethylamino-3-phenyl-propyl)-cyclohexylmethyl]-4-(2-methoxycarbonylethyl)-1-propyl-4,5-dihydro-1H-imidazol-4-carbonsäuremethylester
1-Butyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
1-Butyl-4-sec-butyl-5-{4-[dimethylamino-(3-fluor-phenyl)-methyl]-cyclohexylmethyl}-4,5-dihydro-1H-imidazol-4-carbonsäuremethylester
1-Butyl-5-{4-[dimethylamino-(3-fluor-phenyl)-methyl]-cyclohexylmethyl}-4-methylsulfanylmethyl-4,5-dihydro-1H-imidazol-4-carbonsäuremethylester
4-Benzylsulfanylmethyl-1-butyl-5-{4-[dimethylamino-(3-fluor-phenyl)-methyl]-cyclohexylmethyl}-4,5-dihydro-1H-imidazol-4-carbonsäuremethylester
1-Butyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
1-Butyl-5-{4-[dimethylamino-(4-fluor-phenyl)-methyl]-cyclohexylmethyl}-4-methylsulfanylmethyl-4,5-dihydro-1H-imidazol-4-carbonsäuremethylester
1-Butyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester
4-Benzylsulfanylmethyl-1-butyl-5-[4-(1-dimethylamino-3-phenyl-propyl)-cyclohexylmethyl]-4,5-dihydro-1H-imidazol-4-carbonsäuremethylester
5-{4-[Dimethylamino-(3-fluor-phenyl)-methyl]-cyclohexylmethyl}-4-(2-methoxycarbonyl-ethyl)-1-phenethyl-4,5-dihydro-1H-imidazol-4-carbonsäuremethylester
4-sec-Butyl-5-{4-[dimethylamino-(3-fluor-phenyl)-methyl]-cyclohexylmethyl}-1-phenethyl-4,5-dihydro-1H-imidazol-4-carbonsäuremethylester
4-Benzylsulfanylmethyl-5-{4-[dimethylamino-(3-fluor-phenyl)-methyl]-cyclohexylmethyl}-1-phenethyl-4,5-dihydro-1H-imidazol-4-carbonsäuremethylester
5-{4-[Dimethylamino-(4-fluor-phenyl)-methyl]-cyclohexylmethyl}-4-(2-methoxycarbonyl-ethyl)-1-phenethyl-4,5-dihydro-1H-imidazol-4-carbonsäuremethylester
5-[4-(1-Dimethylamino-3-phenyl-propyl)-cyclohexylmethyl]-4-(2-methoxycarbonylethyl)-1-phenethyl-4,5-dihydro-1H-imidazol-4-carbonsäuremethylester
1-Benzyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
1-Benzyl-4-benzylsulfanylmethyl-5-{4-[dimethylamino-(3-fluor-phenyl)-methyl]-cyclohexylmethyl}-4,5-dihydro-1H-imidazol-4-carbonsäuremethylester
1-Benzyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
1-Butyl-4-sec-butyl-5-{4-[(4-chlor-phenyl)-dimethylamino-methyl]-cyclohexylmethyl}-4,5-dihydro-1H-imidazol-4-carbonsäuremethylester
1-Butyl-5-{4-[(4-chlor-phenyl)-dimethylamino-methyl]-cyclohexylmethyl}-4-methylsulfanylmethyl-4,5-dihydro-1H-imidazol-4-carbonsäuremethylester
4-Benzylsulfanylmethyl-1-butyl-5-{4-[(4-chlor-phenyl)-dimethylamino-methyl]-cyclohexylmethyl}-4,5-dihydro-1H-imidazol-4-carbonsäuremethylester
5-{4-[Dimethylamino-(3-fluor-phenyl)-methyl]-cyclohexyl}-4-(2-methoxycarbonylethyl)-1-phenethyl-4,5-dihydro-1H-imidazol-4-carbonsäuremethylester
4-Benzylsulfanylmethyl-5-{4-[dimethylamino-(3-fluor-phenyl)-methyl]-cyclohexyl}-1-phenethyl-4,5-dihydro-1H-imidazol-4-carbonsäuremethylester
4-Benzylsulfanylmethyl-5-{4-[(4-chlor-phenyl)-dimethylamino-methyl]-cyclohexyl}-1-phenethyl-4,5-dihydro-1H-imidazol-4-carbonsäuremethylester
5-{4-[(4-Chlor-phenyl)-dimethylamino-methyl]-cyclohexylmethyl}-4-(2-methoxycarbonyl-ethyl)-1-phenethyl-4,5-dihydro-1H-imidazol-4-carbonsäuremethylester
1-Benzyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
1-Benzyl-5-{4-[dimethylamino-(3-fluor-phenyl)-methyl]-cyclohexyl}-4-methylsulfanylmethyl-4,5-dihydro-1H-imidazol-4-carbonsäuremethylester
1-Benzyl-4-benzylsulfanylmethyl-5-{4-[dimethylamino-(3-fluor-phenyl)-methyl]-cyclohexyl}-4,5-dihydro-1H-imidazol-4-carbonsäuremethylester
1-Benzyl-4-benzylsulfanylmethyl-5-{4-[(4-chlor-phenyl)-dimethylamino-methyl]-cyclohexyl}-4,5-dihydro-1H-imidazol-4-carbonsäuremethylester
1-Benzyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester
1-Benzyl-5-{4-[(4-chlor-phenyl)-dimethylamino-methyl]-cyclohexylmethyl}-4-methylsulfanylmethyl-4,5-dihydro-1H-imidazol-4-carbonsäuremethylester
1-Benzyl-4-benzylsulfanylmethyl-5-{4-[(4-chlor-phenyl)-dimethylamino-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexylmethyl}-4-propyl-4,5-dihydro-1H-imidazol-4-carbonsäuremethylester
1-Cyclohexyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester
1-Cyclohexyl-5-{4-[dimethylamino-(3-fluor-phenyl)-methyl]-cyclohexylmethyl}-4-methyl-4,5-dihydro-1H-imidazol-4-carbonsäuremethylester
1-Cyclohexyl-5-{4-[dimethylamino-(4-fluor-phenyl)-methyl]-cyclohexylmethyl}-4-propyl-4,5-dihydro-1H-imidazol-4-carbonsäuremethylester
1-Cyclohexyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester
1-Cyclohexyl-5-{4-[dimethylamino-(4-fluor-phenyl)-methyl]-cyclohexylmethyl}-4-methyl-4,5-dihydro-1H-imidazol-4-carbonsäuremethylester
1-Cyclohexyl-5-[4-(1-dimethylamino-3-phenyl-propyl)-cyclohexylmethyl]-4-propyl-4,5-dihydro-1H-imidazol-4-carbonsäuremethylester
1-Cyclohexyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
1-Cyclohexyl-5-[4-(1-dimethylamino-3-phenyl-propyl)-cyclohexylmethyl]-4-methyl-4,5-dihydro-1H-imidazol-4-carbonsäuremethylester
5-{4-[Dimethylamino-(3-fluor-phenyl)-methyl]-cyclohexylmethyl}-1,4-dipropyl-4,5-dihydro-1H-imidazol-4-carbonsäuremethylester
5-{4-[Dimethylamino-(3-fluor-phenyl)-methyl]-cyclohexylmethyl}-4-(2-methylsulfanyl-ethyl)-1-propyl-4,5-dihydro-1H-imidazol-4-carbonsäuremethylester
1-Benzyl-4-(4-chlor-benzyl)-5-{4-[(4-chlor-phenyl)-dimethylamino-methyl]-cyclohexylmethyl}-4,5-dihydro-1H-imidazol-4-carbonsäuremethylester
1-Benzyl-4-butyl-5-{4-[(4-chlor-phenyl)-dimethylamino-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
4-Benzylsulfanylmethyl-1-cyclohexyl-5-{4-[dimethylamino-(3-fluor-phenyl)-methyl]-cyclohexyl}-4,5-dihydro-1H-imidazol-4-carbonsäuremethylester
5-{4-[Dimethylamino-(3-fluor-phenyl)-methyl]-cyclohexylmethyl}-4-methyl-1-propyl-4,5-dihydro-1H-imidazol-4-carbonsäuremethylester
5-{4-[Dimethylamino-(4-fluor-phenyl)-methyl]-cyclohexylmethyl}-1,4-dipropyl-4,5-dihydro-1H-imidazol-4-carbonsäuremethylester
5-{4-[Dimethylamino-(4-fluor-phenyl)-methyl]-cyclohexylmethyl}-4-(2-methylsulfanyl-ethyl)-1-propyl-4,5-dihydro-1H-imidazol-4-carbonsäuremethylester
5-[4-(1-Dimethylamino-3-phenyl-propyl)-cyclohexylmethyl]-1,4-dipropyl-4,5-dihydro-1H-imidazol-4-carbonsäuremethylester
5-[4-(1-Dimethylamino-3-phenyl-propyl)-cyclohexylmethyl]-4-(2-methylsulfanyl-ethyl)-1-propyl-4,5-dihydro-1H-imidazol-4-carbonsäuremethylester
1-Butyl-5-{4-[dimethylamino-(3-fluor-phenyl)-methyl]-cyclohexylmethyl}-4-propyl-4,5-dihydro-1H-imidazol-4-carbonsäuremethylester
1-Butyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester
1-Butyl-5-{4-[dimethylamino-(3-fluor-phenyl)-methyl]-cyclohexylmethyl}-4-methyl-4,5-dihydro-1H-imidazol-4-carbonsäuremethylester
1-Butyl-5-{4-[dimethylamino-(4-fluor-phenyl)-methyl]-cyclohexylmethyl}-4-propyl-4,5-dihydro-1H-imidazol-4-carbonsäuremethylester
1-Butyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester
1-Butyl-5-[4-(1-dimethylamino-3-phenyl-propyl)-cyclohexylmethyl]-4-propyl-4,5-dihydro-1H-imidazol-4-carbonsäuremethylester
1-Butyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
5-{4-[Dimethylamino-(3-fluor-phenyl)-methyl]-cyclohexylmethyl}-1-phenethyl-4-propyl-4,5-dihydro-1H-imidazol-4-carbonsäuremethylester
5-{4-[Dimethylamino-(3-fluor-phenyl)-methyl]-cyclohexylmethyl}-4-(2-methylsulfanyl-ethyl)-1-phenethyl-4,5-dihydro-1H-imidazol-4-carbonsäuremethylester
5-{4-[Dimethylamino-(3-fluor-phenyl)-methyl]-cyclohexylmethyl}-4-methyl-1-phenethyl-4,5-dihydro-1H-imidazol-4-carbonsäuremethylester
5-{4-[Dimethylamino-(4-fluor-phenyl)-methyl]-cyclohexylmethyl}-1-phenethyl-4-propyl-4,5-dihydro-1H-imidazol-4-carbonsäuremethylester
5-{4-[Dimethylamino-(4-fluor-phenyl)-methyl]-cyclohexylmethyl}-4-(2-methylsulfanyl-ethyl)-1-phenethyl-4,5-dihydro-1H-imidazol-4-carbonsäuremethylester
5-{4-[Dimethylamino-(4-fluor-phenyl)-methyl]-cyclohexylmethyl}-4-methyl-1-phenethyl-4,5-dihydro-1H-imidazol-4-carbonsäuremethylester
5-[4-(1-Dimethylamino-3-phenyl-propyl)-cyclohexylmethyl]-1-phenethyl-4-propyl-4,5-dihydro-1H-imidazol-4-carbonsäuremethylester
5-[4-(1-Dimethylamino-3-phenyl-propyl)-cyclohexylmethyl]-4-(2-methylsulfanyl-ethyl)-1-phenethyl-4,5-dihydro-1H-imidazol-4-carbonsäuremethylester
5-[4-(1-Dimethylamino-3-phenyl-propyl)-cyclohexylmethyl]-4-methyl-1-phenethyl-4,5-dihydro-1H-imidazol-4-carbonsäuremethylester
1-Benzyl-5-{4-[dimethylamino-(3-fluor-phenyl)-methyl]-cyclohexylmethyl}-4-propyl-4,5-dihydro-1H-imidazol-4-carbonsäuremethylester
1-Benzyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester
1-Benzyl-5-{4-[dimethylamino-(3-fluor-phenyl)-methyl]-cyclohexylmethyl}-4-methyl-4,5-dihydro-1H-imidazol-4-carbonsäuremethylester
1-Benzyl-5-{4-[dimethylamino-(4-fluor-phenyl)-methyl]-cyclohexylmethyl}-4-propyl-4,5-dihydro-1H-imidazol-4-carbonsäuremethylester
1-Benzyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester
1-Benzyl-5-{4-[dimethylamino-(4-fluor-phenyl)-methyl]-cyclohexylmethyl}-4-methyl-4,5-dihydro-1H-imidazol-4-carbonsäuremethylester
1-Benzyl-5-[4-(1-dimethylamino-3-phenyl-propyl)-cyclohexylmethyl]-4-propyl-4,5-dihydro-1H-imidazol-4-carbonsäuremethylester
1-Benzyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
1-Benzyl-5-[4-(1-dimethylamino-3-phenyl-propyl)-cyclohexylmethyl]-4-methyl-4,5-dihydro-1H-imidazol-4-carbonsäuremethylester
1-Cyclohexyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
4-sec-Butyl-1-cyclohexyl-5-{4-[dimethylamino-(3-fluor-phenyl)-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexylmethyl}-4-methylsulfanylmethyl-4,5-dihydro-1H-imidazol-4-carbonsäuremethylester
4-Benzylsulfanylmethyl-1-cyclohexyl-5-{4-[dimethylamino-(3-fluor-phenyl)-methyl]-cyclohexylmethyl}-4,5-dihydro-1H-imidazol-4-carbonsäuremethylester
1-Cyclohexyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
4-sec-Butyl-1-cyclohexyl-5-{4-[dimethylamino-(4-fluor-phenyl)-methyl]-cyclohexylmethyl}-4,5-dihydro-1H-imidazol-4-carbonsäuremethylester
1-Cyclohexyl-5-{4-[dimethylamino-(4-fluor-phenyl)-methyl]-cyclohexylmethyl}-4-methylsulfanylmethyl-4,5-dihydro-1H-imidazol-4-carbonsäuremethylester
1-Cyclohexyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester
1-Cyclohexyl-5-[4-(1-dimethylamino-3-phenyl-propyl)-cyclohexylmethyl]-4-methylsulfanylmethyl-4,5-dihydro-1H-imidazol-4-carbonsäuremethylester
4-Benzylsulfanylmethyl-1-cyclohexyl-5-[4-(1-dimethylamino-3-phenyl-propyl)-cyclohexylmethyl]-4,5-dihydro-1H-imidazol-4-carbonsäuremethylester
5-{4-[Dimethylamino-(3-fluor-phenyl)-methyl]-cyclohexylmethyl}-4-(2-methoxycarbonyl-ethyl)-1-propyl-4,5-dihydro-1H-imidazol-4-carbonsäuremethylester
5-{4-[Dimethylamino-(3-fluor-phenyl)-methyl]-cyclohexylmethyl}-4-methylsulfanylmethyl-1-propyl-4,5-dihydro-1H-imidazol-4-carbonsäuremethylester
5-{4-[Dimethylamino-(4-fluor-phenyl)-methyl]-cyclohexylmethyl}-4-(2-methoxycarbonyl-ethyl)-1-propyl-4,5-dihydro-1Himidazol-4-carbonsäuremethylester
5-{4-[Dimethylamino-(4-fluor-phenyl)-methyl]-cyclohexylmethyl}-4-methylsulfanylmethyl-1-propyl-4,5-dihydro-1H-imidazol-4-carbonsäuremethylester
4-Benzylsulfanylmethyl-5-{4-[dimethylamino-(4-fluor-phenyl)-methyl]-cyclohexylmethyl}-1-propyl-4,5-dihydro-1H-imidazol-4-carbonsäuremethylester
Methyl-4-sec-butyl-5-((4-((4-chlorphenyl)(dimethylamino)methyl)cyclohexyl)methyl)-1-cyclohexyl-4,5-dihydro-1H-imidazol-4-carboxylat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

### Beispiele

### Allgemeine Vorschrift zur Synthese von 4-[Dimethylamino-methyl]-cyclohexancarbaldehyden bzw. 4-[Dimethylamino-methyl]-cyclohexyl-acetaldehyden

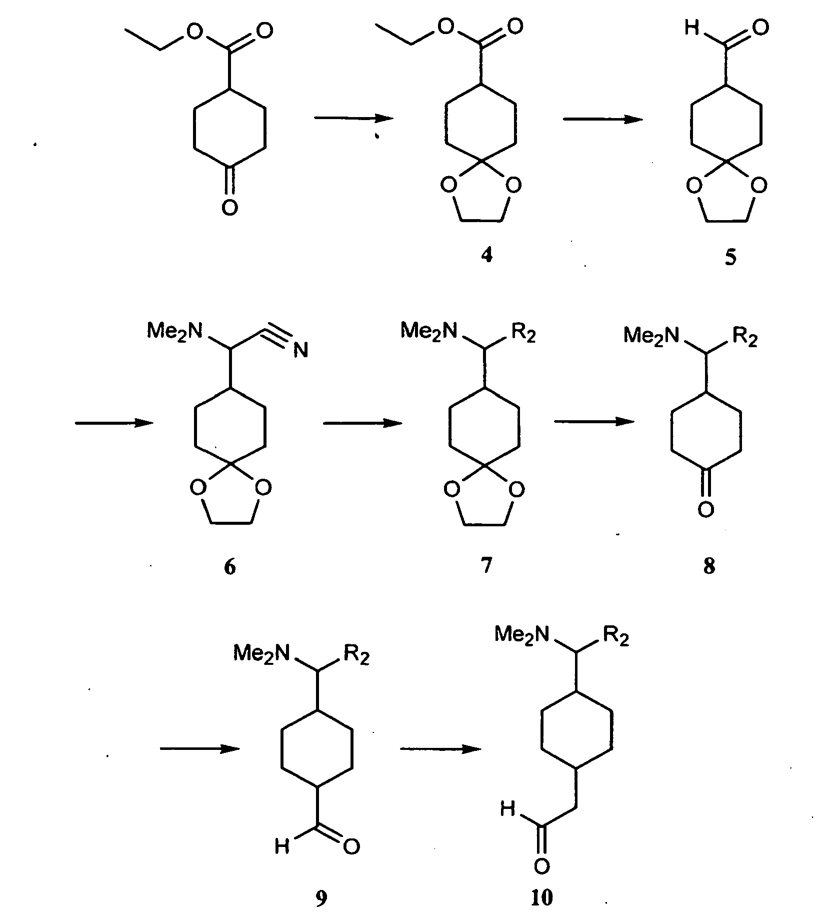

### 1. Stufe

Synthese des 1,4-Dioxa-spiro[4.5]decan-8-carbonsäureethylesters **4** 4-Oxo-cyclohexancarbonsäure-ethylester (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet (Na₂SO₄), i. Vak. eingeengt und die verbliebene farblose Flüssigkeit ohne Reinigung weiter verarbeitet.

### 2. Stufe

### Synthese des 1,4-Dioxa-spiro[4.5]decane-8-carbaldehyds 5

Eine Lösung aus 1,4-Dioxa-spiro[4.5]decan-8-carbonsäureethylester **4** (32,13 g, 150 mmol) in absol. Toluol (160 ml) wurde bei -70 bis -65 °C unter Argon tropfenweise mit Düsobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Rektionslösung über Kieselgur abgesaugt. Das Kiesegur wurde zweimal mit Essigester gewaschen, die wäßrige Lösung abgetrennt und zweimal mit Essigester extrahiert. Die vereinigten organischen Extrakte wurden mit gesättigter Natriumchlorid-Lösung gewaschen, über Natriumsulfat getrocknet und i. Vak. eingeengt.

### 3. Stufe

### Synthese des Dimethylamino-(1,4-dioxa-spiro[4.5]dec-8-yl)-acetonitrils 6

Zu einem Gemisch aus 4N Salzsäure (37 ml) und Methanol (22 ml) wurde unter Eiskühlung 40-proz. wässrige Dimethylaminlösung (85 ml, 0,67 mol), 1,4-Dioxa-spiro-[4.5]decan-8-carbaldehyd **5** (240 g, 0,141 mol) und Kaliumcyanid (22,05 g, 0,338 mol) addiert. Die Mischung wurde 4 d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.

### 4. Stufe

### Synthese der [(1,4-Dioxa-spiro[4.5]dec-8-yl)-methyl]-dimethylamine 7

Eine 1M Lösung des entsprechenden Grignard-Reagenzes in THF oder Diethylether (220 ml, 220 mmol) wurde unter Argon und Eiskühlung tropfenweise mit einer Lösung des Aminonitrils **6i** (19,89 g, 88 mmol) in absol. THF (160 ml) bzw. absol. Diethylether (160 ml), je nach Lösungsmittel des Grignard-Reagenzes, versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet (Na₂SO₄) und eingeengt. Das erhaltene Produkt wurde ohne weitere Aufreinigung in die nächste Stufe eingesetzt.

### 5. Stufe

### Synthese der 4-[Dimethylamino-methyl]-cyclohexanone

Das Rohprodukt des entsprechenden [(1,4-Dioxa-spiro[4.5]dec-8-yl)-methyl]-dimethylamins **7i** (88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit Dichlormethan (3 x 100 ml) extrahiert, getrocknet und eingeengt. Die Produkte wurden als weiße Feststoffe oder Öle erhalten.

### 6. Stufe

### Synthese der 4-[Dimethylamino-methyl]-cyclohexan-carbaldehyde 9

(Methoxymethyl)triphenylphosphoniumchlorid (25,7 g, 75 mmol) wurde in absol. THF (100 ml) unter Argon suspendiert, bei 0 °C tropfenweise mit Kalium-*tert*-butylat (8,42 g, 75 mmol), gelöst in absol. THF (70 ml) versetzt und anschließend 15 min bei 0 °C nachgerührt.

Bei RT wurde dann das entsprechende 4-[Dimethylamino-methyl]-cyclohexanon 8 (50 mmol), gelöst in absol. THF (75 ml), zur obigen Lösung zugetropft und über Nacht bei RT gerührt. Unter Eiswasserkühlung wurde tropfenweise mit Wasser (38 ml) und 6N HCl (112 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) ausgeschüttelt, über Na₂SO₄ getrocknet und i. Vak. eingeengt. Das Rohprodukt wurde durch Flashchromatographie mit Essigester/Cyclohexan (1:1) gereinigt. Man erhielt in der Regel zwei Diastereomere in unterschiedlichen Verhältnissen.

### 7. Stufe

### Synthese der {4-[Dimethylamino-methyl]-cyclohexyl}-acetaldehyde 10

(Methoxymethyl)triphenylphosphoniumchlorid (43,53 g, 127 mmol) wurde in absol. THF (200 ml) unter Argon suspendiert, bei 0 °C tropfenweise mit Kalium-*tert*-butylat (14,25 g, 127 mmol), gelöst in absol. THF (130 ml), versetzt und anschließend 15 min bei 0 °C nachgerührt.

Bei RT wurde dann der entsprechende 4-[Dimethylamino-methyl]-cyclohexancarbaldehyd **9i** (85 mmol), gelöst in absol. THF (130 ml), zugetropft und über Nacht bei RT gerührt. Unter Eiswasserkühlung wurde tropfenweise mit Wasser (80 ml) und 6N HCl (200 ml) hydrolysiert. Nach 1 h Rühren bei RT wurde zehnmal mit Ether (je 100 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:1 oder 1:2) gereinigt. Man erhielt in der Regel zwei Diastereomere in unterschiedlichen Verhältnissen.

Die Isonitrilester (Komponente **C**) waren entweder kommerziell erhältlich oder wurden analog der in R. S. Bon et al, Org. Lett. 2003, 5, 20, 3759-3762 beschriebenen Methode hergestellt. In der ersten Stufe wurde zunächst eine Formylierung durchgeführt. Durch Zugabe von POCl₃ wird hieraus das Isonitril generiert.

### Methyl 2-(formylamino)-3-phenylpropanoat 2

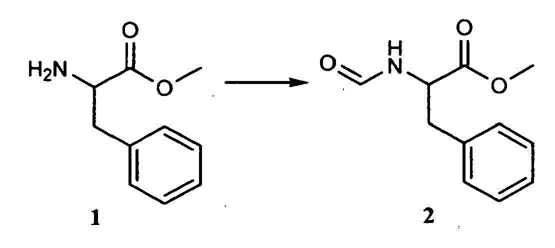

¹H-NMR (300 MHz, CDCl₃): δ = 3,07 - 3,22 (m, 2 H); 3,74 (s, 3 H); 4,92 - 5,01 (m, 1 H); 6,20 (bs, 1 H); 7,07 - 7,15 (m, 2 H); 7,23 - 7,34 (m, 3 H); 8,15 (s, 1 H).

### 2-Isocyano-3-phenyl-propionsäuremethylester 3

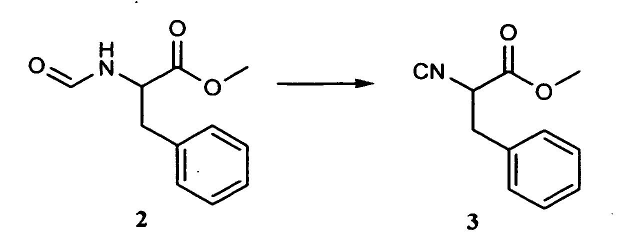

¹H-NMR (300 MHz, CDCl₃): δ = 3,14 (dd, 1 H, *J*_{AA'} = 8,66 Hz, *J*_{AB} = 8,29 Hz); 3,23 (dd, 1 H, *J*_{AA'} = 4,52 Hz, *J*_{AB} = 4,90 Hz); 3,80 (s, 3 H), 4,46 (dd, 1 H, *J* = 8,29; *J* = 4,90 Hz); 7,22 - 7,39 (m, 5 H).

### 1,4-Dioxa-spiro[4.5]decan-8-carbonsäure-ethylester 4

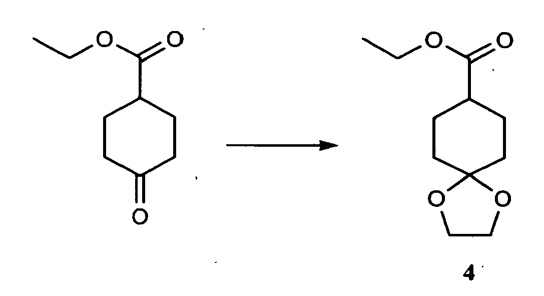

4-Oxo-cyclohexancarbonsäure-ethylester (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet (Na₂SO₄), i. Vak. eingeengt und die verbliebene farblose Flüssigkeit ohne Reinigung weiter verarbeitet.
Ausbeute: 66,5 g (100 %)
¹H-NMR (CDCl₃): 1,24 (t, 3 H); 1,53 (m, 2 H); 1,76 (m, 4 H); 1,92 (m, 2 H); 2,31 (m, 1 H); 3,91 (s, 4 H); 4,11 (q, 2 H).
¹³C-NMR (CDCl₃): 14,28 (q); 26,32 (t); 33,76 (t); 41,59 (d); 60,14 (t); 64,21 (t); 107,90 (d); 174,77 (s).

### 1,4-Dioxa-spiro[4.5]decane-8-carbaldehyd 5

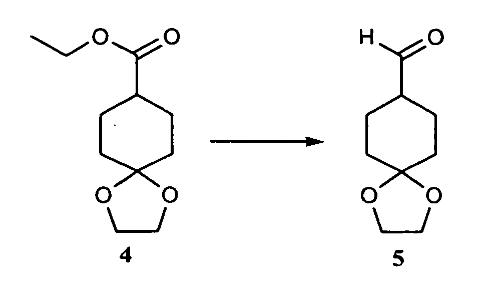

Eine Lösung aus 1,4-Dioxa-spiro[4.5]decan-8-carbonsäureethylester **4** (32,13 g, 150 mmol) in absol. Toluol (160 ml) wurde bei -70 bis -65 °C unter Argon tropfenweise mit Diisobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Rektionslösung über Kieselgur abgesaugt. Kiesegur wurde zweimal mit Essigester gewaschen, die wäßrige Lösung abgetrennt und zweimal mit Essigester extrahiert. Die vereinigten organischen Extrakte wurden mit gesättigter Natriumchlorid-Lösung gewaschen, über Natriumsulfat getrocknet und i. Vak. eingeengt.
**Ausbeute: 24,01 g (94 %), gelbes Öl**
¹H-NMR (CDCl₃): 1,54 (m, 2 H); 1,74 (m, 4 H); 1,91 (m, 2 H); 2,21 (m, 1 H); 3,91 (s, 4 H); 9,60 (s, 1 H).
¹³C-NMR (CDCl₃): 23,35 (t); 33,37 (t); 48,18 (d); 64,30 (t); 107,89 (d); 203,51 (s).

### Dimethylamino-(1,4-dioxa-spiro[4.5]dec-8-yl)-acetonitril 6

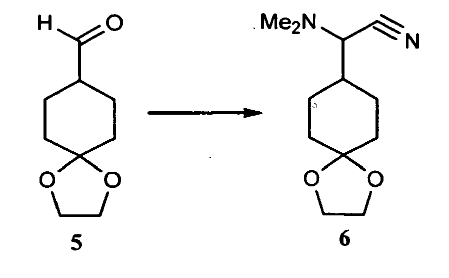

Zu einem Gemisch aus 4N Salzsäure (37 ml) und Methanol (22 ml) wurde unter Eiskühlung 40-proz. wässrige Dimethylaminlösung (85 ml, 0,67 mol), 1,4-Dioxa-spiro-[4.5]decan-8-carbaldehyd 5 (240 g, 0,141 mol) und Kaliumcyanid (22,05 g, 0,338 mol) addiert. Die Mischung wurde 4 d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.
Ausbeute: 25,2 g (81 %)
Schmelzpunkt: 48-51 °C.
¹H-NMR (CDCl₃): 1,23-2,03 (m, 9 H); 2,28 (s, 6 H); 3,16 (d, 1 H); 3,93 (m, 4 H).
¹³C-NMR (CDCl₃): 26,67 (t); 27,93 (t); 33,87 (t); 36,94 (d); 41.90 (q); 64.30 (t); 64.36 (t); 108.33 (d); 115.94 (s).

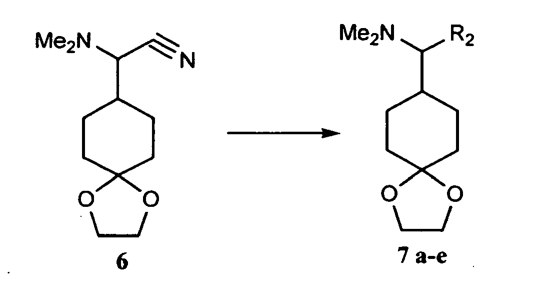

### [(1,4-Dioxa-spiro[4.5]dec-8-yl)-4-fluorphenyl-methyl]-dimethylamin 7a (R² = 4-Fluorphenyl)

Eine 1 M Lösung von 4-Fluorphenylmagnesiumbromid in THF (220 ml, 220 mmol) wurde unter Argon und Eiskühlung tropfenweise mit einer Lösung des Aminonitrils **6** (19,89 g, 88 mmol) in absol. THF (160 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet (Na₂SO₄) und eingeengt.
Ausbeute: 31 g (>100 %)
¹³C-NMR (CDCl₃): 26,68 (t); 28,11 (t); 34,43 (t); 34,55 (t); 37,37 (d); 41,68 (q); 64,12
(t); 73,65 (d); 108,88 (d); 114,23 (d); 114,44 (d); 130,27; 130,35; 132,43; 160,36 (s); 162,78 (s).

### [(1,4-Dioxa-spiro[4.5]dec-8-yl)-3-fluorphenyl-methyl]-dimethyl-amin 7b (R² = 3-Fluorphenyl)

Eine 1 M Lösung von 3-Fluorphenylmagnesiumbromid in THF (208 ml, 208 mmol) wurde unter Argon und Eiskühlung tropfenweise mit einer Lösung des Aminonitrils **6** (23,45 g, 104 mmol) in absol. THF (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt.
Ausbeute: 30,33 g (99 %).
¹H-NMR (CDCl₃): 1,12 (m, 1 H); 1,26 (m, 1 H); 1,46 - 1,81 (m, 7 H); 2,10 (s, 6 H); 3,10 (d, 1 H); 3,90 (m, 4 H); 6,85 (m, 3 H); 7,27 (m, 1 H).
¹³C-NMR (CDCl₃): 26,80 (t); 28,08 (t); 34,48 (t); 34,45 (t); 34,59 (t); 37,26 (d); 41,71 (q); 64,19 (t); 74,04 (t); 108,91 (d); 113,51 (d); 113,71 (d); 115,52 (d); 115,72 (d); 124,83 (d); 128,82 (d); 128,90 (d); 139,66 (s); 161,15 (s); 163,58 (s).

### [(4-Chlorphenyl)-(1,4-dioxa-spiro[4.5]dec-8-yl)-methyl]-dimethyl-amin 7c (R² = 4-Chlorphenyl)

Eine 1 M Lösung von 4-Chlorphenylmagnesiumbromid in Ether (200 ml, 200 mmol) wurde unter Argon und Eiskühlung tropfenweise mit einer Lösung des Aminonitrils **6** (22,43 g, 100 mmol) in absol. Ether (100 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt.
Ausbeute: 30,9 g (100 %)
¹³C-NMR (CDCl₃): 26,65 (t); 28,11 (t); 34,46 (t); 34,60 (t); 37,28 (d); 41,76 (q); 64,17 (t); 73,80 (d); 108,88 (s); 127,72 (d); 129,53 (d); 132,39 (d); 135,33 (d).

### [(1,4-Dioxa-spiro[4.5]dec-8-yl)-thiophen-2-yl-methyl]-dimethylamin 7d (R² = 2-Thienyl)

Eine 1 M Lösung von Thiophen-2-yl-magnesiumbromid in THF (20 ml, 20 mmol) wurde unter Argon und Eiskühlung tropfenweise mit einer Lösung des Aminonitrils **6** (2,24 g, 10 mmol) in absol. THF (10 ml) versetzt und 20 h bei RT gerührt. Zur Aufarbeitung des Reaktionsgemisches wurde unter Eiskühlung gesättigte Ammoniumchlorid-Lösung (10 ml) und Wasser (10 ml) gegeben und mit Diethylether (3 x 10 ml) extrahiert. Die organische Phase wurde mit Wasser und gesättigter Natriumchlorid-Lösung gewaschen, getrocknet und eingeengt.
Ausbeute: 2,8 g (100 %)
¹³C-NMR (CDCl₃): 27,72 (t); 27,88 (t); 34,27 (t); 39,28 (d); 41,10 (q); 64,11 (t); 68,89 (d); 108,88 (s); 123,55 (d); 125,88 (d); 127,53 (d); 139,50 (s).
[1-(1,4-Dioxa-spiro[4.5]dec-8-yl)-3-phenyl-propyl]-dimethylamin **7e** (R² = Phenethyl) Eine 1 M Lösung von Phenylethylmagnesiumchlorid in THF (242 ml, 242 mmol) wurde unter Argon und Eiskühlung tropfenweise mit einer Lösung des Aminonitrils **6** (21,93 g, 97 mmol) in absol. THF (180 ml) versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet und eingeengt.
Ausbeute: 34 g (>100 %).
¹³C-NMR (CDCl₃): 27,43 (t); 28,95 (t); 29,42 (t); 34,82 (t); 35,40 (t); 38,76 (d); 41,16 (q); 64,17 (t); 67,41 (d); 108,86 (s); 125,41 (d); 127,66 (d); 128,11 (d); 142,69 (s).

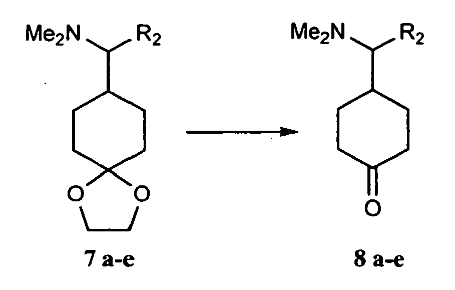

### 4-[Dimethylamino-(4-fluorphenyl)-methyl]-cyclohexanon 8a (R² = 4-Fluorphenyl)

Das Rohprodukt des Ketals **7a** (26 g, 88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit Dichlormethan (3 x 100 ml) extrahiert, getrocknet und eingeengt.
Ausbeute: 21,36 g (98 %)
¹³C-NMR (CDCl₃): 28,90 (t); 30,48 (t); 37,00 (t); 40,49 (t); 40,72 (t); 41,79 (q); 72,98 (d); 114,42 (d); 114,62 (d); 130,20 (d); 130,28 (d); 131,88 (s); 160,50 (s); 162,93 (s); 211,44 (s).

### 4-[Dimethylamino-(3-fluorphenyl)-methyl]-cyclohexanon 8b (R² = 3-Fluorphenyl)

Das Ketal **7b** (30,3 g, 103 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloser Feststoff isoliert.
Ausbeute: 22,4 g (87 %)
Schmelzpunkt: 72-75 °C.
¹³C-NMR (CDCl₃): 28,97 (t); 30,44 (t); 36,90 (t); 40,52 (t); 40,75 (t); 41,82 (q); 73,37 (d); 113,84; 114,06; 115,42; 115,62; 124,71; 129,03; 129,11; 139,00; 139,06; 161,16; 163,60; 211,40 (s).

4-[(4-Chlor-phenyl)-dimethylamino-methyl]-cyclohexanon **8c** (R² = 4-Chlorphenyl) Das Ketal **7c** (30,98 g, 100 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als Öl isoliert.
Ausbeute: 21,9 g (82 %)
¹³C-NMR (CDCl₃): 28,88 (t); 30,45 (t); 36,89 (t); 40,49 (t); 40,74 (t); 41,83 (q); 73,12 (d); 127,87 (d); 130,16 (d); 132,75 (d); 13470 (s); 211,35 (s).

### 4-(Dimethylamino-thiophen-2-yl-methyl)-cyclohexanon 8d (R² = 2-Thienyl)

Das Ketal 7d (2,80 g, 10 mmol) wurde in Wasser (4,4 ml) gelöst, mit konz. Salzsäure (6,4 ml) versetzt und 20 h bei RT gerührt. Das Reaktionsgemisch wurde mit Diethylether (2 x 10 ml) ausgeschüttelt, die wässrige Phase unter Eiskühlung mit 5N NaOH alkalisch gestellt, mit Dichlormethan (3 x 10 ml) extrahiert, getrocknet und eingeengt. Das Keton wurde als Öl isoliert.
Ausbeute: 1,79 g (75 %)
¹³C-NMR (CDCl₃): 30,02 (t); 30,18 (t); 38,84 (t); 40,29 (t); 39,28 (d); 41,17 (q); 68,24 (d); 123,88 (d); 126,01 (d); 126,34 (d); 138,77 (d); 211,49 (s).

### 4-(1-Dimethylamino-3-phenyl-propyl)-cyclohexanon 8e (R² = Phenethyl)

Das Rohprodukt des Ketals 7e (29,6 g, 97 mmol) wurde in Wasser (44 ml) gelöst, mit konz. Salzsäure (64 ml) versetzt und 20 h bei RT gerührt. Das Reaktionsgemisch wurde mit Diethylether (2 x 100 ml) ausgeschüttelt, die wässrige Phase unter Eiskühlung mit 5N NaOH alkalisch gestellt, mit Dichlormethan (3 x 100 ml) extrahiert, getrocknet und eingeengt. Das Keton wurde als farbloses Öl isoliert.
Ausbeute: 16.9 g (58 % )
¹³C-NMR (CDCl₃): 29,40 (t); 30,02 (t); 30,97 (t); 35,34 (t); 38,71 (t); 40,79 (t); 41,01 (t); 41,23 (q); 66,65 (d); 125,66 (d); 128,12 (d); 128,19 (d); 142,27 (s); 211,70 (s).

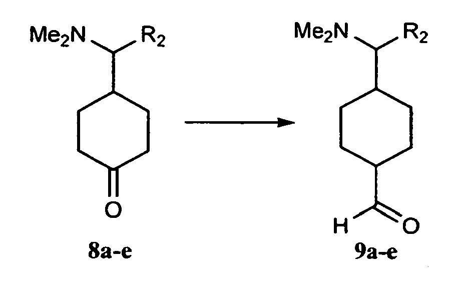

### 4-[Dimethylamino-(4-fluorphenyl)-methyl]-cyclohexan-carbaldehyd 9a (R² = 4-Fluorphenyl)

(Methoxymethyl)triphenylphosphoniumchlorid (25,7 g, 75 mmol) wurde in absol. THF (100 ml) unter Argon suspendiert, bei 0 °C tropfenweise mit Kalium-*tert*-butylat (8,42 g, 75 mmol), gelöst in absol. THF (70 ml) versetzt und anschließend 15 min bei 0 °C nachgerührt.

Bei RT wurde dann das Keton **8a** (12,44 g, 50 mmol), gelöst in absol. THF (75 ml), zur obigen Lösung zugetropft und über Nacht bei RT gerührt. Unter Eiswasserkühlung wurde tropfenweise mit Wasser (38 ml) und 6N HCl (112 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 9,13 g (70 %).
¹H-NMR (DMSO, 600 MHz, ausgesuchte Signale): δ = 1,97 (s, 3 H); 1,99 (s, 3 H); 3,08 (d, 1 H, *J* = 9,06 Hz); 3,14 (d, 1 H, *J* = 9,82 Hz); 9,53 (s, 1 H); 9,56 (s, 1 H).
¹³C-NMR (CDCl₃, beide Diastereomere): δ = 23,97; 24,21; 25,85; 26,02; 26,17; 27,35; 28,00; 29,90; 37,26; 38,34; 41,50; 41,95; 47,36; 50,55; 72,75; 75,84; 114,25; 114,45; 130,33; 130,40; 132,61; 160,41; 162,83; 204,10; 204,93.

### 4-[Dimethylamino-(3-fluorphenyl)-methyl]-cyclohexan-carbaldehyd (R² = 3-Fluorphenyl) 9b

(Methoxymethyl)triphenylphosphoniumchlorid (15,42 g, 45 mmol) wurde in absol. THF (50 ml) unter Argon suspendiert, bei 0 °C tropfenweise mit Kalium-*tert*-butylat (5,05 g, 45 mmol), gelöst in absol. THF (50 ml), versetzt und anschließend 15 min bei 0 °C nachgerührt.

Bei RT wurde dann das Keton **8b** (7,48 g, 0.30 mmol), gelöst in absol.THF (50 ml), zur obigen Lösung zugetropft und über Nacht bei RT gerührt. Unter Eiswasserkühlung wurde tropfenweise mit Wasser (25 ml) und 6N HCl (75 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) extrahiert, über Na₂SO₄ getrocknet und i. Vak. eingeengt. Das Rohprodukt wurde über durch Flashchromatographie mit Essigester/Cyclohexan (1:1) gereinigt.
Ausbeute: 6.55 g (83 %).
Schmelzpunkt: 40-43 °C.
¹H-NMR (DMSO, 600 MHz, ausgesuchte Signale): δ = 1,99 (s, 3 H); 2,01 (s, 3 H); 3,10 (d, 1 H, *J* = 9,06 Hz); 3,18 (d, 1 H, *J* = 9,82 Hz); 9,54 (s, 1 H); 9,56 (s, 1 H).
¹³C-NMR (CDCl₃): 23,93; 24,12; 25,79; 25,95; 26,19; 27,19; 27,99; 29,77; 37,05; 38,16; 41,45; 41,91; 47,30; 50,49; 71,50; 74,78; 113,50; 115,37; 124,78; 128,24; 130,59; 131,24; 131,67; 139,14; 139,76; 160,06; 163,50; 204,01; 204,85.

### 4-[(4-Chlor-phenyl)-dimethylamino-methyl]-cyclohexancarbaldehyd 9c (R² = 4-Chlorphenyl)

(Methoxymethyl)triphenylphosphoniumchlorid (68,55 g, 200 mmol) wurde in absol. THF (200 ml) unter Argon suspendiert, bei 0 °C tropfenweise mit Kalium-*tert*-butylat (22,44 g, 200 mmol), gelöst in absol. THF (300 ml), versetzt und anschließend 15 min bei 0 °C nachgerührt.

Bei RT wurde dann das Keton **8c** (38 g, 143 mmol), gelöst in absol. THF (200 ml), zur obigen Lösung getropft und über Nacht bei RT gerührt. Unter Eiswasserkühlung wurde tropfenweise mit Wasser (150 ml) und 6N HCl (450 ml) hydrolysiert. Nach 1 h Rühren bei RT wurde mit Ether (10 x 100 ml) extrahiert, die wässrige Phase mit 5N NaOH auf pH 11 gebracht, mit Essigester (3 x 100 ml) ausgeschüttelt, über Na₂SO₄ getrocknet und i. Vak. eingeengt. Das Rohprodukt wurde über zwei Kieselgelsäulen (400 g) mit Essigester/Cyclohexan (1:1) gereinigt.
Ausbeute: 32.17 g (80 %).
¹H-NMR (DMSO, 600 MHz, ausgesuchte Signale): δ = 1,97 (s, 3 H); 1,99 (s, 3 H); 3,07 (d, 1 H, *J* = 9,07 Hz); 3,14 (d, 1 H, *J* = 9,82 Hz); 9,53 (s, 1 H); 9,55 (s, 1 H).
¹³C-NMR (CDCl₃ beide Diastereomere): δ = 23,92; 24,16; 25,80; 25,97; 26,13; 27,25; 27,90; 29,81; 37,08; 38,19; 41,47; 41,96; 47,29; 50,48; 72,81; 74,54 ; 127,65 ; 130,28; 132,40; 134,78; 135,43 ; 203.98; 204.82.

4-(Dimethylamino-thiophen-2-yl-methyl)-cyclohexancarbaldehyd **9d** (R² = 2-Thienyl) (Methoxymethyl)triphenylphosphoniumchlorid (20,56 g, 60 mmol) wurde in absol. THF (70 ml) unter Argon suspendiert, bei 0 °C tropfenweise mit Kalium-*tert*-butylat (6,73 g, 60 mmol), gelöst in absol. THF (70 ml), versetzt und anschließend 15 min bei 0 °C nachgerührt Bei RT wurde dann das Keton **8d** (9,4 g, 40 mmol), gelöst in absol. THF (70 ml), zur obigen Lösung zugetropft und über Nacht bei RT gerührt. Unter Eiswasserkühlung wurde tropfenweise mit Wasser (60 ml) und 6 N HCl (180 ml) hydrolysiert. Nach 1 h Rühren bei RT wurde mit Ether (5 x 50 ml) extrahiert, die wässrige Phase mit 5 N NaOH auf pH 11 gebracht, mit Essigester (3 x 50 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 7.66 g (77 %).
¹H-NMR (DMSO, 600 MHz, ausgesuchte Signale): δ = 2,03 (s, 3 H); 2,05 (s, 3 H); 3,44 (d, 1 H, *J* = 9,82 Hz); 3,52 (d, 1 H, *J* = 10,58 Hz); 9,54 (s, 1 H); 9,58 (s, 1 H).
¹³C-NMR (CDCl₃, beide Diastereomere): δ = 23,74; 23,83; 25,80; 25,84; 26,98; 27,09; 29,15; 29,68; 39,13; 40,20; 40,98; 41,29 (N(CH₃)₂); 47,48; 50,49; 67,81; 69,79; 123,61; 123,70; 125,89; 126,20; 126,24; 139,14; 139,48; 204,07; 204,82.

4-(1-Dimethylamino-3-phenyl-propyl)-cyclohexan-carbaldehyd **9e** (R² = Phenethyl) (Methoxymethyl)triphenylphosphoniumchlorid (20,56 g, 60 mmol) wurde in absol. THF (85 ml) unter Argon suspendiert, bei 0 °C tropfenweise mit Kalium-*tert*-butylat (6,73 g, 60 mmol), gelöst in absol. THF (70 ml) versetzt und anschließend 15 min bei 0 °C nachgerührt.
Bei RT wurde dann das Keton **8e** (10,2 g, 40 mmol), gelöst in absol. THF (60 ml), zur obigen Lösung getropft und über Nacht bei RT gerührt. Unter Eiswasserkühlung wurde tropfenweise mit Wasser (35 ml) und 6N HCl (90 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) extrahiert, über Na₂SO₄ getrocknet und i. Vak. eingeengt. Das Rohprodukt wurde durch Flashchromatographie mit Essigester / Cyclohexan (1:1) gereinigt.
Ausbeute: 6.73 g (63 %).
¹H-NMR (DMSO, 600 MHz, ausgesuchte Signale): δ = 2,18 (s, 3 H); 2,20 (s, 3 H Dichlormethan); 9,54 (s, 1 H); 9,61 (s, 1 H).
¹³C-NMR (CDCl₃, beide Diastereomere): δ = 24,35; 24,49; 26,00; 26,09; 26,85; 27,79; 29,07; 29,13; 35,27; 39,02; 40,98; 41,19; 46,99; 50,33; 66,85; 67,85; 70,54; 71,42; 125,40; 125,44; 128,02; 128,13; 131,15; 131,17 ; 142,45; 204,10; 205,01.

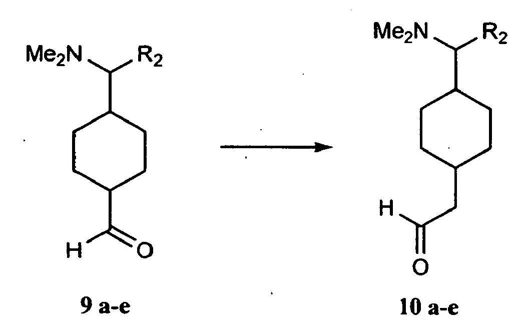

### {4-[Dimethylamino-(4-fluorphenyl)-methyl]-cyclohexyl}-acetaldehyd 10a (R² = 4-Fluorphenyl)

(Methoxymethyl)triphenylphosphoniumchlorid (43,53 g, 127 mmol) wurde in absol. TH F (200 ml) unter Argon suspendiert, bei 0 °C tropfenweise mit Kalium-*tert*-butylat (14,25 g, 127 mmol), gelöst in absol. THF (130 ml), versetzt und anschließend 15 min bei 0 °C nachgerührt.
Bei RT wurde dann der Aldehyd **9a** (22,3 g, 85 mmol), gelöst in absol. THF (130 ml), zugetropft und über Nacht bei RT gerührt. Unter Eiswasserkühlung wurde tropfenweise mit Wasser (80 ml) und 6N HCl (200 ml) hydrolysiert. Nach 1 h Rühren bei RT wurde zehnmal mit Ether (je 100 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 15,8 g (67 %)
¹³C-NMR (CDCl₃, beide Diastereomere): δ = 25,08; 25,87; 28,80; 29,10; 29,13; 29,62; 30,82; 32,90; 33,08; 36,19; 38,43; 41,36; 42,01; 47,94; 51,17; 71,11; 74,69; 114,11; 114,20; 114,32; 130,32; 130,40; 132,00; 132,92; 160,31; 162,74; 202,15; 202,23.

### {4-[Dimethylamino-(3-fluorphenyl)-methyl]-cyclohexyl}-acetaldehyd 10b (R² = 3-Fluorphenyl)

(Methoxymethyl)triphenylphosphoniumchlorid (26,73 g, 78 mmol) wurde in absol. THF (90 ml) unter Argon suspendiert, bei 0 °C tropfenweise mit Kalium-*tert*-butylat (8,75 g, 78 mmol), gelöst in absol. THF (90 ml), versetzt und anschließend 15 min bei 0 °C nachgerührt.
Bei RT wurde dann der Aldehyd **9b** (13,69 g, 52 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 12.61 g (87 %)
¹³C-NMR (CDCl₃, beide Diastereomere): δ = 25,19; 25,83; 28,90; 29,06; 29,14; 29,68; 30,77; 32,92; 32,98; 33,10; 36,05; 38,36; 41,39; 42,04; 48,02; 51,20; 71,48; 75,07; 113,43; 113,49; 113,64; 113,69; 115,55; 115,76; 124,89; 128,70; 128,78; 128,88; 139,24; 140,08; 140,14; 161,09; 163,52; 202,19; 202,27.

### {4-[(4-Chlorphenyl)-dimethylamino-methyl]-cyclohexyl}-acetaldehyd 10c (R² = 4-Chlorphenyl)

Methoxymethyl)triphenylphosphoniumchlorid (25,02 g, 73 mmol) wurde in absol. THF (90 ml) unter Argon suspendiert, bei 0 °C tropfenweise mit Kalium-*tert*-butylat (8,19 g, 73 mmol), gelöst in absol. THF (90 ml), versetzt und anschließend 15 min bei 0 °C nachgerührt.
Bei RT wurde dann der Aldehyd 9c (13,86 g, 49 mmol), gelöst in absol. THF (90 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:1) gereinigt.
Ausbeute: 12.07 g (84 %).
¹³C-NMR (CDCl₃, beide Diastereomere): δ = 25,06; 25,82; 28,74; 29,00; 29,13; 29,60; 30,77; 32,87; 32,94; 33,07; 36,06; 38,32; 41,38; 42,05; 47,95; 51,17; 71,23; 74,80; 127,58; 127,66; 130,31; 132,28; 132,34; 134,81; 135,77; 202,12; 202,20.

### {4-[Dimethylamino-thiophen-2-yl-methyl]-cyclohexyl}-acetaldehyd 10d (R² = 2-Thienyl)

(Methoxymethyl)triphenylphosphoniumchlorid (28,79 g, 84 mmol) wurde in absol. THF (100 ml) unter Argon suspendiert, bei 0 °C tropfenweise mit Kalium-*tert*-butylat (9,42 g, 84 mmol), gelöst in absol. THF (100 ml), versetzt und anschließend 15 min bei 0 °C nachgerührt.

Bei RT wurde dann der Aldehyd **9d** (14,08 g, 56 mmol), gelöst in absol. THF (100 ml), zugetropft und über Nacht bei RT gerührt. Unter Eiswasserkühlung wurde tropfenweise mit Wasser (50 ml) und 6N HCl (150 ml) hydrolysiert. Nach 1 h Rühren bei RT wurde zehnmal mit Ether (je 50 ml) extrahiert. Die wässrige Phase wurde mit 5N NaOH auf pH 11 gebracht, dreimal mit Essigester (je 100 ml) ausgeschüttelt, über Na₂SO₄ getrocknet und i. Vak. eingeengt. Das Rohprodukt wurde durch Flashchromatographie mit Essigester/Cyclohexan (1:2) gereinigt.
Ausbeute: 11.48 g (77 %).
¹³C-NMR (CDCl₃, beide Diastereomere): δ = 25,80; 25,88; 28,73; 29,95; 30,49, 32,23; 32,76; 37,89; 40,21; 40,88; 41,23; 48,36; 51,09; 66,02; 69,97; 123,19; 123,72; 125,95; 126,31; 139,42; 139,91; 202,61.

[4-(1-Dimethylamino-3-phenyl-propyl)-cyclohexyl]-acetaldehyd **10e** (R² = Phenethyl) (Methoxymethyl)triphenylphosphoniumchlorid (50,3 g, 147 mmol) wurde in absol. THF (150 ml) unter Argon suspendiert, bei 0 °C tropfenweise mit Kalium-*tert*-butylat (16,5 g, 147 mmol), gelöst in absol. THF (140 ml), versetzt und anschließend 15 min bei 0 °C nachgerührt.
Bei RT wurde dann der Aldehyd **9e** (27,0 g, 98 mmol), gelöst in absol. THF (150 ml), zugetropft und über Nacht bei RT gerührt. Unter Eiswasserkühlung wurde tropfenweise mit Wasser (102 ml) und 6N HCl (240 ml) hydrolysiert. Nach 1 h Rühren bei RT wurde fünfmal mit Ether (je 200 ml) extrahiert. Die wässrige Phase wurde unter Eiskühlung mit 5 N NaOH auf pH 11 gebracht, dreimal mit Essigester (je 200 ml) ausgeschüttelt, über Na₂SO₄ getrocknet und i. Vak. eingeengt. Das Rohprodukt wurde durch Flash-chromatographie mit Essigester/Cyclohexan (1:1) gereinigt.
Ausbeute: 18,1 g (64 %)
¹³C-NMR (CDCl₃, beide Diastereomere): δ = 25,55; 26,19; 29,04; 29,15; 29,35; 29,85; 31,00; 32,87; 32,68; 33,04; 35,33; 38,49; 40,86; 41,13; 47,51; 51,15; 65,48; 68,09.

### Beispiele:

- 9a: 4-[Dimethylamino-(4-fluor-phenyl)-methyl]-cyclohexancarbaldehyd
- 9b: 4-[Dimethylamino-(3-fluor-phenyl)-methyl]-cyclohexanecarbaldehyd
- 9c: 4-[(4-Chlor-phenyl)-dimethylamino-methyl]-cyclohexanecarbaldehyd
- 9d: 4-(Dimethylamino-thiophen-2-yl-methyl)-cyclohexanecarbaldehyd
- 9e: 4-(1-Dimethylamino-3-phenyl-propyl)-cyclohexanecarbaldehyd
- 10a: {4-[Dimethylamino-(4-fluor-phenyl)-methyl]-cyclohexyl}-acetaldehyd
- 10b: {4-[Dimethylamino-(3-fluor-phenyl)-methyl]-cyclohexyl}-acetaldehyd
- 10c: {4-[(4-Chlor-phenyl)-dimethylamino-methyl]-cyclohexyl}-acetaldehyd
- 10d: [4-(Dimethylamino-thiophen-2-yl-methyl)-cyclohexyl]-acetaldehyd
- 10e: [4-(1-Dimethylamino-3-phenyl-propyl)- cyclohexyl]-acetaldehyd

Die Synthese der erfindungsgemäßen Imidazolin-Derivaten erfolgte mit Hilfe des Accelerators SLT106 der Firma Chemspeed Ltd.

### Synthesevorschrift für die automatisierte Synthese

In einen trockenen 13 ml double jacket-Glasreaktor (Chemspeed) wurden bei Raumtemperatur 220 µmol Amin-Derivat (Lösung II, 1,1 ml, 0,2 M in Methanol) vorgelegt und mit 220 µmol Aldehyd-Derivat (Lösung I, 1,1 ml, 0, 2 M im Methanol) und 110 µmol Isonitril-Derivat (Lösung III, 1 ml, 0,1 M in Methanol) versetzt. Anschließend wurde 10 h unter Rückfluss gekocht. Nach beendeter Reaktion wurde das Lösungsmittel entfernt. Die Aufreinigung erfolgte mittels HPLC. Die Aufreinigung erfolgte über HPLC-MS. In allen aufgeführten Fällen wurde die exakte Masse als M+1 gefunden.

### Beispiele:

| **Nr.** | **Name** | **Masse** |
|---|---|---|
| 11. | 1-Cyclohexyl-5-{4-[dimethylamino-(3-fluor-phenyl)-methyl]-cyclohexyl}-4-isobutyl-4,5-dihydro-1H-imidazol-4-carbonsäuremethylester | 499,36 |
| 12. | 4-(4-Chlor-benzyl)-1-cyclohexyl-5-{4-[dimethylamino-(3-fluor-phenyl)-methyl]-cyclohexyl}-4,5-dihydro-1H-imidazol-4-carbonsäuremethylester | 567,30 |
| 13. | 4-Butyl-1-cyclohexyl-5-{4-[dimethylamino-(3-fluor-phenyl)-methyl]-cyclohexyl}-4,5-dihydro-1H-imidazol-4-carbonsäuremethylester | 499,36 |
| 14. | 5-{4-[(4-Chlor-phenyl)-dimethylamino-methyl]-cyclohexyl}-1-cyclohexyl-4-isobutyl-4,5-dihydro-1H-imidazol-4-carboxylic acid methyl ester | 515,33 |
| 15. | 4-(4-Chlor-benzyl)-5-{4-[(4-chlor-phenyl)-dimethylamino-methyl]-cyclohexyl}-1-cyclohexyl-4,5-dihydro-1H-imidazol-4-carbonsäuremethylester | 583,27 |
| 16. | 4-Butyl-5-{4-[(4-chlor-phenyl)-dimethylamino-methyl]-cyclohexyl}-1-cyclohexyl-4,5-dihydro-1H-imidazol-4-carboxylic acid methyl ester | 515,33 |
| 17. | 5-{4-[(4-Chlor-phenyl)-dimethylamino-methyl]-cyclohexylmethyl}-1-cyclohexyl-4-isobutyl-4,5-dihydro-1H-imidazol-4-carbonsäuremethylester | 529,34 |
| 18. | 4-(4-Chlor-benzyl)-5-{4-[(4-chlor-phenyl)-dimethylamino-methyl]-cyclohexylmethyl}-1-cyclohexyl-4,5-dihydro-1H-imidazol-4-carbonsäuremethylester | 597,29 |
| 19. | 4-Butyl-5-{4-[(4-chlor-phenyl)-dimethylamino-methyl]-cyclohexylmethyl}-1-cyclohexyl-4,5-dihydro-1H-imidazol-4-carbonsäuremethylester | 529,34 |
| 20. | 4-(4-Chlor-benzyl)-1-cyclohexyl-5-[4-(dimethylamino-thiophen-2-yl-methyl)-cyclohexylmethyl]-4,5-dihydro-1H-imidazol-4-carbonsäuremethylester | 569,28 |
| 21. | 5-{4-[Dimethylamino-(3-fluor-phenyl)-methyl]-cyclohexyl}-4-isobutyl-1-propyl-4,5-dihydro-1H-imidazol-4-carbonsäuremethylester | 459,33 |
| 22. | 4-(4-Chlor-benzyl)-5-{4-[dimethylamino-(3-fluor-phenyl)-methyl]-cyclohexyl}-1-propyl-4,5-dihydro-1H-imidazol-4carbonsäuremethylester | 527,27 |
| 23. | 4-Butyl-5-{4-[dimethylamino-(3-fluor-phenyl)-methyl]-cyclohexyl}-1-propyl-4,5-dihydro-1H-imidazol-4-carbonsäuremethylester | 459,33 |
| 24. | 5-{4-[(4-Chlor-phenyl)-dimethylamino-methyl]-cyclohexyl}-4-isobutyl-1-propyl-4,5-dihydro-1H-imidazol-4-carbonsäuremethylester | 475,30 |
| 25. | 4-(4-Chlor-benzyl)-5-{4-[(4-chlor-phenyl)-dlmethylamino-methyl]-cyclohexyl}-1-propyl-4,5-dihydro-1H-imidazol-4-carbonsäuremethylester | 543,2 |
| 26. | 5-{4-[(4-Chlor-phenyl)-dimethylamino-methyl]-cyclohexylmethyl}-4-isobutyl-1-propyl-4,5-dihydro-1H-imidazol-4-carbonsäuremethylester | 489,31 |
| 27. | 4-(4-Chlorbenzyl)-5-{4-[(4-chlor-phenyl)dimethylamino-methyl]-cyclohexylmethyl}-1-propyl-4,5-dihydro-1H-imidazol-4-carbonsäuremethylester | 557,26 |
| 28. | 4-Butyl-5-{4-[(4-chlor-phenyl)-dimethylamino-methyl]-cyclohexylmethyl}-1-propyl-4,5-dihydro-1H-imidazol-4-carbonsäuremethylester | 489,31 |
| 29. | 1-Butyl-5-{4-[dimethylamino-(3-fluor-phenyl)-methyl]-cyclohexyl}-4-isobutyl-4,5-dihydro-1H-imidazol-4-carbonsäuremethylester | 473,34 |
| 30. | 1-Butyl-4-(4-chlor-benzyl)-5-{4-[dimethylamino-(3-fluor-phenyl)-methyl]-cyclohexyl}-4,5-dihydro-1H-imidazol-4-carbonsäuremethylester | 541 29 |
| 31. | 1-Butyl-5-{4-[(4-chlor-phenyl)-dimethylamino-methyl]-cyclohexyl}-4-isobutyl-4,5-dihydro-1H-imidazol-4-carbonsäuremethylester | 489,31 |
| 32. | 1-Butyl-4-(4-chlor-benzyl)-5-{4-[(4-chlor-phenyl)dimethylamino-methyl]-cyclohexyl}-4,5-dihydro-1H-imidazol-4-carbonsäuremethylester | 557,26 |
| 33. | 1,4-Dibutyl-5-{4-[(4-chlor-phenyl)-dimethylamino-methyl]-cyclohexyl}-4,5-dihydro-1H-imidazol-4-carbonsäuremethylester | 489,31 |
| 34. | 1-Butyl-5-{4-[(4-chlor-phenyl)-dimethylamino-methyl]-cyclohexylmethyl}-4-isobutyl-4,5-dihydro-1H-imidazol-4-carbonsäuremethylester | 503,33 |
| 35. | 1-Butyl-4-(4-chlor-benzyl)-5-{4-[(4-chlor-phenyl)-dimethylamino-methyl]-cyclohexylmethyl}-4,5-dihydro-1H-imidazol-4-carboxylic acid methyl ester | 571,27 |
| 36. | 1,4-Dibutyl-5-{4-[(4-chlor-phenyl)-dimethylamino-methyl]-cyclohexylmethyl}-4,5-dihydro-1H-imidazol-4-carbonsäuremethylester | 503,33 |
| 37. | 5-{4-[Dimethylamino-(3-fluor-phenyl)-methyl]-cyclohexyl}-4-isobutyl-1-phenethyl-4,5-dihydro-1H-imidazol-4-carbonsäuremethylester | 521,34 |
| 38. | 4-(4-Chlor-benzyl)-5-{4-[dimethylamino-(3-fluor-phenyl)-methyl]-cyclohexyl}-1-phenethyl-4,5-dihydro-1H-imidazol-4-carbonsäuremethylester | 589,29 |
| 39. | 4-Butyl-5-{4-[dimethylamino-(3-fluor-phenyl)-methyl]-cyclohexyl}-1-phenethyl-4,5-dihydro-1H-imidazol-4-carbonsäuremethylester | 521,34 |
| 40. | 5-{4-[(4-Chlor-phenyl)-dimethylamino-methyl]-cyclohexyl}-4-isobutyl-1-phenethyl-4,5-dihydro-1H-imidazol-4-carbonsäuremethylester | 537,31 |
| 41. | 4-(4-Chlor-benzyl)-5-{4-[(4-chlor-phenyl)-dimethylamino-methyl]-cyclohexyl}-1-phenethyl-4,5-dihydro-1H-imidazol-4-carbonsäuremethylester | 605,26 |
| 42. | 4-Butyl-5-{4-[(4-chlor-phenyl)-dimethylamino-methyl]-cyclohexyl}-1-phenethyl-4,5-dihydro-1H-imidazol-4-carbonsäuremethylester | 537,31 |
| 43. | 5-{4-[(4-Chlor-phenyl)-dimethylamino-methyl]-cyclohexylmethyl}-4-isobutyl-1-phenethyl-4,5-dihydro-1H-imidazol-4carbonsäuremethylester | 551,33 |
| 44. | 4-(4-Chlor-benzyl)-5-{4-[(4-chlor-phenyl)-dimethylamino-methyl]-cyclohexylmethyl}-1-phenethyl-4,5-dihydro-1H-imidazol-4-carbonsäuremethylester | 619,27 |
| 45. | 4-Butyl-5-{4-[(4-chlor-phenyl)-dimethylamino-methyl]-cyclohexylmethyl}-1- phenethyl-4,5-dihydro-1H-imidazol-4-carbonsäuremethylester | 551,33 |
| 46. | 1-Benzyl-5-{4-[dimethylamino-(3-fluor-phenyl)-methyl]-cyclohexyl}-4-isobutyl-4,5-dihydro-1H-imidazol-4-carbonsäuremethylester | 507,33 |
| 47. | 1-Benzyl-4-(4-chlor-benzyl)-5-{4-[dimethylamino-(3-fluor-phenyl)-methyl]-cyclohexyl}-4,5-dihydro-1H-imidazol-4-carbonsäuremethylester | 575,27 |
| 48. | 1-Benzyl-5-{4-[(4-chlor-phenyl)-dimethylamino-methyl]-cyclohexyl}4- isobutyl-4,5-dihydro-1H-imidazol-4-carbonsäuremethylester | 523,30 |
| 49. | 1-Benzyl-4-(4-chlor-benzyl)-5-{4-[(4-chlor-phenyl)-dimethylamino-methyl]- cyclohexyl}-4,5-dihydro-1H-imidazol-4-carbonsäuremethylester | 591,24 |
| 50. | 1-Benzyl-5-{4-[(4-chlor-phenyl)-dimethylamino-methyl]-cyclohexylmethyl}-4- isobutyl-4,5-dihydro-1H-imidazol-4-carbonsäuremethylester | 537,31 |
| 51. | 5-{4-[(4-Chlor-phenyl)-dimethylamino-methyl]-cyclohexyl}-1-cyclohexyl-4-(2-methoxycarbonyl-ethyl)-4,5-dihydro-1H-imidazol-4-carbonsäuremethylester | 545,30 |
| 52. | 4-Benzylsulfanylmethyl-5-{4-[(4-chlor-phenyl)-dimethylamino-methyl]-cyclohexyl}-1-cyclohexyl-4,5-dihydro-1H-imidazol-4-carbonsäuremethylester | 595,30 |
| 53. | 5-{4-[(4-Chlor-phenyl)-dimethylamino-methyl]-cyclohexylmethyl}-1-cyclohexyl-4-(2-methoxycarbonyl-ethyl)-4,5-dihydro-1H-imidazol-4-carbonsäuremethylester | 559,32 |
| 54. | Methyl-4-sec-butyl-5-((4-((4-chlorphenyl)(dimethylamino)methyl)cyclohexyl)methyl)-1-cyclohexyl-4,5-dihydro-1H-imidazol-4-carboxylat | 529,34 |
| 55. | 5-{4-[(4-Chlor-phenyl)-dimethylamino-methyl]-cyclohexylmethyl}-1-cyclohexyl-4-methylsulfanylmethyl-4,5-dihydro-1H-imidazol-4-carbonsäuremethylester | 533,28 |
| 56. | 4-Benzylsulfanylmethyl-5-{4-[(4-chlor-phenyl)-dimethylamino-methyl]-cyclohexylmethyl}-1-cyclohexyl-4,5-dihydro-1H-imidazol-4-carbonsäuremethylester | 609,32 |
| 57. | 5-{4-[Dimethylamino-(3-fluor-phenyl)-methyl]-cyclohexyl}-4-methylsulfanylmethyl-1-propyl-4,5-dihydro-1H-imidazol-4-carbonsäuremethylester | 463,27 |
| 58. | 4-Benzylsulfanylmethyl-5-{4-[dimethylamino-(3-fluor-phenyl)-methyl]-cyclohexyl}-1-propyl-4,5-dihydro-1H-imidazol-4-carbonsäuremethylester | 539,30 |
| 59. | 5-{4-[(4-Chlor-phenyl)-dimethylamino-methyl]-cyclohexyl}-4-(2-methoxycarbonyl-ethyl)-1-propyl-4,5-dihydro-1H-imidazol-4-carbonsäuremethylester | 505,27 |
| 60. | 4-Benzylsulfanylmethyl-5-{4-[(4-chlor-phenyl)-dimethylamino-methyl]-cyclohexyl}1-propyl-4,5-dihydro-1H-imidazol-4-carbonsäuremethylester | 555,27 |
| 61. | 5-{4-[(4-Chlor-phenyl)-dimethylamino-methyl]-cyclohexylmethyl}-4-(2-methoxycarbonyl-ethyl)-1-propyl-4,5-dihydro-1H-imidazol-4-carbonsäuremethylester | 519,29 |
| 62. | 4-sec-Butyl-5-{4-[(4-chlor-phenyl)-dimethylamino-methyl]-cyclohexylmethyl}-1-propyl-4,5-dihydro-1H-imidazol-4-carbonsäuremethylester | 489,31 |
| 63. | 5-{4-[(4-Chlor-phenyl)-dimethylamino-methyl]-cyclohexylmethyl}-4-methylsulfanylmethyl-1-propyl-4,5-dihydro-1H-imidazol-4-carbonsäuremethylester | 493,25 |
| 64. | 4-Benzylsulfanylmethyl-5-{4-[(4-chlor-phenyl)-dimethylamino-methyl]-cyclohexylmethyl}-1-propyl-4,5-dihydro-1H-imidazol-4-carbonsäuremethylester | 569,28 |
| 65. | 1-Butyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester | 503,32 |
| 66. | 1-Butyl-5-{4-[dimethylamino-(3-fluor-phenyl)-methyl]-cyclohexyl}-4-methylsulfanylmethyl-4,5-dihydro-1H-imidazol-4-carbonsäuremethylester | 477,28 |
| 67. | 4-Benzylsulfanylmethyl-1-butyl-5-{4-[dimethylamino-(3-fluor-phenyl)-methyl]-cyclohexyl}-4,5-dihydro-1H-imidazol-4-carbonsäuremethylester | 553,31 |
| 68. | 1-Butyl-5-{4-[(4-chlor-phenyl)-dimethylamino-methyl]-cyclohexyl}-4-(2-methoxycarbonyl-ethyl)-4,5-dihydro-1H-imidazole-4-carbonsäuremethylester | 519,29 |
| 69. | 4-Benzylsulfanylmethyl-1-butyl-5-{4-[(4-chlor-phenyl)-dimethylamino-methyl]-cyclohexyl}-4,5-dihydro-1H-imidazol-4-carbonsäuremethylester | 569,28 |
| 70. | 1-Butyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester | 533,30 |
| 71. | 5-[4-(1-Dimethylamino-3-phenyl-propyl)-cyclohexylmethyl]-4-(2-methoxycarbonyl-ethyl)-1-propyl-4,5-dihydro-1H-imidazol-4-carbonsäuremethylester | 513,36 |
| 72. | 1-Butyl-5-{4-[dimethylamino-(3-fluor-phenyl)-methyl]-cyclohexylmethyl}-4-(2- methoxycarbonyl-ethyl)-4,5-dihydro-1H-imidazol-4-carbonsäuremethylester | 517,33 |
| 73. | 1-Butyl-4-sec-butyl-5-{4-[dimethylamino-(3-fluor-phenyl)-methyl]-cyclohexylmethyl}-4,5-dihydro-1H-imidazol-4-carbonsäuremethylester | 487,36 |
| 74. | 1-Butyl-5-{4-[dimethylamino-(3-fluor-phenyl)-methyl]-cydohexylmethyl}4-methylsulfanylmethyl-4,5-dihydro-1H-imidazol-4-carbonsäuremethylester | 491,30 |
| 75. | 4-Benzylsulfanylmethyl-1-butyl-5-{4-[dimethylamino-(3-fluor-phenyl)-methyl]-cyclohexylmethyl}-4,5-dihydro-1H-imidazol-4-carbonsäuremethylester | 567,33 |
| 76. | 1-Butyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester | 517,33 |
| 77. | 1-Butyl-5-{4-[dimethylamino-(4-fluor-phenyl)-methyl]-cyclohexylmethyl}-4-methylsulfanylmethyl-4,5-dihydro-1H-imidazol-4-carbonsäuremethylester | 491,30 |
| 78. | 1-Butyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester | 527,37 |
| 79. | 4-Benzylsulfanylmethyl-1-butyl-5-[4-(1-dimethylamino-3-phenyl-propyl)-cyclohexylmethyl]-4,5-dihydro-1H-imidazol-4-carbonsäuremethylester | 577,37 |
| 80. | 5-{4-[Dimethylamino-(3-fluor-phenyl)-methyl]-cyclohexylmethyl}-4-(2-methoxycarbonyl-ethyl)-1-phenethyl-4,5-dihydro-1H-imidazol-4-carbonsäuremethylester | 565,33 |
| 81. | 4-sec-Butyl-5-{4-[dimethylamino-(3-fluor-phenyl)-methyl]-cyclohexylmethyl}-1-phenethyl-4,5-dihydro-1H-imidazol-4-carbonsäuremethylester | 535,36 |
| 82. | 4-Benzylsulfanylmethyl-5-{4-[dimethylamino-(3-fluor-phenyl)-methyl]-cyclohexylmethyl}-1-phenethyl-4,5-dihydro-1H-imidazol-4-carbonsäuremethylester | 615,33 |
| 83. | 5-{4-[Dimethylamino-(4-fluor-phenyl)-methyl]-cyclohexylmethyl}-4-(2-methoxycarbonyl-ethyl)-1-phenethyl-4,5-dihydro-1H-imidazol-4-carbonsäuremethylester | 565,33 |
| 84. | 5-[4-(1-Dimethylamino-3-phenyl-propyl)-cyclohexylmethyl]-4-(2-methoxycarbonyl-ethyl)-1-phenethyl-4,5-dihydro-1H-imidazol-4-carbonsäuremethylester | 575,37 |
| 85. | 1-Benzyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester | 551,32 |
| 86. | 1-Benzyl-4-benzylsulfanylmethyl-5-{4-[dimethylamino-(3-fluor-phenyl)-methyl]-cyclohexylmethyl}-4,5-dihydro-1H-imidazol-4-carbonsäuremethylester | 601,31 |
| 87. | 1-Benzyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester | 551,32 |
| 88. | 1-Butyl-4-sec-butyl-5-{4-[(4-chlor-phenyl)-dimethylamino-methyl]-cyclohexylmethyl}-4,5-dihydro-1H-imidazol-4-carbonsäuremethylester | 503,33 |
| 89. | 1-Butyl-5-{4-[(4-chlr-phenyl)-dimethylamino-methyl]-cyclohexylmethyl}-4-methylsulfanylmethyl-4,5-dihydro-1H-imidazol-4-carbonsäuremethylester | 507,27 |
| 90. | 4-Benzylsulfanylmethyl-1-butyl-5-{4-[(4-chlor-phenyl)-dimethylamino-methyl]-cyclohexylmethyl}-4,5-dihydro-1H-imidazol-4-carbonsäuremethylester | 583,30 |
| 91. | 5-{4-[Dimethylamino-(3-fluor-phenyl)-methyl]-cyclohexyl}-4-(2-methoxycarbonyl-ethyl)-1-phenethyl-4,5-dihydro-1H-imidazol-4-carbonsäuremethylester | 551,32 |
| 92. | 4-Benzylsulfanylmethyl-5-{4-[dimethylamino-(3-fluor-phenyl)-methyl]-cyclohexyl}-1-phenethyl-4,5-dihydro-1H-imidazol-4-carbonsäuremethylester | 601,31 |
| 93. | 4-Benzylsufanylmethyl-5-{4-[(4-chlor-phenyl)-dimethylamino-methyl]-cyclohexyl}-1-phenethyl-4,5-dihydro-1H-imidazol-4-carbonsäuremethylester | 617,28 |
| 94. | 5-{4-[(4-Chlor-phenyl)-dimethylamino-methyl]-cyclohexylmethyl}-4-(2-methoxycarbonyl-ethyl)-1-phenethyl-4,5-dihydro-1H-imidazol-4-carbonsäuremethylester | 581,30 |
| 95. | 1-Benzyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester | 537,30 |
| 96. | 1-Benzyl-5-{4-[dimethylamino-(3-fluor-phenyl)-methyl]-cyclohexyl}-4-methylsulfanylmethyl-4,5-dihydro-1H-imidazol-4-carbonsäuremethylester | 511,27 |
| 97. | 1-Benzyl-4-benzylsulfanylmethyl-5-{4-[dimethylamino-(3-fluor-phenyl)-methyl]-cyclohexyl}-4,5-dihydro-1H-imidazol-4-carbonsäuremethylester | 587,30 |
| 98. | 1-Benzyl-4-benzylsulfanylmethyl-5-{4-[(4-chlor-phenyl)-dimethylamino-methyl]-cyclohexyl}-4,5-dihydro-1H-imidazol-4-carbonsäuremethylester | 603,27 |
| 99. | 1-Benzyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester | 567,29 |
| 100. | 1-Benzyl-5-{4-[(4-chlor-phenyl)-dimethylamino-methyl]-cyclohexylmethyl}-4-methylsulfanylmethyl-4,5-dihydro-1H-imidazol-4-carbonsäuremethylester | 541,25 |
| 101. | 1-Benzyl-4-benzylsulfanylmethyl-5-{4-[(4-chlor-phenyl)-dimethylamino-methyl]-cyclohexylmethyl}-4,5-dihydro-1H-imidazol-4-carbonsäuremethylester | 617,28 |
| 102. | 1-Cyclohexyl-5-{4-[dimethylamino-(3-fluor-phenyl)-methyl]-cyclohexylmethyl}-4-propyl-4,5-dihydro-1H-imidazol-4-carbonsäuremethylester | 499,36 |
| 103. | 1-Cyclohexyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester | 531,33 |
| 104. | 1-Cyclohexyl-5-{4-[dimethylamino-(3-fluor-phenyl)-methyl]-cyclohexylmethyl}-4-methyl-4,5-dihydro-1H-imidazol-4-carbonsäuremethylester | 471,33 |
| 105. | 1-Cyclohexyl-5-{4-[dimethylamino-(4-fluor-phenyl)-methyl]-cyclohexylmethyl}-4-propyl-4,5-dihydro-1H-imidazol-4-carbonsäuremethylester | 499,36 |
| 106. | 1-Cyclohexyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester | 531,33 |
| 107. | 1-Cyclohexyl-5-{4-[dimethylamino-(4-fluor-phenyl)-methyl]-cyclohexylmethyl}-4-methyl-4,5-dihydro-1H-imidazol-4-carbonsäuremethylester | 471,33 |
| 108. | 1-Cyclohexyl-5-[4-(1-dimethylaminor3-phenyl-propyl)-cyclohexylmethyl]-4-propyl-4,5-dihydro-1H-imidazol-4-carbonsäuremethylester | 509,40 |
| 109. | 1-Cyclohexyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester | 541,37 |
| 110. | 1-Cyclohexyl-5-[4-(1-dimethylamino-3-phenyl-propyl)-cyclohexylmethyl]-4-methyl-4,5-dihydro-1H-imidazol-4-carbonsäuremethylester | 481,37 |
| 111. | 5-{4-[Dimethylamino-(3-fluor-phenyl)-methyl]-cyclohexylmethyl}-1,4-dipropyl-4,5-dihydro-1H-imidazol-4-carbonsäuremethylester | 459,33 |
| 112. | 5-{4-[Dimethylamino-(3-fluor-phenyl)-methyl]-cyclohexylmethyl}-4-(2-methylsulfanyl-ethyl)-1-propyl-4,5-dihydro-1H-imidazol-4-carbonsäuremethylester | 491,30 |
| 113. | 1-Benzyl-4-(4-chlor-benzyl)-5-{4-[(4-chlor-phenyl)-dimethylamino-methyl]-cyclohexylmethyl}-4,5-dihydro-1H-imidazol-4-carbonsäuremethylester | 605,26 |
| 114. | 1-Benzyl-4-butyl-5-{4-[(4-chlor-phenyl)-dimethylamino-methyl]-cyclohexylmethyl}-4,5-dihydro-1H-imidazol-4-carbonsäuremethylester | 537,31 |
| 115. | 1-Cyclohexyl-5-{4-[dimethylamino-(3-fluor-phenyl)-methyl]-cyclohexyl}-4-(2- methoxycarbonyl-ethyl)-4,5-dihydro-1H-imidazol-4-carbonsäuremethylester | 529,33 |
| 116. | 4-Benzylsulfanylmethyl-1-cycloheoyl-5-{4-[dimethylamino-(3-fluor-phenyl)-methyl]-cyclohexyl}-4,5-dihydro-1H-imidazol-4-carbonsäuremethylester | 579,33 |
| 117. | 5-{4-[Dimethylamino-(3-fluor-phenyl)-methyl]-cyclohexylmethyl}-4-methyl-1-propyl-4,5-dihydro-1H-imidazol-4-carbonsäuremethylester | 431,29 |
| 118. | 5-{4-[Dimethylamino-(4-fluor-phenyl)-methyl]-cyclohexylmethyl}-1,4-dipropyl-4,5-dihydro-1H-imidazol-4-carbonsäuremethylester | 459,33 |
| 119. | 5-{4-[Dimethylamino-(4-fluor-phenyl)-methyl]-cyclohexylmethyl}-4-(2-methylsulfanyl-ethyl)-1-propyl-4,5-dihydro-1H-imidazol-4-carbonsäuremethylester | 491,30 |
| 120. | 5-[4-(1-Dimethylamino-3-phenyl-propyl)-cyclohexylmethyl]-1,4-dipropyl-4,5-dihydro-1H-imidazole-imidazol-4-carbonsäuremethylester | 469,37 |
| 121. | 5-[4-(1-Dimethylamino-3-phenyl-propyl)-cyclohexylmethyl]-4-(2-methylsulfanyl-ethyl)-1-propyl-4,5-dihydro-1H-imidazol-4-carbonsäuremethylester | 501,34 |
| 122. | 1-Butyl-5-{4-[dimethylamino-(3-fluor-phenyl)-methyl]-cyclohexylmethyl}-4-propyl-4,5-dihydro-1H-imidazol-4-carbonsäuremethylester | 473,34 |
| 123. | 1-Butyl-5-{4-[dimethylamino-(3-fluor-phenyl)-methyl]-cyclohexylmethyl}4-(2-methylsulfanyl-ethyl)-4,5-dihydro-1H-imidazol-4-carbonsäuremethylester | 505,31 |
| 124. | 1-Butyl-5-{4-[dimethylamino-(3-fluor-phenyl)-methyl]-cycloheemethyl}-4-methyl-4,5-dihydro-1H-imidazol-4-carbonsäuremethylester | 445,31 |
| 125. | 1-Butyl-5-{4-[dimethylamino-(4-fluor-phenyl)-methyl]-cyclohexylmethyl}-4-propyl-4,5-dihydro-1H-imidazol-4-carbonsäuremethylester | 473,34 |
| 126. | 1-Butyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester | 505,31 |
| 127. | 1-Butyl-5-[4-(1-dimethylamino-3-phenyl-propyl)-cyclohexylmethyl]-4-propyl-4,5-dihydro-1H-imidazol-4-carbonsäuremethylester | 483,38 |
| 128. | 1-Butyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester | 515,35 |
| 129. | 5-{4-[Dimethylamino-(3-fluor-phenyl)-methyl]-cyclohexylmethyl}-1-phenethyl-4-propyl-4,5-dihydro-1H-imidazol-4-carbonsäuremethylester | 521,34 |
| 130. | 5-{4-[Dimethylamino-(3-fluor-phenyl)-methyl]-cyclohexylmethyl}-4-(2-methylsulfanyl-ethyl)-1-phenethyl-4,5-dihydro-1H-imidazol-4-carbonsäuremethylester | 553,31 |
| 131. | 5-{4-[Dimethylamino-(3-fluor-phenyl)-methyl]-cyclohexylmethyl}-4-methyl-1-phenethyl-4,5-dihydro-1H-imidazol-4-carbonsäuremethylester | 493,31 |
| 132. | 5-{4-[Dimethylamino-(4-fluor-phenyl)-methyl]-cyclohexylmethyl}-1-phenethyl-4-propyl-4,5-dihydro-1H-imidazol-4-carbonsäuremethylester | 521,34 |
| 133. | 5-{4-[Dimethylamino-(4-fluor-phenyl)-methyl]-cyclohexylmethyl}-4-(2-methylsulfanyl-ethyl)-1-phenethyl-4,5-dihydro-1H-imidazol-4-carbonsäu-remethylester | 553,31 |
| 134. | 5-{4-[Dimethylamino-(4-fluor-phenyl)-methyl]-cyclohecylmethyl}-4-methyl-1-phenethyl-4,5-dihydro-1H-imidazol-4-carbonsäuremethylester | 493,31 |
| 135. | 5-[4-(1-Dimethylamino-3-phenyl-propyl)-cyclohexylmethyl]-1-phenethyl-4-propyl-4,5-dihydro-1H-imidazol-4-carbonsäuremethylester | 531,38 |
| 136. | 5-[4-(1-Dimethylamino-3-phenyl-propyl)-cyclohexylmethyl]-4-(2-methylsulfanyl-ethyl)-1-phenethyl-4,5-dihydro-1H-imidazol-4-carbonsäuremethylester | 563,35 |
| 137. | 5-[4-(1-Dimethylamino-3-phenyl-propyl)-cyclohexylmethyl]-4-methyl-1-phenethyl-4,5-dihydro-1H-imidazol-4-carbonsäuremethylester | 503,35 |
| 138. | 1-Benzyl-5-{4-[dimethylamino-(3-fluor-phenyl)-methyl]-cyclohexylmethyl}-4-propyl-4,5-dihydro-1H-imidazol-4-carbonsäuremethylester | 507.33 |
| 139. | 1-Benzyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester | 539,30 |
| 140. | 1-Benzyl-5-{4-[dimethylamino-(3-fluor-phenyl)-methyl]-cyclohexylmethyl}-4-methyl-4,5-dihydro-1H-imidazol-4-carbonsäuremethylester | 479,29 |
| 141. | 1-Benzyl-5-{4-[dimethylamino-(4-fluor-phenyl)-methyl]-cyclohexylmethyl}-4-propyl-4,5-dihydro-1H-imidazol-4-carbonsäuremethylester | 507,33 . |
| 142. | 1-Benzyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester | 539,30 |
| 143. | 1-Benzyl-5-{4-[dimethylamino-(4-fluor-phenyl)-methyl]-cyclohexylmethyl}-4-methyl-4,5-dihydro-1H-imidazol-4-carbonsäuremethylester | 479,29 |
| 144. | 1-Benzyl-5-[4-(1-dimethylamino-3-phenyl-propyl)-cyclohexylmethyl]-4-propyl-4,5-dihydro-1H-imidazol-4-carbonsäuremethylester | 517,37 |
| 145. | 1-Benzyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester | 549,34 |
| 146. | 1-Benzyl-5-[4-(1-dimethylamino-3-phenyl-propyl)-cyclohexylmethyl]-4-methyl-4,5-dihydro-1H-imidazol-4-carbonsäuremethylester | 489,34 |
| 147. | 1-Cyclohexyl-5-{4-[dimethylamino-(3-fluor-phenyl)-methyl]-cyclohexylmethyl}-4-(2-methoxycarbonyl-ethyl)-4,5-dihydro-1H-imidazole4-carbonsäuremethylester | 543,35 |
| 148. | 4-sec-Butyl-1-cyclohexyl-5-{4-[dimethylamino-(3-fluor-phenyl)-methyl]-cyclohexylmethyl}-4,5-dihydro-1H-imidazol-4-carbonsäuremethylester | 513,37 |
| 149. | 1-Cyclohexyl-5-{4-[dimethylamino-(3-fluor-phenyl)-methyl]-cyclohexylmethyl}-4-methylsulfanylmethyl-4,5-dihydro-1H-imidazol-4-carbonsäuremethylester | 517,31 |
| 150. | 4-Benzylsulfanylmethyl-1-cyclohexyl-5-{4-[dimethylamino-(3-fluor-phenyl)-methyl]-cyclohexylmethyl}-4,5-dihydro-1H-imidazol-4-carbonsäuremethylester | 593,35 |
| 151. | 1-Cyclohexyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester | 543,35 |
| 152. | 4-sec-Butyl-1-cyclohexyl-5-{4-[dimethylamino-(4-fluor-phenyl)-methyl]-cyclohexylmethyl}-4,5-dihydro-1H-imidazol-4-carbonsäuremethylester | 513,37 |
| 153. | 1-Cyclohexyl-5-{4-[dimethylamino-(4-fluor-phenyl)-methyl]-cyclohexylmethyl}-4-methylsulfanylmethyl-4,5-dihydro-1H-imidazol-4-carbonsäuremethylester | 517,31 |
| 154. | 1-Cyclohexyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester | 553,39 |
| 155. | 1-Cyclohexyl-5-[4-(1-dimethylamino-3-phenyl-propyl)-cyclohexylmethyl]-4-methylsulfanylmethyl-4,5-dihydro-1H-imidazol-4-carbonsäuremethylester | 527,35 |
| 156. | 4-Benzylsulfanylmethyl-1-cyclohexyl-5-[4-(1-dimethylamino-3-phenyl-propy)-cyclohexylmethyl]-4,5-dihydro-1H-imidazol-4-carbonsäuremethylester | 603,39 |
| 157. | 5-{4-[Dimethylamino-(3-fluor-phenyl)-methyl]-cyclohexylmethyl}-4-(2-methoxycarbonyl-ethyl)-1-propyl-4,5-dihydro-1H-imidazol-4-carbonsäure-methylester | 503,32 |
| 158. | 5-{4-[Dimethylamino-(3-fluor-phenyl)-methyl]-cyclohexylmethyl}-4-methylsulfanylmethyl-1-propyl-4,5-dihydro-1H-imidazol-4-carbonsäuremethylester | 477,28 |
| 159. | 5-{4-[Dimethylamino-(4-fluor-phenyl)-methyl]-cyclohexylmethyl}-4-(2-methoxycarbonyl-ethyl)-1-propyl-4,5-dihydro-1Himidazol-4-carbonsäure-methylester | 503,32 |
| 160. | 5-{4-[Dimethylamino-(4-fluor-phenyl)-methyl]-cyclohexylmethyl}-4-methylsulfanylmethyl-1-propyl-4,5-dihydro-1H-imidazol-4-carbonsäuremethylester | 477,28 |
| 161. | 4-Benzylsulfanylmethyl-5-{4-[dimethylamino-(4-fluor-phenyl)-methyl]-cyclohexylmethyl}-1-propyl-4,5-dihydro-1H-imidazol-4-carbonsäuremethylester | 553,31 |

### Untersuchungen zur Wirksamkeit der erfindungsgemäßen Verbindungen

### Methode zur Bestimmung der Affinität zum humanen µ-Opiatrezeptor

Die Rezeptoraffinität zum humanen µ-Opiatrezeptor wird in einem homogenen Ansatz in Mikrotiterplatten bestimmt. Hierzu werden Verdünnungsreihen der zu prüfenden Substanzen mit einer Rezeptormembranpräparation (15-40 µg Protein / 250 µl Inkubationsansatz) von CHO-K1-Zellen, welche den humanen µ-Opiatrezeptor exprimieren (RB-HOM-Rezeptormembran-Präparation von Fa PerkinElmer Life Sciences, Zaventem, Belgien) in Gegenwart von 1 nmol/l des radioaktiven Liganden [³H]-Naloxon (NET719, Fa. PerkinElmer Life Sciences, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,06 % bovinem Serumalbumin verwendet. Zur Bestimmung der unspezifischen Bindung wird zusätzlich 100 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der Prüfsubstanzen von 1 µmol/l bestimmt und als Prozent Hemmung der spezifischen Bindung angegeben.

**Tabelle 1: µ-Affinitäten ausgewählter Aldehyde**

| Nr. | µ-Opioid-Rezeptor, %Hemmung [1 µM] |
|---|---|
| 9c | 45 |
| 10c | 50 |
| 10b | 67 |
| 9d | 69 |
| 10d | 79 |

**Tabelle 2: µ-Affinitäten der Beispiele 11-161:**

| 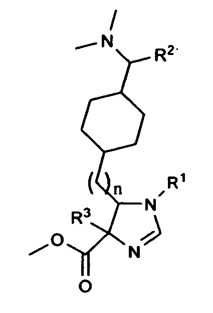 | | | | | |
|---|---|---|---|---|---|
| **Nr.** | **n** | **R¹** | **R²** | **R³** | **µ-Opioid-Rezeptor, %Hemmung [1 µM]** |
| 11 | 0 | 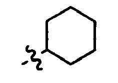 | 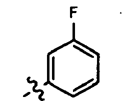 | 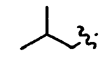 | 84 |
| 12 | 0 | 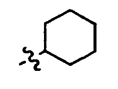 | 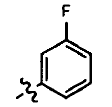 | 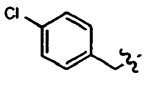 | 73 |
| 13 | 0 | 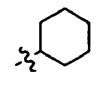 | 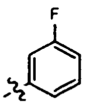 | 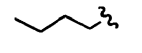 | 94 |
| 14 | 0 | 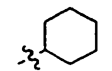 | 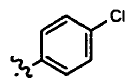 | 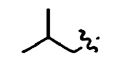 | 92 |
| 15 | 0 | 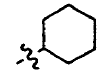 | 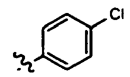 | 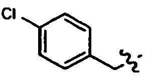 | 76 |
| 16 | 0 | 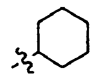 | 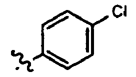 | 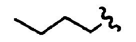 | 92 |
| 17 | 1 | 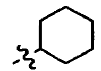 | 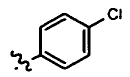 | 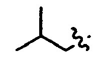 | 96 |
| 18 | 1 | 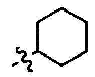 | 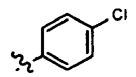 | 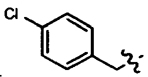 | 90 |
| 19 | 1 | 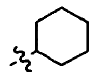 | 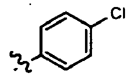 | 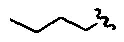 | 96 |
| 20 | 1 | 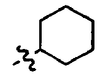 | 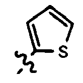 | 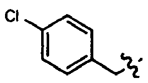 | 95 |
| 21 | 0 | 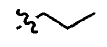 | 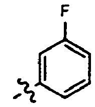 | 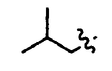 | 83 |
| 22 | 0 | 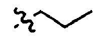 | 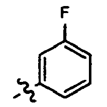 | 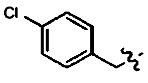 | 88 |
| 23 | 0 | 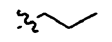 | 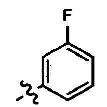 | 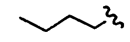 | 76 |
| 24 | 0 | 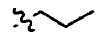 | 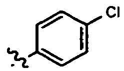 | 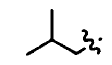 | 89 |
| 25 | 0 | 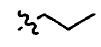 | 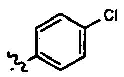 | 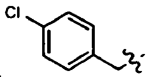 | 83 |
| 26 | 1 | 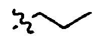 | 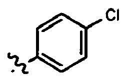 | 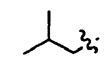 | 96 |
| 27 | 1 | 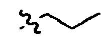 | 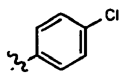 | 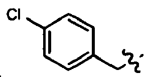 | 96 |
| 28 | 1 | 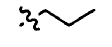 | 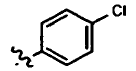 | 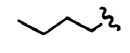 | 97 |
| 29 | 0 | 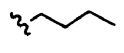 | 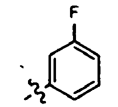 | 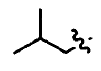 | 89 |
| 30 | 0 | 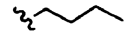 | 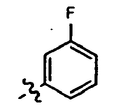 | 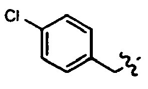 | 89 |
| 31 | 0 | 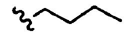 | 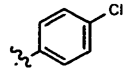 | 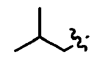 | 91 |
| 32 | 0 | 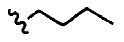 | 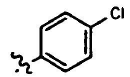 | 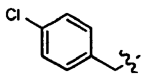 | 74 |
| 33 | 0 | 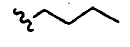 | 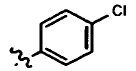 | 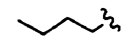 | 85 |
| 34 | 1 | 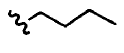 | 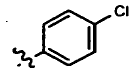 | 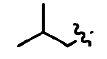 | 98 |
| 35 | 1 | 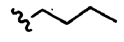 | 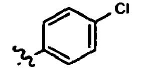 | 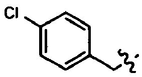 | 92 |
| 36 | 1 | 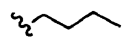 | 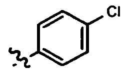 | 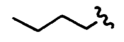 | 97 |
| 37 | 0 | 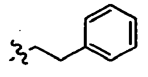 | 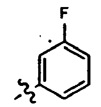 | 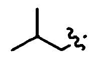 | 93 |
| 38 | 0 | 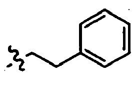 | 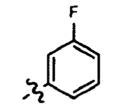 | 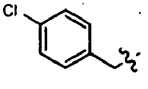 | 84 |
| 39 | 0 | 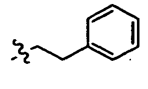 | 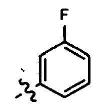 | 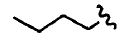 | 88 |
| 40 | 0 | 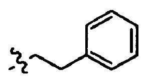 | 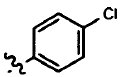 | 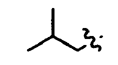 | 95 |
| 41 | 0 | 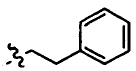 | 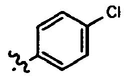 | 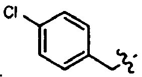 | 79 |
| 42 | 0 | 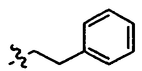 | 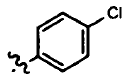 | 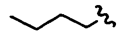 | 94 |
| 43 | 1 | 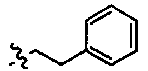 | 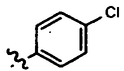 | 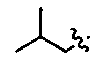 | 97 |
| 44 | 1 | 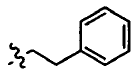 | 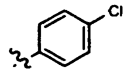 | 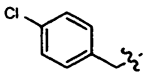 | 91 |
| 45 | 1 | 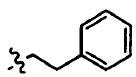 | 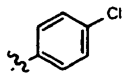 | 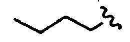 | 97 |
| 46 | 0 | 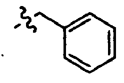 | 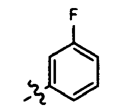 | 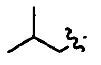 | 92 |
| 47 | 0 | 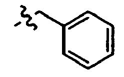 | 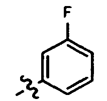 | 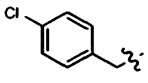 | 74 |
| 48 | 0 | 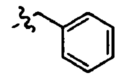 | 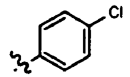 | 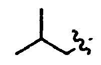 | 93 |
| 49 | 0 | 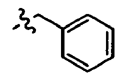 | 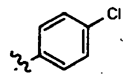 | 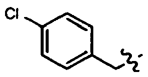 | 84 |
| 50 | 1 | 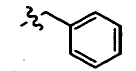 | 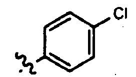 | 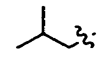 | 94 |
| 51 | 0 | 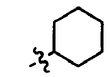 | 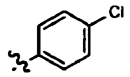 | 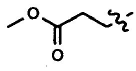 | 96 |
| 52 | 0 | 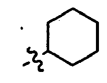 | 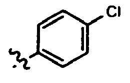 | 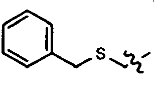 | 99 |
| 53 | 1 | 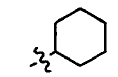 | 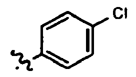 | 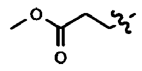 | 96 |
| 54 | 1 | 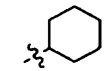 | 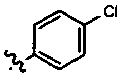 | 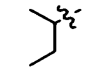 | 100 |
| 55 | 1 | 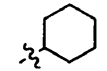 | 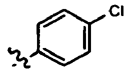 | 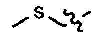 | 91 |
| 56 | 1 | 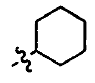 | 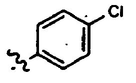 | 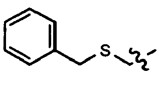 | 97 |
| 57 | 0 | 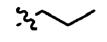 | 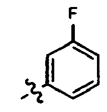 | 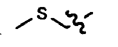 | 86 |
| 58 | 0 | 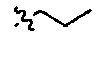 | 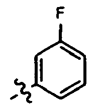 | 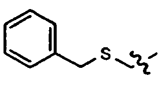 | 81 |
| 59 | 0 | 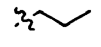 | 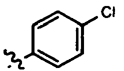 | 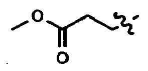 | 84 |
| 60 | 0 | 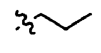 | 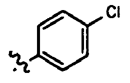 | 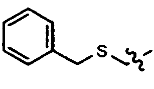 | 95 |
| 61 | 1 | 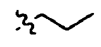 | 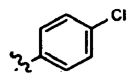 | 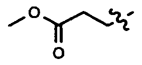 | 93 |
| 62 | 1 | 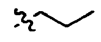 | 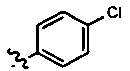 | 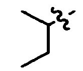 | 98 |
| 63 | 1 | 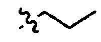 | 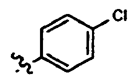 | 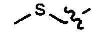 | 97 |
| 64 | 1 | 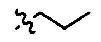 | 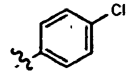 | 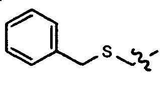 | 98 |
| 65 | 0 | 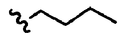 | 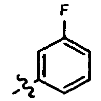 | 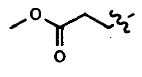 | 78 |
| 66 | 0 | 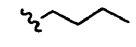 | 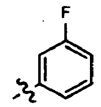 | 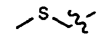 | 89 |
| 67 | 0 | 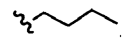 | 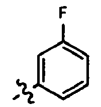 | 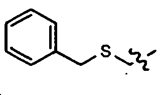 | 88 |
| 68 | 0 | 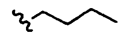 | 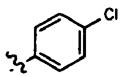 | 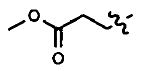 | 90 |
| 69 | 0 | 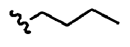 | 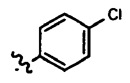 | 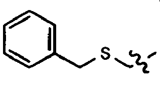 | 95 |
| 70 | 1 | 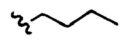 | 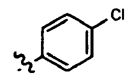 | 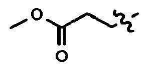 | 96 |
| 71 | 1 | 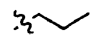 | 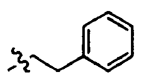 | 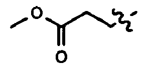 | 78 |
| 72 | 1 | 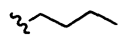 | 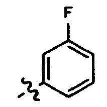 | 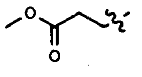 | 95 |
| 73 | 1 | 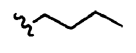 | 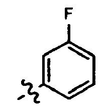 | 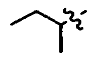 | 92 |
| 74 | 1 | 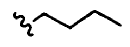 | 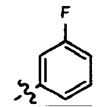 | 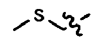 | 95 |
| 75 | 1 | 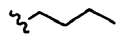 | 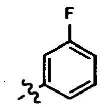 | 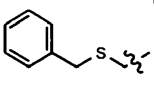 | 98 |
| 76 | 1 | 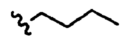 | 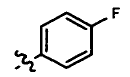 | 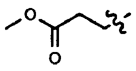 | 93 |
| 77 | 1 | 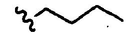 | 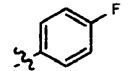 | 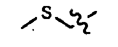 | 88 |
| 78 | 1 | 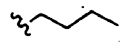 | 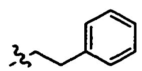 | 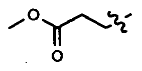 | 67 |
| 79 | 1 | 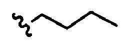 | 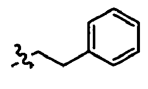 | 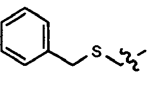 | 79 |
| 80 | 1 | 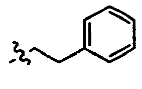 | 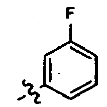 | 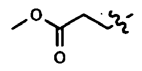 | 90 |
| 81 | 1 | 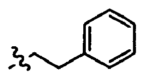 | 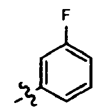 | 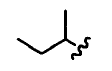 | 76 |
| 82 | 1 | 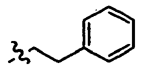 | 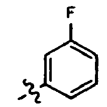 | 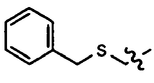 | 95 |
| 83 | 1 | 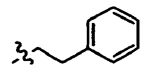 | 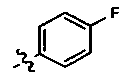 | 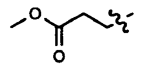 | 77 |
| 84 | 1 | 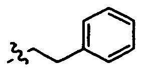 | 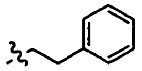 | 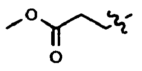 | 75 |
| 85 | 1 | 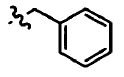 | 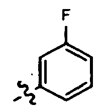 | 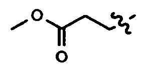 | 95 |
| 86 | 1 | 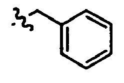 | 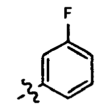 | 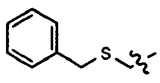 | 71 |
| 87 | 1 | 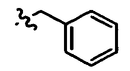 | 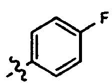 | 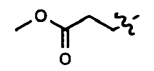 | 95 |
| 88 | 1 | 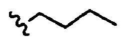 | 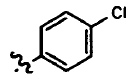 | 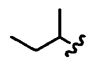 | 98 |
| 89 | 1 | 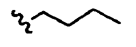 | 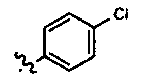 | 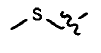 | 96 |
| 90 | 1 | 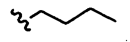 | 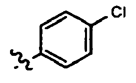 | 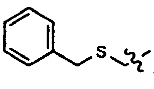 | 99 |
| 91 | 0 | 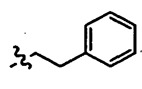 | 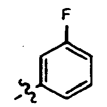 | 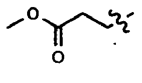 | 85 |
| 92 | 0 | 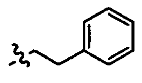 | 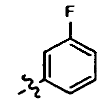 | 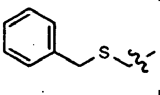 | 95 |
| 93 | 0 | 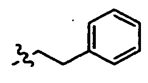 | 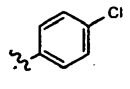 | 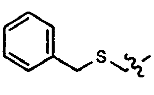 | 92 |
| 94 | 1 | 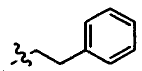 | 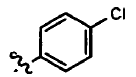 | 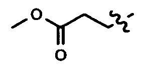 | 97 |
| 95 | 0 | 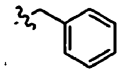 | 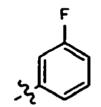 | 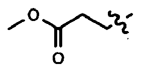 | 94 |
| 96 | 0 | 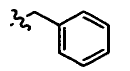 | 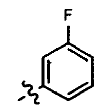 | 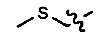 | 97 |
| 97 | 0 | 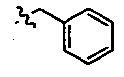 | 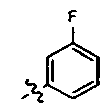 | 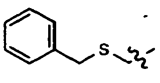 | 97 |
| 98 | 0 | 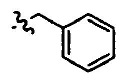 | 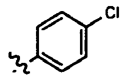 | 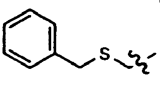 | 95 |
| 99 | 1 | 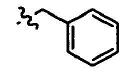 | 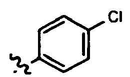 | 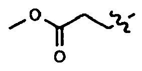 | 100 |
| 100 | 1 | 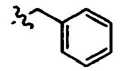 | 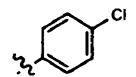 | 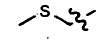 | 97 |
| 101 | 1 | 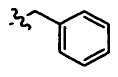 | 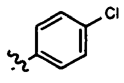 | 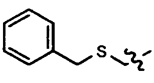 | 96 |
| 102 | 1 | 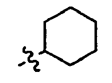 | 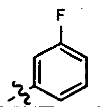 | 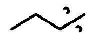 | 91 |
| 103 | 1 | 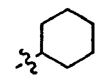 | 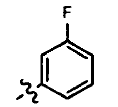 | 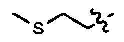 | 99 |
| 104 | 1 | 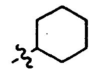 | 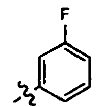 | Me | 93 |
| 105 | 1 | 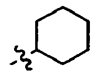 | 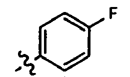 | 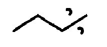 | 95 |
| 106 | 1 | 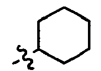 | 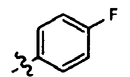 | 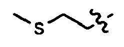 | 92 |
| 107 | 1 | 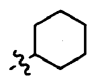 | 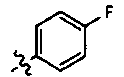 | Me | 87 |
| 108 | 1 | 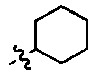 | 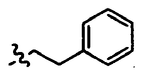 | 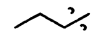 | 86 |
| 109 | 1 | 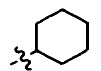 | 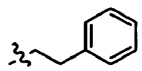 | 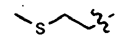 | 87 |
| 110 | 1 | 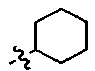 | 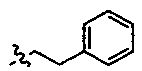 | Me | 67 |
| 111 | 1 | 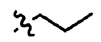 | 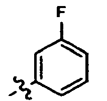 | 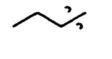 | 90 |
| 112 | 1 | 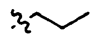 | 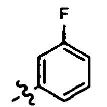 | 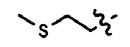 | 95 |
| 113 | 1 | 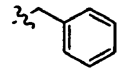 | 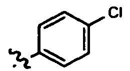 | 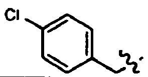 | 92 |
| 114 | 1 | 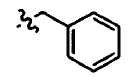 | 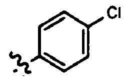 | 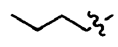 | 86 |
| 115 | 0 | 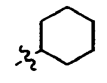 | 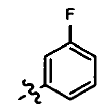 | 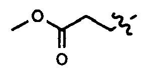 | 98 |
| 116 | 0 | 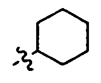 | 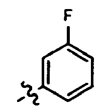 | 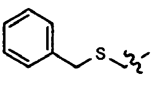 | 101 |
| 117 | 1 | 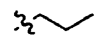 | 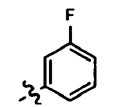 | Me | 88 |
| 118 | 1 | 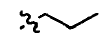 | 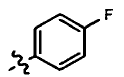 | 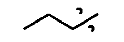 | 87 |
| 119 | 1 | 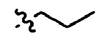 | 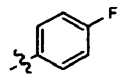 | 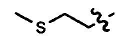 | 90 |
| 120 | 1 | 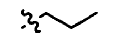 | 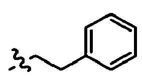 | 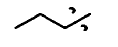 | 62 |
| 121 | 1 | 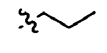 | 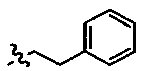 | 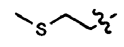 | 77 |
| 122 | 1 | 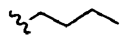 | 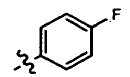 | 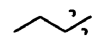 | 95 |
| 123 | 1 | 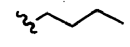 | 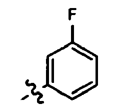 | 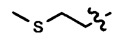 | 98 |
| 124 | 1 | 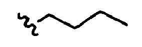 | 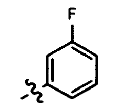 | Me | 95 |
| 125 | 1 | 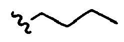 | 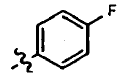 | 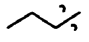 | 91 |
| 126 | 1 | 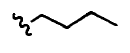 | 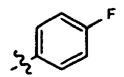 | 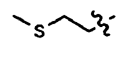 | 98 |
| 127 | 1 | 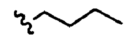 | 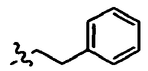 | 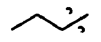 | 74 |
| 128 | 1 | 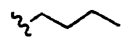 | 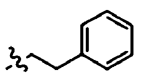 | 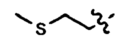 | 83 |
| 129 | 1 | 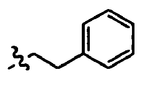 | 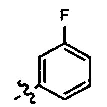 | 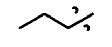 | 93 |
| 130 | 1 | 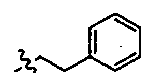 | 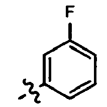 | 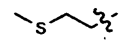 | 97 |
| 131 | 1 | 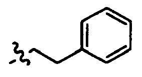 | 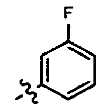 | Me | 100 |
| 132 | 1 | 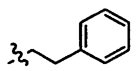 | 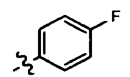 | 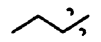 | 98 |
| 133 | 1 | 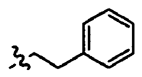 | 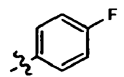 | 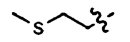 | 84 |
| 134 | 1 | 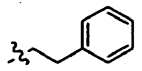 | 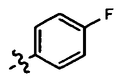 | Me | 92 |
| 135 | 1 | 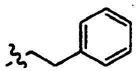 | 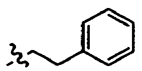 | 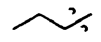 | 85 |
| 136 | 1 | 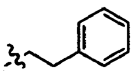 | 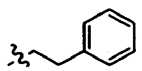 | 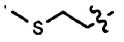 | 76 |
| 137 | 1 | 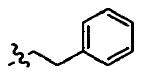 | 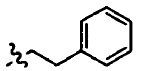 | Me | 61 |
| 138 | 1 | 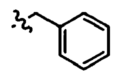 | 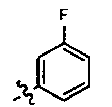 | 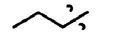 | 101 |
| 139 | 1 | 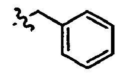 | 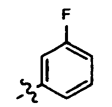 | 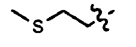 | 100 |
| 140 | 1 | 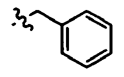 | 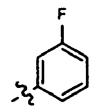 | Me | 98 |
| 141 | 1 | 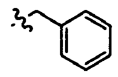 | 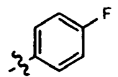 | 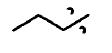 | 98 |
| 142 | 1 | 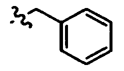 | 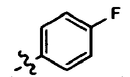 | 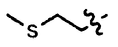 | 97 |
| 143 | 1 | 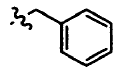 | 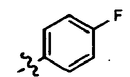 | Me | 100 |
| 144 | 1 | 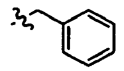 | 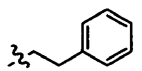 | 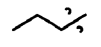 | 90 |
| 145 | 1 | 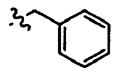 | 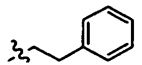 | 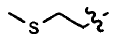 | 79 |
| 146 | 1 | 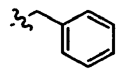 | 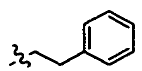 | Me | 79 |
| 147 | 1 | 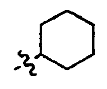 | 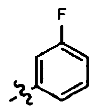 | 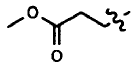 | 98 |
| 148 | 1 | 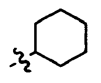 | 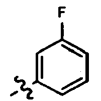 | 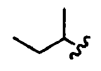 | 95 |
| 149 | 1 | 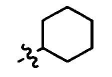 | 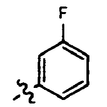 | 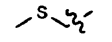 | 99 |
| 150 | 1 | 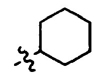 | 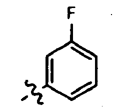 | 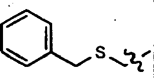 | 98 |
| 151 | 1 | 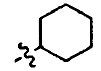 | 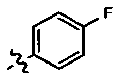 | 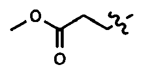 | 95 |
| 152 | 1 | 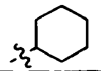 | 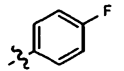 | 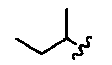 | 93 |
| 153 | 1 | 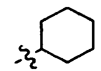 | 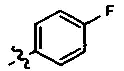 | 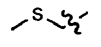 | 93 |
| 154 | 1 | 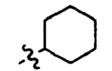 | 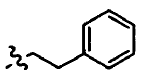 | 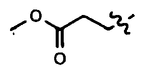 | 82 |
| 155 | 1 | 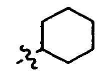 | 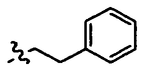 | 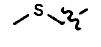 | 86 |
| 156 | 1 | 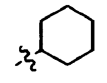 | 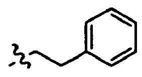 | 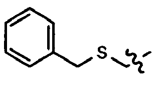 | 92 |
| 157 | 1 | 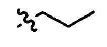 | 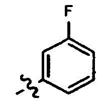 | 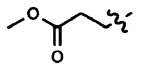 | 91 |
| 158 | 1 | 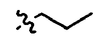 | 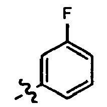 | 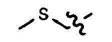 | 95 |
| 159 | 1 | 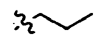 | 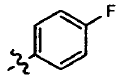 | 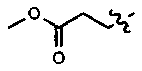 | 79 |
| 160 | 1 | 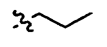 | 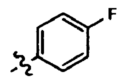 | 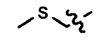 | 74 |
| 161 | 1 | 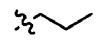 | 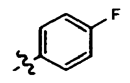 | 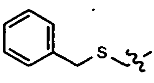 | 91 |

## Patentansprüche

1. Substituierte Imidazolin-Derivate der allgemeinen Formel I, 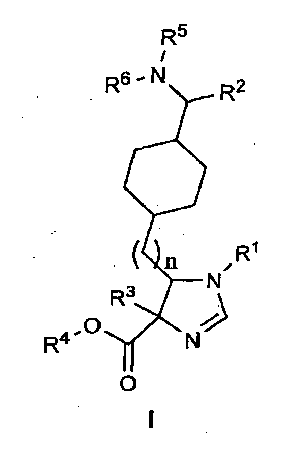 worin
n 0, 1 oder 2 bedeutet
R¹ C₁₋₈-Alkyl, gesättigt oder ungesättigt, verzweigt oder unverzweigt, unsubstituiert oder einfach oder mehrfach substituiert; C₃₋₈-Cycloalkyl oder ein entsprechendes Cycloalkyl, in dem ein oder zwei Kohlenstoffatome durch ein Heteroatom S, N oder O ersetzt sind, gesättigt oder ungesättigt, unsubstituiert oder einfach oder mehrfach substituiert;
wobei einfach oder mehrfach substituiert jeweils die Substitution eines oder mehrerer Wasserstoffreste durch F, Cl, Br, I, -CN, NH₂, NH-C₁₋₆Alkyl, NH-C₁-₆-Alkyl-OH, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, C₁₋₆-Alkyl, Benzyl, O-Benzyl, C(=O)C₁₋₆-Alkyl, CO₂H, CO₂-C₁₋₆-Alkyl bedeutet und die Mehrfachsubstitution entweder an verschiedenen oder gleichen Atomen mit gleichen oder verschiedenen Substituenten vorliegt;
einen über eine C₁₋₃-Alkylkette gebundenen Aryl- oder Heteroarylrest, jeweils unsubstituiert oder einfach oder mehrfach substituiert, bedeutet,
wobei einfach oder mehrfach substituiert jeweils die Substitution eines oder mehrerer Wasserstoffatome des Ringsystems durch F, Cl, Br, I, CN, NH₂, NH-C₁-₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkl-OH, C(=O)C₁₋₆-Alkyl, CO₂H, CO₂-C₁₋₆-Alkyl, CF₃, C₁₋₆-Alkyl bedeutet und die Mehrfachsubstitution an einem oder verschiedenen Atomen mit dem gleichen oder mit unterschiedlichen Substituenten vorliegt,
R² Aryl oder Heteroaryl, jeweils unsubstituiert oder einfach oder mehrfach substituiert wie oben für den Aryl- oder Heteroarylrest definiert; einen über eine C₁₋₃-Alkylkette gebundenen Arylrest, jeweils unsubstituiert oder einfach oder mehrfach substituiert wie oben für den Arylrest definiert, bedeutet,
R³ C₁₋₈-Alkyl, gesättigt oder ungesättigt, verzweigt oder unverzweigt, unsubstituiert oder einfach oder mehrfach substituiert wie oben für C₁₋₈-Alkyl definiert; einen über eine C₁₋₃-Alkylkette gebundenen Arylrest, jeweils unsubstituiert oder einfach oder mehrfach substituiert wie oben für den Arylrest definiert, bedeutet,
R⁴ H, C₁₋₄-Alkyl oder einen über eine C₁₋₃-Alkylkette gebundenen Arylrest, bedeutet,
R⁵ und R⁶ unabhängig voneinander H; C₁₋₆-Alkyl, jeweils gesättigt oder ungesättigt, verzweigt oder unverzweigt, bedeutet, wobei R⁵ und R⁶ nicht gleichzeitig H bedeuten,
oder die Reste R⁵ und R⁶ zusammen CH₂CH₂OCH₂CH₂, oder (CH₂)₃₋₆ bedeuten,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

2. Substituierte Imidazolin-Derivate gemäß Anspruch 1, worin R¹ C₁₋₈-Alkyl, verzweigt oder unverzweigt, unsubstituiert oder einfach oder mehrfach substituiert mit F, Cl,-CN, SH, S-C₁₋₆-Alkyl, S-Benzyl, OH, O-Benzyl, O-C₁₋₆-Alkyl, CO₂H, CO₂-C₁₋₆-Alkyl; C₃₋₈-Cycloalkyl, jeweils unsubstituiert oder einfach oder mehrfach substituiert mit F, Cl, -CN, SH, S-C₁₋₆-Alkyl, S-Benzyl, OH, O-Benzyl, O-C₁₋₆-Alkyl, CO₂H, CO₂-C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; bedeutet.

3. Substituierte Imidazolin-Derivate gemäß Anspruch 1, worin R¹ Cyclohexyl, n-Propyl, n-Butyl, Phenethyl oder Benzyl bedeutet.

4. Substituierte Imidazolin-Derivate gemäß einem der Ansprüche 1-3, worin R² Phenyl oder Thienyl, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl, bedeutet;

5. Substituierte Imidazofin-Derivate gemäß einem der Ansprüche 1-3, worin R² Phenyl, unsubstituiert oder einfach substituiert mit Cl oder F, Thienyl oder Phenethyl bedeutet.

6. Substituierte Imidazolin-Derivate gemäß einem der Ansprüche 1-5, worin R³ C₁₋₈-Alkyl, verzweigt oder unverzweigt, unsubstituiert oder einfach oder mehrfach substituiert mit F, Cl, -CN, SH, S-C₁₋₆-Alkyl, S-Benzyl, OH, O-Benzyl, O-C₁₋₆-Alkyl, CO₂H, CO₂-C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl CO₂H, CO₂-C₁₋₆-Alkyl, CF₃, C₁₋₆-Alkyl; bedeutet,

7. Substituierte Imidazolin-Derivate gemäß einem der Ansprüche 1-5, worin R³ sec-Butyl, iso-Butyl, n-Butyl, n-Propyl, CH₃, CH₂CH₂COOCH₃, CH₂-S-Benzyl, CH₂CH₂-S-CH₃, CH₂-S-CH₃ oder 4-Cl-Benzyl bedeutet.

8. Substituierte Imidazolin-Derivate gemäß einem der Ansprüche 1-7, worin R⁴ CH₃ bedeutet.

9. Substituierte Imidazolin-Derivate gemäß einem der Ansprüche 1-8, worin R⁵ und R⁶ CH₃ bedeuten.

10. Substituierte Imidazolin-Derivate gemäß einem der Ansprüche 1-9 aus der Gruppe
1-Cyclohexyl-5-{4-[dimethylamino-(3-fluor-phenyl)-methyl]-cyclohexyl}-4-isobutyl-4,5-dihydro-1H-imidazol-4-carbonsäuremethylester
4-(4-Chlor-benzyl)-1-cyclohexyl-5-{4-[dimethylamino-(3-fluor-phenyl)-methyl]-cyclohexyl}-4,5-dihydro-1H-imidazol-4-carbonsäuremethylester
4-Butyl-1-cyclohexyl-5-{4-[dimethylamino-(3-fluor-phenyl)-methyl]-cyclohexyl}-4,5-dihydro-1H-imidazol-4-carbonsäuremethylester
5-{4-[(4-Chlor-phenyl)-dimethylamino-methyl]-cyclohexyl}-cyclohexyl-4-isobutyl-4,5-dihydro-1H-imidazol-4- carbonsäuremethylester
4-(4-Chlor-benzyl)-5-{4-[(4-chlor-phenyl)-dimethylamino-methyl]-cyclohexyl}-1-cyclohexyl-4,5-dihydro-1H-imidazol-4-carbonsäuremethylester
4-Butyl-5-{4-[(4-chlor-phenyl)-dimethylamlno-methyl]-cyclohexyl}-1-cyclohexyl-4,5-dihydro-1H-imidazol-4-carboxylic carbonsäuremethylester
5-{4-[(4-Chlor-phenyl)-dimethylamino-methyl]-cyclohexylmethyl}-1-cyclohexyl-4-isobutyl-4,5-dihydro-1H-imidazol-4-carbonsäuremethylester
4-(4-Chlor-benzyl)-5-{4-[(4-chlor-phenyl)-dimethylamino-methyl]-cyclohexylmethyl}-1-cyclohexyl-4,5-dihydro-1H-imidazol-4-carbonsäuremethylester
4-Butyl-5-{4-[(4-chlor-phenyl)-dimethylamino-methyl]-cyclohexylmethyl}-1-cyclohexyl-4,5-dihydro-1H-imidazol-4-carbonsäuremethylester
4-(4-Chlor-benzyl)-1-cyclohexyl-5-[4-(dimethylamino-thiophen-2-yl-methyl)-cyclohexylmethyl]-4,5-dihydro-1H-imidazol-4-carbonsäuremethylester
5-{4-[Dimethylamino-(3-fluor-phenyl)-methyl]-cyclohexyl}-4-isobutyl-1-propyl-4,5-dihydro-1H-imidazol-4-carbonsäuremethylester
4-(4-Chlor-benzyl)-5-{4-[dimethylamino-(3-fluor-phenyl)-methyl]-cyclohexyl}-1-propyl-4,5-dihydro-1H-imidazol-4carbonsäuremethylester
4-Butyl-5-{4-[dimethylamino-(3-fluor-phenyl)-methyl]-cyclohexyl}-1-propyl-4,5-dihydro-1H-imidazol-4-carbonsäuremethylester
5-{4-[(4-Chlor-phenyl)-dimethylamino-methyl]-cyclohexyl}-4-isobutyl-1-propyl-4,5-dihydro-1H-imidazol-4-carbonsäuremethylester
4-(4-Chlor-benzyl)-5-{4-[(4-chlor-phenyl)-dimethylamino-methyl]-cyclohexyl}-1-propyl-4,5-dihydro-1H-imidazol-4-carbonsäuremethylester
5-{4-[(4-Chlor-phenyl)-dimethylamino-methyl]-cyclohexylmethyl}-4-isobutyl-1-propyl-4,5-dihydro-1H-imidazol-4-carbonsäuremethylester
4-(4-Chlor-benzyl)-5-{4-[(4-chlor-phenyl)-dimethylamino-methyl]-cyclohexylmethyl}-1-propyl-4,5-dihydro-1H-imidazol-4-carbonsäuremethylester
4-Butyl-5-{4-[(4-chlor-phenyl)-dimethylamino-methyl]-cyclohexylmethyl}-1-propyl-4,5-dihydro-1H-imidazol-4-carbonsäuremethylester
1-Butyl-5-{4-[dimethylamino-(3-fluor-phenyl)-methyl]-cyclohexyl}-4-isobutyl-4,5-dihydro-1H-imidazol-4-carbonsäuremethylester
1-Butyl-4-(4-chlor-benzyl)-5-{4-[dimethylamino-(3-fluor-phenyl)-methyl]-cyclohexyl}-4,5-dihydro-1H-imidazol-4-carbonsäuremethylester
1-Butyl-5-{4-[(4-chlor-phenyl)-dimethylamino-methyl]-cyclohexyl}-4-isobutyl-4,5-dihydro-1H-imidazol-4-carbonsäuremethylester
1-Butyl-4-(4-chlor-benzyl)-5-{4-[(4-chlor-phenyl)-dimethylamino-methyl]-cyclohexyl}-4,5-dihydro-1H-imidazol-4-carbonsäuremethylester
1,4-Dibutyl-5-{4-[(4-chlor-phenyl)-dimethylamino-methyl]-cyclohexyl}-4,5-dihydro-1H-imidazol-4-carbonsäuremethylester
1-Butyl-5-{4-[(4-chlor-phenyl)-dimethylamino-methyl]-cyclohexylmethyl}-4-isobutyl-4,5-dihydro-1H-imidazol-4-carbonsäuremethylester
1-Butyl-4-(4-chlor-benzyl)-5-{4-[(4-chlor-phenyl)-dimethylamino-methyl]-cyclohexylmethyl}-4,5-dihydro-1H-imidazol-4-carbonsäuremethylester
1,4-Dibutyl-5-{4-[(4-chlor-phenyl)-dimethylamino-methyl]-cyciohexylmethyl}-4,5-dihydro-1H-imidazol-4-carbonsäuremethylester
5-{4-[Dimethylamino-(3-fluor-phenyl)-methyl]-cyclohexyl}-4-isobutyl-1-phenethyl-4,5-dihydro-1H-imidazol-4-carbonsäuremethylester
4-(4-Chlor-benzyl)-5-{4-[dimethylamino-(3-fluor-phenyl)-methyl]-cyclohexyl}-1-phenethyl-4,5-dihydro-1H-imidazol-4-carbonsäuremethylester
4-Butyl-5-{4-[dimethylamino-(3-fluor-phenyl)-methyl]-cyclohexyl}-1-phenethyl-4,5-dihydro-1H-imidazol-4-carbonsäuremethylester
5-{4-[(4-Chlor-phenyl)-dimethylamino-methyl]-cyclohexyl}-4-isobutyl-1-phenethyl-4,5-dihydro-1H-imidazol-4-carbonsäuremethylester
4-(4-Chlor-benzyl)-5-{4-[(4-chlor-phenyl)-dimethylamino-methyl]-cyclohexyl}-1-phenethyl-4,5-dihydro-1H-imidazol-4-carbonsäuremethylester
4-Butyl-5-{4-[(4-chlor-phenyl)-dimethylamino-methyl]-cyclohexyl}-1-phenethyl-4,5-dihydro-1H-imidazol-4-carbonsäuremethylester
5-{4-[(4-Chlorphenyl)-dimethylamino-methyl]-cyclohexylmethyl}-4-isobutyl-1-phenethyl-4,5-dihydro-1H-imidazol-4cärbonsäuremethylester
4-(4-Chlor-benzyl)-5-{4-[(4-chlor-phenyl)-dimethylamino-methyl]-cyclohexylmethyl}-1-phenethyl-4,5-dihydro-1H-imidazol-4-carbonsäuremethylester
4-Butyl-5-{4-[(4-chlor-phenyl)-dimethylamino-methyl]-cyclohexylmethyl}-1-phenethyl-4,5-dihydro-1H-imidazol-4-carbonsäuremethylester
1-Benzyl-5-{4-[dimethylamino-(3-fluor-phenyl)-methyl]-cyclohexyl}-4-isobutyl-4,5-dihydro-1H-imidazol-4-carbonsäuremethylester
1-Benzyl-4-(4-chlor-benzyl)-5-{4-[dimethylamino-(3-fluor-phenyl)-methyl]-cyclohexyl}-4,5-dihydro-1H-imidazol-4-carbonsäuremethylester
1-Benzyl-5-{4-[(4-chlor-phenyl)-dimethylamino-methyl]-cyclohexyl}-4-isobutyl-4,5-dihydro-1H-imidazol-4-carbonsäuremethylester
1-Benzyl-4-(4-chlor-benzyl)-5-{4-[(4-chlor-phenyl)-dimethylamino-methyl]-cyclohexyl}-4,5-dihydro-1H-imidazol-4-carbonsäuremethylester
1-Benzyl-5-{4-[(4-chlor-phenyl)-dimethylamino-methyl]-cyclohexylmethyl}-4-isobutyl-4,5-dihydro-1H-imidazol-4-carbonsäuremethylester
5-{4-[(4-Chlor-phenyl)-dimethylamino-methyl]-cyclohexyl}-1-cyclohexyl-4-(2-methoxycarbonyl-ethyl)-4,5-dihydro-1H-imidazol-4-carbonsäuremethylester
4-Benzylsulfanylmethyl-5-{4-[(4-chlor-phenyl)-dimethylamino-methyl]-cyclohexyl}-1-cyclohexyl-4,5-dihydro-1H-imidazol-4-carbonsäuremethylester
5-{4-[(4-Chlor-phenyl)-dimethylamino-methyl]-cyclohexylmethyl}-1-cyclohexyl-4-(2-methoxycarbonyl-ethyl)-4,5-dihydro-1H-imidazol-4-carbonsäuremethylester
1-Benzyl-5-{4-[dimethylamino-(3-fluor-phenyl)-methyl]-cyclohexylmethyl}-4-propyl-4,5-dihydro-1H-imidazol-4-carbonsäuremethylester
5-{4-[(4-Chlor-phenyl)-dimethylamino-methyl]-cyclohexylmethyl}-1-cyclohexyl-4-methylsulfanylmethyl-4,5-dihydro-1H-imidazol-4-carbonsäuremethylester
4-Benzylsulfanylmethyl-5-{4-[(4-chlor-phenyl)-dimethylamino-methyl]-cyclohexylmethyl}-1-cyclohexyl-4,5-dihydro-1H-imidazol-4-carbonsäuremethylester
5-{4-[Dimethylamino-(3-fluor-phenyl)-methyl]-cyclohexyl}-4-methylsulfanylmethyl-1-propyl-4,5-dihydro-1H-imidazol-4-carbonsäuremethylester
4-Benzylsulfanylmethyl-5-{4-[dimethylamino-(3-fluor-phenyl)-methyl]-cyclohexyl}-1-propyl-4,5-dihydro-1H-imidazol-4-carbonsäuremethylester
5-{4-[(4-Chlor-phenyl)-dimethylamino-methyl]-cyclohexyl}-4-(2-methoxycarbonylethyl)-1-propyl-4,5-dihydro-1H-imidazol-4-carbonsäuremethylester
4-Benzylsulfanylmethyl-5-{4-[(4-chlor-phenyl)-dimethylamino-methyl]-cyclohexyl}-1-propyl-4,5-dihydro-1H-imidazol-4-carbonsäuremethylester
5-{4-[(4-Chlor-phenyl)-dimethylamino-methyl]-cyclohexylmethyl}-4-(2-methoxycarbonyl-ethyl)-1-propyl-4,5-dihydro-1H-imidazol-4-carbonsäuremethylester
4-sec-Butyl-5-{4-[(4-chlor-phenyl)-dimethylamino-methyl]-cyclohexylmethyl}-1-propyl-4,5-dihydro-1H-imidazol-4-carbonsäuremethylester
5-{4-[(4-Chlor-phenyl)-dimethylamino-methyl]-cycloheoylmethyl}-4-methylsulfanylmethyl-1-propyl-4,5-dihydro-1H-imidazol-4-carbonsäuremethylester
4-Benzylsulfanylmethyl-5-{4-[(4-chlor-phenyl)-dimethylamino-methyl]-cyclohexylmethyl}-1-propyl-4,5-dihydro-1H-imidazol-4-carbonsäuremethylester
1-Butyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
1-Butyl-5-{4-[dimethylamino-(3-fluor-phenyl)-methyl]-cyclohexyl}-4-methylsulfanylmethyl-4,5-dihydro-1H-imidazol-4-carbonsäuremethylester
4-Benzylsulfanylmethyl-1-butyl-5-{4-[dimethylamino-(3-fluor-phenyl)-methyl]-cyclohexyl}-4,5-dihydro-1H-imidazol-4-carbonsäuremethylester
1-Butyl-5-{4-[(4-chlor-phenyl)-dimethylamino-methyl]-cyclohexyl}-4-(2-methoxycarbonyl-ethyl)-4,5-dihydro-1H-imidazol-4-carbonsäuremethylester
4-Benzylsulfanylmethyl-1-butyl-5-{4-[(4-chlor-phenyl)-dimethylamino-methyl]-cyclohexyl}-4,5-dihydro-1H-imidazol-4-carbonsäuremethylester
1-Butyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester
5-[4-(1-Dimethylamino-3-phenyl-propyl)-cyclohexylmethyl]-4-(2-methoxycarbonylethyl)-1-propyl-4,5-dihydro-1H-imidazol-4-carbonsäuremethylester
1-Butyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
1-Butyl-4-sec-butyl-5-{4-[dimethylamino-(3-fluor-phenyl)-methyl]-cyclohexylmethyl}-4,5-dihydro-1H-imidazol-4-carbonsäuremethylester
1-Butyl-5-{4-[dimethylamino-(3-fluor-phenyl)-methyl]-cyclohexylmethyl}-4-methylsulfanylmethyl-4,5-dihydro-1H-imidazol-4-carbonsäuremethylester
4-Benzylsulfanylmethyl-1-butyl-5-{4-[dimethylamino-(3-fluor-phenyl)-methyl]-cyclohexylmethyl}-4,5-dihydro-1H-imidazol-4-carbonsäuremethylester
1-Butyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
1-Butyl-5-{4-[dimethylamino-(4-fluor-phenyl)-methyl]-cyclohexylmethyl}-4-methylsulfanylmethyl-4,5-dihydro-1H-imidazol-4-carbonsäuremethylester
1-Butyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester
4-Benzylsulfanylmethyl-1-butyl-5-[4-(1-dimethylamino-3-phenyl-propyl)-cyclohexylmethyl]-4,5-dihydro-1H-imidazol-4-carbonsäuremethylester
5-{4-[Dimethylamino-(3-fluor-phenyl)-methyl]-cyclohexylmethyl}-4-(2-methoxycarbonyl-ethyl)-1-phenethyl-4,5-dihydro-1H-imidazol-4-carbonsäuremethylester
4-sec-Butyl-5-{4-[dimethylamino-(3-fluor-phenyl)-methyl]-cyclohexylmethyl}-1-phenethyl-4,5-dihydro-1H-imidazol-4-carbonsäuremethylester
4-Benzylsulfanylmethyl-5-{4-[dimethylamino-(3-fluor-phenyl)-methyl]-cyclohexylmethyl}-1-phenethyl-4,5-dihydro-1H-imidazol-4-carbonsäuremethylester
5-{4-[Dimethylamino-(4-fluor-phenyl)-methyl]-cyclohexylmethyl}-4-(2-methoxycarbonyl-ethyl)-1-phenethyl-4,5-dihydro-1H-imidazol-4-carbonsäuremethylester
S-[4-(1-Dimethylamino-3-phenyl-propyl)-cyclohexylmethyl]-4-(2-methoxycarbonylethyl)-1-phenethyl-4,5-dihydro-1H-imidazol-4-carbonsäuremethylester
1-Benzyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
1-Benzyl-4-benzylsulfanylmethyl-5-{4-[dimethylamino-(3-fluor-phenyl)-methyl]-cyclohexylmethyl}-4,5-dihydro-1H-imidazol-4-carbonsäuremethylester
1-Benzyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
1-Butyl-4-sec-butyl-5-{4-[(4-chlor-phenyl)-dimethylamino-methyl]-cyclohexylmethyl}-4,5-dihydro-1H-imidazol-4-carbonsäuremethylester
1-Butyl-5-{4-[(4-chlor-phenyl)-dimethylamino-methyl]-cyclohexylmethyl}-4-methylsulfanylmethyl-4,5-dihydro-1H-imidazol-4-carbonsäuremethylester
4-Benzylsulfanylmethyl-1-butyl-5-{4-[(4-chlor-phenyl)-dimethylamino-methyl]-cyclohexylmethyl}-4,5-dihydro-1H-imidazol-4-carbonsäuremethylester
5-{4-[Dimethylamino-(3-fluor-phenyl)-methyl]-cyclohexyl}-4-(2-methoxycarbonylethyl)-1-phenethyl-4,5-dihydro-1H-imidazol-4-carbonsäuremethylester
4-Benzylsulfanylmethyl-5-{4-[dimethylamino-(3-fluor-phenyl)-methyl]-cyclohexyl}-1-phenethyl-4,5-dihydro-1H-imidazol-4-carbonsäuremethylester
4-Benzylsulfanylmethyl-5-{4-[(4-chlorphenyl)-dimethylamino-methyl]-cyclohexyl}-1-phenethyl-4,5-dihydro-1H-imidazol-4-carbonsäuremethylester
5-{4-[(4-Chior-phenyl)-dimethylamino-methyl]-cyclohexylmethyl}-4-(2-methoxycarbonyl-ethyl)-1-phenethyl-4,5-dihydro-1H-imidazol-4-carbonsäuremethylester
1-Benzyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
1-Benzyl-5-{4-[dimethylamino-(3-fluor-phenyl)-methyl]-cyclohexyl}-4-methylsulfanylmethyl-4,5-dihydro-1H-imidazol-4-carbonsäuremethylester
1-Benzyl-4-benzylsulfanylmethyl-5-{4-[dimethylamino-(3-fluor-phenyl)-methyl]-cyclohexyl}-4,5-dihydro-1H-imidazol-4-carbonsäuremethylester
1-Benzyl-4-benzylsulfanylmethyl-5-{4-[(4-chlor-phenyl)-dimethylamino-methyl]-cyclohexyl}-4,5-dihydro-1H-imidazol-4-carbonsäuremethylester
1-Benzyl-5-{4-[(4-chlor-phenyl)-dimethylamino-methyl]-cyclohexylmethyl}-4-(2-methoxycarbonyl-ethyl)-4,5-dihydro-1H-imidazol-4-carbonsäuremethylester
1-Benzyl-5-{4-[(4-chlor-phenyl)-dimethylamino-methyl]-cyclohexylmethyl}-4-methylsulfanylmethyl-4,5-dihydro-1H-imidazol-4-carbonsäuremethylester
1-Benzyl-4-benzylsulfanylmethyl-5-{4-[(4-chlor-phenyl)-dimethylamino-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexylmethyl}-4-propyl-4,5-dihydro-1H-imidazol-4-carbonsäuremethylester
1-Cyclohexyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester
1-Cyclohexyl-5-{4-[dimethylamino-(3-fluor-phenyl)-methyl]-cyclohexylmethyl}-4-methyl-4,5-dihydro-1H-imidazol-4-carbonsäuremethylester
1-Cyclohexyl-5-{4-[dimethylamino-(4-fluor-phenyl)-methyl]-cyclohexylmethyl}-4-propyl-4,5-dihydro-1H-imidazol-4-carbonsäuremethylester
1-Cyclohexyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester
1-Cyclohexyl-5-{4-[dimethylamino-(4-fluorphenyl)-methyl]-cyclohexylmethyl}-4-methyl-4,5-dihydro-1H-imidazol-4-carbonsäuremethylester
1-Cyclohexyl-5-[4-(1-dimethylamino-3-phenyl-propyl)-cyclohexylmethyl]-4-propyl-4,5-dihydro-1H-imidazol-4-carbonsäuremethylester
1-Cyclohexyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
1-Cyclohexyl-5-[4-(1-dimethylamino-3-phenyl-propyl)-cyclohexylmethyl]-4-methyl-4,5-dihydro-1H-imidazol-4-carbonsäuremethylester
5-{4-[Dimethylamino-(3-fluor-phenyl)-methyl]-cyclohexylmethyl}-1,4-dipropyl-4,5-dihydro-1H-imidazol-4-carbonsäuremethylester
5-{4-[Dimethylamino-(3-fluor-phenyl)-methyl]-cyclohexylmethyl}-4-(2-methylsulfanyl-ethyl)-1-propyl-4,5-dihydro-1H-imidazol-4-carbonsäuremethylester
1-Benzyl-4-(4-chlor-benzyl)-5-{4-[(4-chlor-phenyl)-dimethylamino-methyl]-cyclohexylmethyl}-4,5-dihydro-1H-imidazol-4-carbonsäuremethylester
1-Benzyl-4-butyl-5-{4-[(4-chlor-phenyl)-dimethylamino-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexyl}-4-(2-methoxycarbonyl-ethyl)-4,5-dihydro-1H-imidazol-4-carbonsäuremethylester
4-Benzylsulfanylmethyl-1-cyclohexyl-5-{4-[dimethylamino-(3-fluor-phenyl)-methyl]-cyclohexyl}-4,5-dihydro-1H-imidazol-4-carbonsäuremethylester
5-{4-[Dimethylamino-(3-fluor-phenyl)-methyl]-cyclohexylmethyl}-4-methyl-1-propyl-4,5-dihydro-1H-imidazol-4-carbonsäuremethylester
5-{4-[Dimethylamino-(4-fluor-phenyl)-methyl]-cyclohexylmethyl}-1,4-dipropyl-4,5-dihydro-1H-imidazol-4-carbonsäuremethylester
5-{4-[Dimethylamino-(4-fluor-phenyl)-methyl]-cyclohexylmethyl}-4-(2-methylsulfanyl-ethyl)-1-propyl-4,5-dihydro-1H-imidazol-4-carbonsäuremethylester
5-[4-(1-Dimethylamino-3-phenyl-propyl)-cyclohexylmethyl]-1,4-dipropyl-4,5-dihydro-1H-imidazol-4-carbonsäuremethylester
5-[4-(1-Dimethylamino-3-phenyl-propyl)-cyclohexylmethyl]-4-(2-methylsulfanyl-ethyl)-1-propyl-4,5-dihydro-1H-imidazol-4-carbonsäuremethylester
1-Butyl-5-{4-[dimethylamino-(3-fluor-phenyl)-methyl]-cyclohexylmethyl}-4-propyl-4,5-dihydro-1H-imidazol-4-carbonsäuremethylester
1-Butyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester
1-Butyl-5-{4-[dimethylamino-(3-fluor-phenyl)-methyl]-cyclohexylmethyl}-4-methyl-4,5-dihydro-1H-imidazol-4-carbonsäuremethylester
1-Butyl-5-{4-[dimethylamino-(4-fluor-phenyl)-methyl]-cyclohexylmethyl}-4-propyl-4,5-dihydro-1H-imidazol-4-carbonsäuremethylester
1-Butyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester
1-Butyl-5-[4-(1-dimethylamino-3-phenyl-propyl)-cyclohexylmethyl]-4-propyl-4,5-dihydro-1H-imidazol-4-carbonsäuremethylester
1-Butyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
5-{4-[Dimethylamino-(3-fluor-phenyl)-methyl]-cyclohexylmethyl}-1-phenethyl-4-propyl-4,5-dihydro-1H-imidazol-4-carbonsäuremethylester
5-{4-[Dimethylamino-(3-fluor-phenyl)-methyl]-cyclohexylmethyl}-4-(2-methylsulfanyl-ethyl)-1-phenethyl-4,5-dihydro-1H-imidazol-4-carbonsäuremethylester
5-{4-[Dimethylamino-(3-fluor-phenyl)-methyl]-cyclohexylmethyl}-4-methyl-1-phenethyl-4,5-dihydro-1H-imidazol-4-carbonsäuremethylester
5-{4-[Dimethylamino-(4-fluor-phenyl)-methyl]-cyclohexylmethyl}-1-phenethyl-4-propyl-4,5-dihydro-1H-imidazol-4-carbonsäuremethylester
5-{4-[Dimethylamino-(4-fluor-phenyl)-methyl]-cyclohexylmethyl}-4-(2-methylsulfanyl-ethyl)-1-phenethyl-4,5-dihydro-1H-imidazol-4-carbonsäuremethylester
5-{4-[Dimethylamino-(4-fluorphenyl)-methyl]-cyclohexylmethyl}-4-methyl-1-phenethyl-4,5-dihydro-1H-imidazol-4-carbonsäuremethylester
5-[4-(1-Dimethylamino-3-phenyl-propyl)-cyclohexylmethyl]-1-phenethyl-4-propyl-4,5-dihydro-1H-imidazol-4-carbonsäuremethylester
5-[4-(1-Dimethylamino-3-phenyl-propyl)-cyclohexylmethyl]-4-(2-methylsulfanyl-ethyl)-1-phenethyl-4,5-dihydro-1H-imidazol-4-carbonsäuremethylester
5-[4-(1-Dimethylamino-3-phenyl-propyl)-cyclohexylmethyl]-4-methyl-1-phenethyl-4,5-dihydro-1H-imidazol-4-carbonsäuremethylester
1-Benzyl-5-{4-[dimethylamino-(3-fluor-phenyl)-methyl]-cyclohexylmethyl}-4-propyl-4,5-dihydro-1H-imidazol-4-carbonsäuremethylester
1-Benzyl-5-{4-[dimethylamino-(3-fluor-phenyl)-methyl]-cyclohexylmethyl}-4-(2-methylsulfanyl-ethyl)-4,5-dihydro-1H-imidazol-4-carbonsäuremethylester
1-Benzyl-5-{4-[dimethylamino-(3-fluor-phenyl)-methyl]-cyclohexylmethyl}-4-methyl-4,5-dihydro-1H-imidazol-4-carbonsäuremethylester
1-Benzyl-5-{4-[dimethylamino-(4-fluor-phenyl)-methyl]-cyclohexylmethyl}-4-propyl-4,5-dihydro-1H-imidazol-4-carbonsäuremethylester
1-Benzyl-5-{4-[dimethylamino-(4-fluor-phenyl)-methyl]-cyclohexylmethyl}-4-(2-methylsulfanyl-ethyl)-4,5-dihydro-1H-imidazol-4-carbonsäuremethylester
1-Benzyl-5-{4-[dimethylamino-(4-fluor-phenyl)-methyl]-cyclohexylmethyl}-4-methyl-4,5-dihydro-1H-imidazol-4-carbonsäuremethylester
1-Benzyl-5-[4-(1-dimethylamino-3-phenyl-propyl)-cyclohexylmethyl]-4-propyl-4,5-dihydro-1H-imidazol-4-carbonsäuremethylester
1-Benzyl-5-[4-(1-dimethylamino-3-phenyl-propyl)-cyclohexylmethyl]-4-(2-methylsulfanyl-ethyl)-4,5-dihydro-1H-imidazol-4-carbonsäuremethylester
1-Benzyl-5-[4-(1-dimethylamino-3-phenyl-propyl)-cyclohexylmethyl]-4-methyl-4,5-dihydro-1H-imidazol-4-carbonsäuremethylester
1-Cyclohexyl-5-{4-[dimethylamino-(3-fluor-phenyl)-methyl]-cyclohexylmethyl}-4-(2-methoxycarbonyl-ethyl)-4,5-dihydro-1H-imidazol-4-carbonsäuremethylester
4-*sec*-Butyl-1-cyclohexyl-5-{4-[dimethylamino-(3-fluor-phenyl)-methyl]-cyclohexylmethyl}-4,5-dihydro-1H-imidazol-4-carbonsäuremethylester
1-Cyclohexyl-5-{4-[dimethylamino-(3-fluor-phenyl)-methyl]-cyclohexylmethyl}-4-methylsulfanylmethyl-4,5-dihydro-1H-imidazol-4-carbonsäuremethylester
4-Benzylsulfanylmethyl-1-cyclohexyl-5-{4-[dimethylamino-(3-fluor-phenyl)-methyl]-cyclohexylmethyl}-4,5-dihydro-1H-imidazol-4-carbonsäuremethylester
1-Cyclohexyl-5-{4-[dimethylamino-(4-fluor-phenyl)-methyl]-cyclohexylmethyl}-4-(2-methoxycarbonyl-ethyl)-4,5-dihydro-1H-imidazol-4-carbonsäuremethylester
4-sec-Butyl-1-cyclohexyl-5-{4-[dimethylamino-(4-fluor-phenyl)-methyl]-cyclohexylmethyl}-4,5-dihydro-1H-imidazol-4-carbonsäuremethylester
1-Cyclohexyl-5-{4-[dimethylamino-(4-fluor-phenyl)-methyl]-cyclohexylmethyl}-4-methylsulfanylmethyl-4,5-dihydro-1H-imidazol-4-carbonsäuremethylester
1-Cyclohexyl-5-[4-(1-dimethylamino-3-phenyl-propyl)-cyclohexylmethyl]-4-(2-methoxycarbonyl-ethyl)-4,5-dihydro-1H-imidazol-4-carbonsäuremethylester
1-Cyclohexyl-5-[4-(1-dimethylamino-3-phenyl-propyl)-cyclohexylmethyl]-4-methylsulfanylmethyl-4,5-dihydro-1H-imidazol-4-carbonsäuremethylester
4-Benzylsulfanylmethyl-1-cyclohexyl-5-[4-(1-dimethylamino-3-phenyl-propyl)-cyclohexylmethyl]-4,5-dihydro-1H-imidazol-4-carbonsäuremethylester
5-{4-[Dimethylamino-(3-fluor-phenyl)-methyl]-cyclohexylmethyl}-4-(2-methoxycarbonyl-ethyl)-1-propyl-4,5-dihydro-1H-imidazol-4-carbonsäuremethylester
5-{4-[Dimethylamino-(3-fluor-phenyl)-methyl]-cyclohexylmethyl}-4-methylsulfanylmethyl-1-propyl-4,5-dihydro-1H-imidazol-4-carbonsäuremethylester
5-{4-[Dimethylamino-(4-fluor-phenyl)-methyl]-cyclohexylmethyl}-4-(2-methoxycarbonyl-ethyl)-1-propyl-4,5-dihydro-1Himidazol-4-carbonsäuremethylester
5-{4-[Dimethylamino-(4-fluor-phenyl)-methyl]-cyclohexylmethyl}-4-methylsulfanylmethyl-1-propyl-4,5-dihydro-1H-imidazol-4-carbonsäuremethylester
4-Benzylsulfanylmethyl-5-{4-[dimethylamino-(4-fluor-phenyl)-methyl]-cyclohexylmethyl}-1-propyl-4,5-dihydro-1H-imidazol-4-carbonsäuremethylester
Methyl-4-*sec*-butyl-5-((4-((4-chlorphenyl)(dimethylamino)methyl)cyclohexyl)methyl)-1-cyclohexyl-4,5-dihydro-1H-imidazol-4-carboxylat
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

11. Verfahren zur Herstellung eines erfindungsgemäßen Imidazolin-Derivates, wobei man Amine der allgemeinen Formel A mit Aldehyden der allgemeinen Formel B und Isonitrilestern der allgemeinen Formel C in einem organischen Lösungsmittel, beispielsweise Ethanol oder Methanol bei einer Temperatur von 20-100°C nach dem folgenden Syntheseschema zu substituierten Imidazolin-Derivaten der allgemeinen Formel I umsetzt.

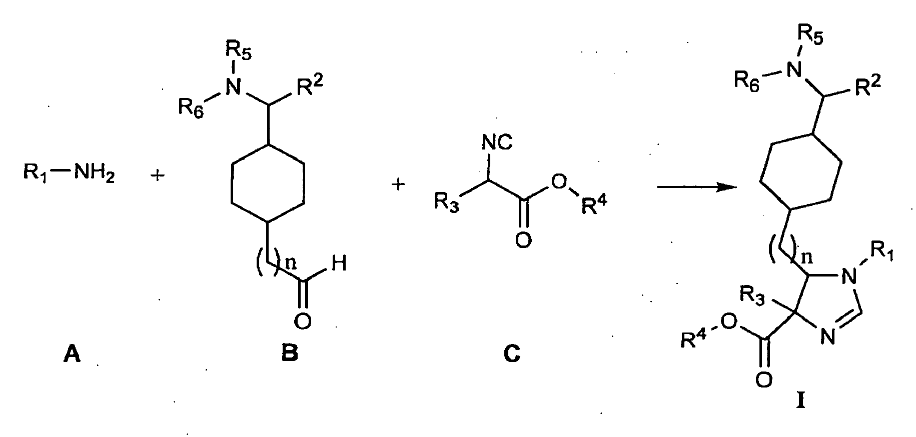

12. Substituierter Aldehyd der allgemeinen Formel B 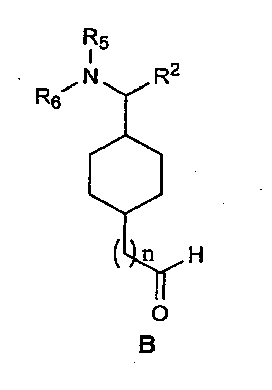 worin die Reste R², R⁵, R⁶ sowie n die in den Ansprüchen 1, 4, 5 und 9 angegebene Bedeutung haben.

13. Substituierte Aldehyde gemäß Anspruch 12 aus der Gruppe
4-[Dimethylamino-(4-fluor-phenyl)-methyl]-cyclohexanecarbaldehyd
4-(1-Dimethylamino-3-phenyl-propyl)-cyclohexanecarbaldehyd
4-[Dimethylamino-(3-fluor-phenyl)-methyl]-cyclohexanecarbaldehyd
4-[(4-Chlor-phenyl)-dimethylamino-methyl]-cyclohexanecarbaldehyd
{4-[(4-Chlor-phenyl)-dimethylamino-methyl]-cyclohexyl}-acetaldehyd
{4-[Dimethylamino-(3-fluor-phenyl)-methyl]-cyclohexyl}-acetaldehyd
4-(Dimethylamino-thiophen-2-yl-methyl)-cyclohexanecarbaldehyd
{4-[Dimethylamino-(4-fluor-phenyl)-methyl]-cyclohexyl}-acetaldehyd
[4-(Dimethylamino-thiophen-2-yl-methyl)-cyclohexyl]-acetaldehyd
[4-(1-Dimethylamino-3-phenyl-propyl)-cyclohexyl]-acetaldehyd
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

14. Arzneimittel enthaltend wenigstens ein substituiertes Imidazolin-Derivat gemäß Anspruch 1 oder einen substituierten Aldehyd gemäß Anspruch 12, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, sowie gegebenenfalls enthaltend geeignete Zusatz- und/oder Hilfsstoffe und/oder gegebenenfalls weiterer Wirkstoffe.

15. Verwendung eines substituierten Imidazolin-Derivats gemäß Anspruch 1 oder eines substituierten Aldehyds gemäß Anspruch 12, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Schmerz, insbesondere von akutem, neuropathischem oder chronischem Schmerz.

16. Verwendung eines substituierten Imidazolin-Derivats gemäß Anspruch 1 oder eines substituierten Aldehyds gemäß Anspruch 12, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Depressionen, Harninkontinenz, Diarrhöe, Pruritus, Alkohol- und Drogenmißbrauch, Medikamentenabhängigkeit, Antriebslosigkeit und/oder zur Anxiolyse.

## Claims

1. Substituted imidazoline derivatives of the general Formula I, 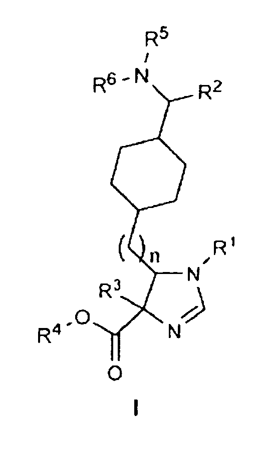 , wherein
n is 0, 1 or 2;
R¹ denotes C₁₋₈-alkyl, saturated or unsaturated, branched or unbranched, unsubstituted or monosubstituted or polysubstituted; C₃₋₈-cycloalkyl or a corresponding cycloalkyl in which one or two carbon atoms is/are replaced by a heteroatom S, N or O, saturated or unsaturated, unsubstituted or monosubstituted or polysubstituted;
wherein monosubstituted or polysubstituted in each case denotes the substitution of one or more hydrogen radicals by F, Cl, Br, I, -CN, NH₂, NH-C₁₋₆-alkyl, NH-C₁₋₆-alkyl-OH, N(C₁₋₆-alkyl)₂, N(C₁₋₆-alkyl-OH)₂, NO₂, SH, S-C₁₋₆-alkyl, S-benzyl, O-C₁₋₆-alkyl, OH, O-C₁₋₆-alkyl-OH, =O, C₁₋₆-alkyl, benzyl, O-benzyl, C(=O)C₁₋₆-alkyl, CO₂H, CO₂-C₁₋₆-alkyl, and polysubstitution takes place either on different atoms or on the same atom with the same or with different substituents;
an aryl or heteroaryl radical bonded via a C₁₋₃-alkyl chain, in each case unsubstituted or monosubstituted or polysubstituted;
wherein monosubstituted or polysubstituted in each case denotes the substitution of one or more hydrogen atoms of the ring system by F, Cl, Br, I, CN, NH₂, NH-C₁₋₆-alkyl, NH-C₁₋₆-alkyl-OH, N(C₁₋₆-alkyl)₂, N(C₁₋₆-alkyl-OH)₂, NO₂, SH, S-C₁₋₆-alkyl, OH, O-C₁₋₆-alkyl, O-C₁₋₆-alkyl-OH, C(=O)C₁₋₆-alkyl, CO₂H, CO₂-C₁₋₆-alkyl, CF₃, C₁₋₆-alkyl, and polysubstitution takes place on one atom or on different atoms with the same or with different substituents;
R² denotes aryl or heteroaryl, in each case unsubstituted or monosubstituted or polysubstituted as defined above for the aryl or heteroaryl radical; an aryl radical bonded via a C₁₋₃-alkyl chain, in each case unsubstituted or monosubstituted or polysubstituted as defined above for the aryl radical;
R³ denotes C₁₋₈-alkyl, saturated or unsaturated, branched or unbranched, unsubstituted or monosubstituted or polysubstituted as defined above for C₁₋₈-alkyl; an aryl radical bonded via a C₁₋₃-alkyl chain, in each case unsubstituted or monosubstituted or polysubstituted as defined above for the aryl radical;
R⁴ denotes H, C₁₋₄-alkyl, or an aryl radical bonded via a C₁₋₃-alkyl chain;
R⁵ and R⁶ independently of one another denote H; C₁₋₆-alkyl, in each case saturated or unsaturated, branched or unbranched, wherein R⁵ and R⁶ do not simultaneously denote H,
or the radicals R⁵ and R⁶ together denote CH₂CH₂OCH₂CH₂, or (CH₂)₃₋₆,
in the form of the racemate; in the form of the enantiomers, diastereomers, mixtures of the enantiomers or diastereomers or in the form of an individual enantiomer or diastereomer; in the form of the bases and/or salts of physiological compatible acids.

2. Substituted imidazoline derivatives according to claim 1, wherein R¹ denotes C₁₋₈-alkyl, branched or unbranched, unsubstituted or monosubstituted or polysubstituted with F, Cl, -CN, SH, S-C₁₋₆-alkyl, S-benzyl, OH, O-benzyl, O-C₁₋₆-alkyl, CO₂H, CO₂-C₁₋₆-alkyl; C₃₋₈-cycloalkyl, in each case unsubstituted or monosubstituted or polysubstituted with F, Cl, -CN, SH, S-C₁₋₆-alkyl, S-benzyl, OH, O-benzyl, O-C₁₋₆-alkyl, CO₂H, CO₂-C₁₋₆-alkyl; a phenyl radical bonded via a C₁₋₃-alkyl chain, in each case unsubstituted or monosubstituted or polysubstituted with F, Cl, CN, NO₂, SH, S-C₁₋₆-alkyl, OH, O-C₁₋₆-alkyl, CO₂H, CO₂-C₁₋₆-alkyl, CF₃, C₁₋₆-alkyl.

3. Substituted imidazoline derivatives according to claim 1, wherein R¹ denotes cyclohexyl, n-propyl, n-butyl, phenethyl or benzyl.

4. Substituted imidazoline derivatives according to one of claims 1 to 3, wherein R² denotes phenyl or thienyl, in each case unsubstituted or monosubstituted or polysubstituted with F, Cl, CN, NO₂, SH, S-C₁₋₆-alkyl, OH, O-C₁₋₆-alkyl, CO₂H, CO₂-C₁₋₆-alkyl, CF₃, C₁₋₆-alkyl; a phenyl radical bonded via a C₁₋₃-alkyl chain, in each case unsubstituted or monosubstituted or polysubstituted with F, Cl, CN, NO₂, SH, S-C₁₋₆-alkyl, OH, O-C₁₋₆-alkyl, CO₂H, CO₂-C₁₋₆-alkyl, CF₃, C₁₋₆-alkyl.

5. Substituted imidazoline derivatives according to one of claims 1 to 3, wherein R² denotes phenyl, unsubstituted or monosubstituted with Cl or F, or thienyl or phenethyl.

6. Substituted imidazoline derivatives according to one of claims 1 to 5, wherein R³ denotes C₁₋₈-alkyl, branched or unbranched, unsubstituted or monosubstituted or polysubstituted with F, Cl, -CN, SH, S-C₁₋₆-alkyl, S-benzyl, OH, O-benzyl, O-C₁₋₆-alkyl, CO₂H, CO₂-C₁₋₆-alkyl; a phenyl radical bonded via a C₁₋₃-alkyl chain, in each case unsubstituted or monosubstituted or polysubstituted with F, Cl, CN, NO₂, SH, S-C₁₋₆-alkyl, OH, O-C₁₋₆-alkyl, CO₂H, CO₂-C₁₋₆-alkyl, CF₃, C₁₋₆-alkyl.

7. Substituted imidazoline derivatives according to one of claims 1 to 5, wherein R³ denotes sec-butyl, iso-butyl, n-butyl, n-propyl, CH₃, CH₂ CH₂ COOCH₃, CH₂-S-benzyl, CH₂CH₂-S-CH₃, CH₂-S-CH₃ or 4-Cl-benzyl.

8. Substituted imidazoline derivatives according to one of claims 1 to 7, wherein R⁴ denotes CH₃.

9. Substituted imidazoline derivatives according to one of claims 1 to 8, wherein R⁵ and R⁶ denotes CH₃.

10. Substituted imidazoline derivatives according to one of claims 1 to 9, from the group:
1-cyclohexyl-5-{4-[dimethylamino-(3-fluorophenyl)-methyl]-cyclohexyl}-4-isobutyl-4,5-dihydro-1H-imidazole-4-carboxylic acid methyl ester
4-(4-chlorobenzyl)-1-cyclohexyl-5-{4-[dimethylamino-(3-fluorophenyl)-methyl]-cyclohexyl}-4,5-dihydro-1H-imidazole-4-carboxylic acid methyl ester
4-butyl-1-cyclohexyl-5-{4-[dimethylamino-(3-fluorophenyl)-methyl]-cyclohexyl}-4,5-dihydro-1H-imidazole-4-carboxylic acid methyl ester
5-{4-[(4-chlorophenyl)-dimethylaminomethyl]-cyclohexyl}-1-cyclohexyl-4-isobutyl-4,5-dihydro-1H-imidazole-4-carboxylic acid methyl ester
4-(4-chlorobenzyl)-5-{4-[(4-chlorophenyl)-dimethylaminomethyl]-cyclohexyl}-1-cyclohexyl-4,5-dihydro-1H-imidazole-carboxylic acid methyl ester
4-butyl-5-{4-[(4-chlorophenyl)-dimethylaminomethyl]-cyclohexyl}-1-cyclohexyl-4,5-dihydro-1H-imidazole-4-carboxylic carboxylic acid methyl ester
5-{4-[(4-chlorophenyl)-dimethylaminomethyl]-cyclohexylmethyl}-1-cyclohexyl-4-isobutyl-4,5-dihydro-1H-imidazole-4-carboxylic acid methyl ester
4-(4-chlorobenzyl)-5-{4-[(4-chlorophenyl)-dimethylaminomethyl]-cyclohexylmethyl}-1-cyclohexyl-4,5-dihydro-1H-imidazole-4-carboxylic acid methyl ester
4-butyl-5-{4-[(4-chlorophenyl)-dimethylaminomethyl]-cyclohexylmethyl}-1-cyclohexyl-4,5-dihydro-1H-imidazole-4-carboxylic acid methyl ester
4-(4-chlorobenzyl)-1-cyclohexyl-5-[4-(dimethylamino-thiophen-2-yl-methyl)-cyclohexylmethyl]-4,5-dihydro-1H-imidazole-4-carboxylic acid methyl ester
5-{4-[dimethylamino-(3-fluorophenyl)-methyl]-cyclohexyl}-4-isobutyl-1-propyl-4,5-dihydro-1H-imidazole-4-carboxylic acid methyl ester
4-(4-chlorobenzyl)-5-{4-[dimethylamino-(3-fluorophenyl)-methyl]-cyclohexyl}-1-propyl-4,5-dihydro-1H-imidazole-4-carboxylic acid methyl ester
4-butyl-5-{4-[dimethylamino-(3-fluorophenyl)-methyl]-cyclohexyl}-1-propyl-4,5-dihydro-1H-imidazole-4-carboxylic acid methyl ester
5-{4-[(4-chlorophenyl)-dimethylaminomethyl)-cyclohexyl}-4-isobutyl-1-propyl-4,5-dihydro-1H-imidazole-4-carboxylic acid methyl ester
4-(4-chlorobenzyl)-5-{4-[(4-chlorophenyl)-dimethylaminomethyl]-cyclohexyl}-1-propyl-4,5-dihydro-1H-imidazole-4-carboxylic acid methyl ester
5-(4-[(4-chlorophenyl)-dimethylaminomethyl)-cyclohexylmethyl}-4-isobutyl-1-propyl-4,5-dihydro-1H-imidazole-4-carboxylic acid methyl ester
4-(4-chlorobenzyl)-5-{4-[(4-chlorophenyl)-dimethylaminomethyl]-cyclohexylmethyl}-1-propyl-4,5-dihydro-1H-imidazole-4-carboxylic acid methyl ester
4-butyl-5-{4-[(4-chlorophenyl)-dimethylaminomethyl]-cyclohexylmethyl}-1-propyl-4,5-dihydro-1H-imidazole-4-carboxylic acid methyl ester
1-butyl-5-{4-[dimethylamino-(3-fluorophenyl)-methyl]-cyclohexyl}-4-isobutyl-4,5-dihydro-1H-imidazole-4-carboxylic acid methyl ester
1-butyl-4-(4-chlorobenzyl)-5-{4-[dimethylamino-(3-fluorophenyl)-methyl]-cyclohexyl}-4,5-dihydro-1H-imidazole-4-carboxylic acid methyl ester
1-butyl-5-{4-[(chlorophenyl)-dimethylaminomethyl]-cyclohexyl}-4-isobutyl-4,5-dihydro-1H-imidazole-4-carboxylic acid methyl ester
1-butyl-4-(4-chlorobenzyl)-5-{4-[(4-chlorophenyl)-dimethylaminomethyl]-cyclohexyl}-4,5-dihydro-1H-imidazole-4-carboxylic acid methyl ester
1,4-dibutyl-5-{4-[(4-chlorophenyl)-dimethylaminomethyl]-cyclohexyl}-4,5-dihydro-1H-imidazole-4-carboxylic acid methyl ester
1-butyl-5-{4-[(chlorophenyl)-dimethylaminomethyl]-cyclohexylmethyl}-4-isobutyl-4,5-dihydro-1H-imidazole-4-carboxylic acid methyl ester
1-butyl-4-(4-chlorobenzyl)-5-{4-[(4-chlorophenyl)-dimethylaminomethyl]-cyclohexylmethyl}-4,5-dihydro-1H-imidazole-4-carboxylic acid methyl ester
1,4-dibutyl-5-{4-[(4-chlorophenyl)-dimethylaminomethyl]-cyclohexylmethyl}-4,5-dihydro-1H-imidazole-4-carboxylic acid methyl ester
5-{4-[dimethylamino-(3-fluorophenyl)-methyl]-cyclohexyl}-4-isobutyl-1-phenethyl-4,5-dihydro-1H-imidazole-4-carboxylic acid methyl ester
4-(4-chlorobenzyl)-5-{4-[dimethylamino-(3-fluorophenyl)-methyl]-cyclohexyl}-1-phenethyl-4,5-dihydro-1H-imidazole-4-carboxylic acid methyl ester
4-butyl-5-{4-[dimethylamino-(3-fluorophenyl)-methyl]-cyclohexyl}-1-phenethyl-4,5-dihydro-1H-imidazole-4-carboxylic acid methyl ester
5-{4-[(4-chlorophenyl)-dimethylaminomethyl]-cyclohexyl}-4-isobutyl-1-phenethyl-4,5-dihydro-1H-imidazole-4-carboxylic acid methyl ester
4-(4-chlorobenzyl)-5-{4-[(4-chlorophenyl)-dimethylaminomethyl]-cyclohexyl}-1-phenethyl-4,5-dihydro-1H-imidazole-4-carboxylic acid methyl ester
4-butyl-5-{4-[(4-chlorophenyl)-dimethylaminomethyl]-cyclohexyl}-1-phenethyl-4,5-dihydro-1H-imidazole-4-carboxylic acid methyl ester
5-{4-[(4-chlorophenyl)-dimethylaminomethyl]-cyclohexylmethyl}-4-isobutyl-1-phenethyl-4,5-dihydro-1H-imidazole-4-carboxylic acid methyl ester
4-(4-chlorobenzyl)-5-{4-[(4-chlorophenyl)-dimethylaminomethyl]-cyclohexylmethyl}-1-phenethyl-4,5-dihydro-1H-imidazole-4-carboxylic acid methyl ester
4-butyl-5-{4-[(4-chlorophenyl)-dimethylaminomethyl]-cyclohexylmethyl}-1-phenethyl-4,5-dihydro-1H-imidazole-4-carboxylic acid methyl ester
1-benzyl-5-{4-[dimethylamino-(3-fluorophenyl)-methyl]-cyclohexyl}-4-isobutyl-4,5-dihydro-1H-imidazole-4-carboxylic acid methyl ester
1-benzyl-4-(4-chlorobenzyl)-5-{4-[dimethylamino-(3-fluorophenyl)-methyl]-cyclohexyl}-4,5-dihydro-1H-imidazole-4-carboxylic acid methyl ester
1-benzyl-5-{4-[(4-chlorophenyl)-dimethylaminomethyl]-cyclohexyl}-4-isobutyl-4,5-dihydro-1H-imidazole-4-carboxylic acid methyl ester
1-benzyl-4-(4-chlorobenzyl)-5-{4-[(4-chlorophenyl)-dimethylaminomethyl]-cyclohexyl}-4,5-dihydro-1H-imidazole-4-carboxylic acid methyl ester
1-benzyl-5-{4-[(4-chlorophenyl)-dimethylaminomethyl]-cyclohexylmethyl}-4-isobutyl-4,5-dihydro-1H-imidazole-4-carboxylic acid methyl ester
5-{4-[(4-chlorophenyl)-dimethylaminomethyl]-cyclohexyl}-1-cyclohexyl-4-(2-methoxycarbonylethyl)-4,5-dihydro-1H-imidazole-4-carboxylic acid methyl ester
4-benzylsulfanylmethyl-5-{4-[(4-chlorophenyl)-dimethylaminomethyl]-cyclohexyl}-1-cyclohexyl-4,5-dihydro-1H-imidazole-4-carboxylic acid methyl ester
5-{4-[(4-chlorophenyl)-dimethylaminomethyl]-cyclohexylmethyl}-1-cyclohexyl-4-(2-methoxycarbonylethyl)-4,5-dihydro-1H-imidazole-4-carboxylic acid methyl ester
1-benzyl-5-{4-[dimethylamino-(3-fluorophenyl)-methyl]-cyclohexylmethyl}-4-propyl-4,5-dihydro-1H-imidazole-4-carboxylic acid methyl ester
5-{4-[(4-chlorophenyl)-dimethylaminomethyl]-cyclohexylmethyl}-1-cyclohexyl-4-methylsulfanylmethyl-4,5-dihydro-1H-imidazole-4-carboxylic acid methyl ester
4-benzylsulfanylmethyl-5-{4-[(4-chlorophenyl)-dimethylaminomethyl]-cyclohexylmethyl}-1-cyclohexyl-4,5-dihydro-1H-imidazole-4-carboxylic acid methyl ester
5-{4-[dimethylamino-(3-fluorophenyl)-methyl]-cyclohexyl}-4-methylsulfanylmethyl-1-propyl-4,5-dihydro-1H-imidazole-4-carboxylic acid methyl ester
4-benzylsulfanylmethyl-5-{4-[dimethylamino-(3-fluorophenyl)-methyl]-cyclohexyl}-1-propyl-4,5-dihydro-1H-imidazole-4-carboxylic acid methyl ester
5-{4-[(4-chlorophenyl)-dimethylaminomethyl]-cyclohexyl}-4-(2-methoxycarbonylethyl)-1-propyl-4,5-dihydro-1H-imidazole-4-carboxylic acid methyl ester
4-benzylsulfanylmethyl-5-{4-[(4-chlorophenyl)-dimethylaminomethyl]-cyclohexyl}-1-propyl-4,5-dihydro-1H-imidazole-4-carboxylic acid methyl ester
5-{4-[(4-chlorophenyl)-dimethylaminomethyl]-cyclohexylmethyl}-4-(2-methoxycarbonylethyl)-1-propyl-4,5-dihydro-1H-imidazole-4-carboxylic acid methyl ester
4-sec-butyl-5-{4-[(4-chlorophenyl)-dimethylaminomethyl]-cyclohexylmethyl}-1-propyl-4,5-dihydro-1H-imidazole-4-carboxylic acid methyl ester
5-{4-[(4-chlorophenyl)-dimethylaminomethyl]-cyclohexylmethyl}-4-methylsulfanylmethyl-1-propyl-4,5-dihydro-1H-imidazole-4-carboxylic acid methyl ester
4-benzylsulfanylmethyl-5-{4-[(4-chlorophenyl)-dimethylaminomethyl]-cyclohexylmethyl}-1-propyl-4,5-dihydro-1H-imidazole-4-carboxylic acid methyl ester
1-butyl-5-{4-[dimethylamino-(3-fluorophenyl)-methyl]-cyclohexyl}-4-(2-methoxycarbonylethyl)-4,5-dihydro-1H-imidazole-4-carboxylic acid methyl ester
1-butyl-5-{4-[dimethylamino-(3-fluorophenyl)-methyl]-cyclohexyl}-4-methylsulfanylmethyl-4,5-dihydro-1H-imidazole-4-carboxylic acid methyl ester
4-benzylsulfanylmethyl-1-butyl-5-{4-[dimethylamino-(3-fluorophenyl)-methyl]-cyclohexyl}-4,5-dihydro-1H-imidazole-4-carboxylic acid methyl ester
1-butyl-5-{4-[(4-chlorophenyl)-dimethylaminomethyl]-cyclohexyl}-4-(2-methoxycarbonylethyl)-4,5-dihydro-1H-imidazole-4-carboxylic acid methyl ester
4-benzylsulfanylmethyl-1-butyl-5-{4-[(4-chlorophenyl)-dimethylaminomethyl]-cyclohexyl}-4,5-dihydro-1H-imidazole-4-carboxylic acid methyl ester
1-butyl-5-{4-[(4-chlorophenyl)-dimethylaminomethyl]-cyclohexylmethyl}-4-(2-methoxycarbonylethyl)-4,5-dihydro-1H-imidazole-4-carboxylic acid methyl ester
5-[4-(1-dimethylamino-3-phenylpropyl)-cyclohexylmethyl]-4-(2-methoxycarbonylethyl)-1-propyl-4,5-dihydro-1H-imidazole-4-carboxylic acid methyl ester
1-butyl-5-{4-[dimethylamino-(3-fluorophenyl)-methyl]-cyclohexylmethyl}-4-(2-methoxycarbonylethyl)-4,5-dihydro-1H-imidazole-4-carboxylic acid methyl ester
1-butyl-4-sec-butyl-5-{4-[dimethylamino-(3-fluorophenyl)-methyl]-cyclohexylmethyl}-4,5-dihydro-1H-imidazole-4-carboxylic acid methyl ester
1-butyl-5-{4-[dimethylamino-(3-fluorophenyl)-methyl]-cyclohexylmethyl}-4-methylsulfanylmethyl-4,5-dihydro-1H-imidazole-4-carboxylic acid methyl ester
4-benzylsulfanylmethyl-1-butyl-5-{4-[dimethylamino-(3-fluorophenyl)-methyl]-cyclohexylmethyl}-4,5-dihydro-1H-imidazole-4-carboxylic acid methyl ester
1-butyl-5-{4-[dimethylamino-(4-fluorophenyl)-methyl]-cyclohexylmethyl}-4-(2-methoxycarbonylethyl)-4,5-dihydro-1H-imidazole-4-carboxylic acid methyl ester
1-butyl-5-{4-[dimethylamino-(4-fluorophenyl)-methyl]-cyclohexylmethyl}-4-methylsulfanylmethyl-4,5-dihydro-1H-imidazole-4-carboxylic acid methyl ester
1-butyl-5-[4-(1-dimethylamino-3-phenylpropyl)-cyclohexyl methyl]-4-(2-methoxycarbonylethyl)-4,5-dihydro-1H-imidazole-4-carboxylic acid methyl ester
4-benzylsulfanylmethyl-1-butyl-5-[4-(1-dimethylamino-3-phenylpropyl)-cyclohexylmethyl]-4,5-dihydro-1H-imidazole-4-carboxylic acid methyl ester
5-{4-[dimethylamino-(3-fluorophenyl)-methyl]-cyclohexylmethyl}-4-(2-methoxycarbonylethyl)-1-phenethyl-4,5-dihydro-1H-imidazole-4-carboxylic acid methyl ester
4-sec-butyl-5-{4-[dimethylamino-(3-fluorophenyl)-methyl]-cyclohexylmethyl}-1-phenethyl-4,5-dihydro-1H-imidazole-4-carboxylic acid methyl ester
4-benzylsulfanylmethyl-5-{4-[dimethylamino-(3-fluorophenyl)-methyl]-cyclohexylmethyl}-1-phenethyl-4,5-dihydro-1H-imidazole-4-carboxylic acid methyl ester
5-{4-[dimethylamino-(4-fluorophenyl)-methyl]-cyclohexylmethyl}-4-(2-methoxycarbonylethyl)-1-phenethyl-4,5-dihydro-1H-imidazole-4-carboxylic acid methyl ester
5-[4-(1-dimethylamino-3-phenylpropyl)-cyclohexylmethyl]-4-(2-methoxycarbonylethyl)-1-phenethyl-4,5-dihydro-1H-imidazole-4-carboxylic acid methyl ester
1-benzyl-5-{4-[dimethylamino-(3-fluorophenyl)-methyl]-cyclohexylmethyl}-4-(2-methoxycarbonylethyl)-4,5-dihydro-1H-imidazole-4-carboxylic acid methyl ester
1-benzyl-4-benzylsulfanylmethyl-5-{4-[dimethylamino-(3-fluorophenyl)-methyl]-cyclohexylmethyl}-4,5-dihydro-1H-imidazole-4-carboxylic acid methyl ester
1-benzyl-5-{4-[dimethylamino-(4-fluorophenyl)-methyl]-cyclohexylmethyl}-4-(2-methoxycarbonylethyl)-4,5-dihydro-1H-imidazole-carboxylic acid methyl ester
1-butyl-4-sec-butyl-5-{4-[(4-chlorophenyl)-dimethylaminomethyl]-cyclohexylmethyl}-4,5-dihydro-1H-imidazole-4-carboxylic acid methyl ester
1-butyl-5-{4-[(4-chlorophenyl)-dimethylaminomethyl]-cyclohexylmethyl}-4-methylsulfanylmethyl-4,5-dihydro-1H-imidazole-4-carboxylic acid methyl ester
4-benzylsulfanylmethyl-1-butyl-5-{4-[(4-chlorophenyl)-dimethylaminomethyl]-cyclohexylmethyl}-4,5-dihydro-1H-imidazole-4-carboxylic acid methyl ester
5-{4-[dimethylamino-(3-fluorophenyl)-methyl]-cyclohexyl}-4-(2-methoxycarbonylethyl)-1-phenethyl-4,5-dihydro-1H-imidazole-4-carboxylic acid methyl ester
4-benzylsulfanylmethyl-5-{4-[dimethylamino-(3-fluorophenyl)-methyl]-cyclohexyl}-1-phenethyl-4,5-dihydro-1H-imidazole-4-carboxylic acid methyl ester
4-benzylsulfanylmethyl-5-{4-[(4-chlorophenyl)-dimethylaminomethyl]-cyclohexyl}-1-phenethyl-4,5-dihydro-1H-imidazole-4-carboxylic acid methyl ester
5-{4-[(4-chlorophenyl)-dimethylaminomethyl]-cyclohexylmethyl}-4-(2-methoxycarbonylethyl)-1-phenethyl-4,5-dihydro-1H-imidazole-4-carboxylic acid methyl ester
1-benzyl-5-{4-[dimethylamino-(3-fluorophenyl)-methyl]-cyclohexyl}-4-(2-methoxycarbonylethyl)-4,5-dihydro-1H-imidazole-4-carboxylic acid methyl ester
1-benzyl-5-{4-[dimethylamino-(3-fluorophenyl)-methyl]-cyclohexyl-4-methylsulfanylmethyl-4,5-dihydro-1H-imidazole-4-carboxylic acid methyl ester
1-benzyl-4-benzylsulfanylmethyl-5-{4-[dimethylamino-(3-fluorophenyl)-methyl]-cyclohexyl}-4,5-dihydro-1H-imidazole-4-carboxylic acid methyl ester
1-benzyl-4-benzylsulfanylmethyl-5-{4-[(4-chlorophenyl)-dimethylaminomethyl]-cyclohexyl}-4,5-dihydro-1H-imidazole-4-carboxylic acid methyl ester
1-benzyl-5-{4-[(4-chlorophenyl)-dimethylaminomethyl]-cyclohexylmethyl}-4-(2-methoxycarbonylethyl)-4,5-dihydro-1H-imidazole-4-carboxylic acid methyl ester
1-benzyl-5-{4-[(4-chlorophenyl)-dimethylaminomethyl]-cyclohexylmethyl}-4-methylsulfanylmethyl-4,5-dihydro-1H-imidazole-4-carboxylic acid methyl ester
1-benzyl-4-benzylsulfanylmethyl-5-{4-[(4-chlorophenyl)-dimethylaminomethyl]-cyclohexylmethyl}-4,5-dihydro-1H-imidazole-4-carboxylic acid methyl ester
1-cyclohexyl-5-{4-[dimethylamino-(3-fluorophenyl)-methyl]-cyclohexylmethyl}-4-propyl-4,5-dihydro-1H-imidazole-4-carboxylic acid methyl ester
1-cyclohexyl-5-{4-[dimethylamino-(3-fluorophenyl)-methyl]-cyclohexylmethyl}-4-(2-methylsulfanylethyl)-4,5-dihydro-1H-imidazole-4-carboxylic acid methyl ester
1-cyclohexyl-5-{4-[dimethylamino-(3-fluorophenyl)-methyl]-cyclohexylmethyl}-4-methyl-4,5-dihydro-1H-imidazole-4-carboxylic acid methyl ester
1-cyclohexyl-5-{4-[dimethylamino-(4-fluorophenyl)-methyl]-cyclohexylmethyl}-4-propyl-4,5-dihydro-1H-imidazole-4-carboxylic acid methyl ester
1-cyclohexyl-5-{4-[dimethylamino-(4-fluorophenyl)-methyl]-cyclohexylmethyl}-4-(2-methylsulfanylethyl)-4,5-dihydro-1H-imidazole-4-carboxylic acid methyl ester
1-cyclohexyl-5-{4-[dimethylamino-(4-fluorophenyl)-methyl]-cyclohexylmethyl}-4-methyl-4,5-dihydro-1H-imidazole-4-carboxylic acid methyl ester
1-cyclohexyl-5-[4-(1-dimethylamino-3-phenylpropyl)-cyclohexylmethyl]-4-propyl-4,5-dihydro-1H-imidazole-4-carboxylic acid methyl ester
1-cyclohexyl-5-[4-(1-dimethylamino-3-phenylpropyl)-cyclohexylmethyl]-4-(2-methylsulfanylethyl)-4,5-dihydro-1H-imidazole-4-carboxylic acid methyl ester
1-cyclohexyl-5-[4-(1-dimethylamino-3-phenylpropyl)-cyclohexylmethyl]-4-methyl-4,5-dihydro-1H-imidazole-4-carboxylic acid methyl ester
5-{4-[dimethylamino-(3-fluorophenyl)-methyl]-cyclohexylmethyl}-1,4-dipropyl-4,5-dihydro-1H-imidazole-4-carboxylic acid methyl ester
5-{4-[dimethylamino-(3-fluorophenyl)-methyl]-cyclohexylmethyl}-4-(2-methylsulfanylethyl)-1-propyl-4,5-dihydro-1H-imidazole-4-carboxylic acid methyl ester
1-benzyl-4-(4-chlorobenzyl)-5-{4-[(4-chlorophenyl)-dimethylaminomethyl]-cyclohexylmethyl}-4,5-dihydro-1H-imidazole-4-carboxylic acid methyl ester
1-benzyl-4-butyl-5-{4-[(chlorophenyl)-dimethylaminomethyl]-cyclohexylmethyl}-4,5-dihydro-1H-imidazole-4-carboxylic acid methyl ester
1-cyclohexyl-5-{4-[dimethylamino-(3-fluorophenyl)-methyl]-cyclohexyl}-4-(2-methoxycarbonylethyl)-4,5-dihydro-1H-imidazole-4-carboxylic acid methyl ester
4-benzylsulfanylmethyl-1-cyclohexyl-5-{4-[dimethylamino-(3-fluorophenyl)-methyl]-cyclohexyl}-4,5-dihydro-1H-imidazole-4-carboxylic acid methyl ester
5-{4-[dimethylamino-(3-fluorophenyl)-methyl]-cyclohexylmethyl}-4-methyl-1-propyl-4,5-dihydro-1H-imidazole-4-carboxylic acid methyl ester
5-{4-[dimethylamino-(4-fluorophenyl)-methyl]-cyclohexylmethyl}-1,4-dipropyl-4,5-dihydro-1H-imidazole-4-carboxylic acid methyl ester
5-{4-[dimethylamino-(4-fluorophenyl)-methyl]-cyclohexylmethyl}-4-(2-methylsulfanylethyl)-1-propyl-4,5-dihydro-1H-imidazole-4-carboxylic acid methyl ester
5-[4-(1-dimethylamino-3-phenylpropyl)-cyclohexylmethyl]-1,4-dipropyl-4,5-dihydro-1H-imidazole-4-carboxylic acid methyl ester
5-[4-(1-dimethylamino-3-phenylpropyl)-cyclohexylmethyl]-4-(2-methylsulfanylethyl)-1-propyl-4,5-dihydro-1H-imidazole-4-carboxylic acid methyl ester
1-butyl-5-{4-[dimethylamino-(3-fluorophenyl)-methyl]-cyclohexylmethyl}-4-propyl-4,5-dihydro-1H-imidazole-4-carboxylic acid methyl ester
1-butyl-5-{4-[dimethylamino-(3-fluorophenyl)-methyl]-cyclohexylmethyl}-4-(2-methylsulfanylethyl)-4,5-dihydro-1H-imidazole-4-carboxylic acid methyl ester
1-butyl-5-{4-[dimethylamino-(3-fluorophenyl)-methyl]-cyclohexylmethyl}-4-methyl-4,5-dihydro-1H-imidazole-4-carboxylic acid methyl ester
1-butyl-5-{4-[dimethylamino-(4-fluorophenyl)-methyl]-cyclohexylmethyl}-4-propyl-4,5- dihydro-1H-imidazole-4-carboxylic acid methyl ester
1-butyl-5-{4-[dimethylamino-(4-fluorophenyl)-methyl]-cyclohexylmethyl}-4-(2-methylsulfanylethyl)-4,5-dihydro-1H-imidazole-4-carboxylic acid methyl ester
1-butyl-5-[4-(1-dimethylamino-3-phenylpropyl)-cyclohexylmethyl]-4-propyl-4,5-dihydro-1H-imidazole-4-carboxylic acid methyl ester
1-butyl-5-[4-(1-dimethylamino-3-phenylpropyl)-cyclohexylmethyl]-4-(2-methylsulfanylethyl)-4,5-dihydro-1H-imidazole-4-carboxylic acid methyl ester
5-{4-[dimethylamino-(3-fluorophenyl)-methyl]-cyclohexylmethyl}-1-phenethyl-4-propyl-4,5-dihydro-1H-imidazole-4-carboxylic acid methyl ester
5-{4-[dimethylamino-(3-fluorophenyl)-methyl]-cyclohexylmethyl}-4-(2-methylsulfanylethyl)-1-phenethyl-4,5-dihydro-1H-imidazole-4-carboxylic acid methyl ester
5-{4-[dimethylamino-(3-fluorophenyl)-methyl]-cyclohexylmethyl}-4-methyl-1-phenethyl-4,5-dihydro-1H-imidazole-4-carboxylic acid methyl ester
5-{4-[dimethylamino-(4-fluorophenyl)-methyl]-cyclohexylmethyl}-1-phenethyl-4-propyl-4,5-dihydro-1H-imidazole-4-carboxylic acid methyl ester
5-{4-[dimethylamino-(4-fluorophenyl)-methyl]-cyclohexylmethyl}-4-(2-methylsulfanylethyl)-1-phenethyl-4,5-dihydro-1H-imidazole-4-carboxylic acid methyl ester
5-{4-[dimethylamino-(4-fluorophenyl)-methyl]-cyclohexylmethyl}-4-methyl-1-phenethyl-4,5-dihydro-1H-imidazole-4-carboxylic acid methyl ester
5-[4-(1-dimethylamino-3-phenylpropyl)-cyclohexylmethyl]-1-phenethyl-4-propyl-4,5-dihydro-1H-imidazole-4-carboxylic acid methyl ester
5-[4-(1-dimethylamino-3-phenylpropyl)-cyclohexylmethyl]-4-(2-methylsulfanylethyl)-1-phenethyl-4,5-dihydro-1H-imidazole-4-carboxylic acid methyl ester
5-[4-(1-dimethylamino-3-phenylpropyl)-cyclohexylmethyl]-4-methyl-1-phenethyl-4,5-dihydro-1H-imidazole-4-carboxylic acid methyl ester
1-benzyl-5-{4-[dimethylamino-(3-fluorophenyl)-methyl]-cyclohexylmethyl}-4-propyl-4,5-dihydro-1H-imidazole-4-carboxylic acid methyl ester
1-benzyl-5-{4-[dimethylamino-(3-fluorophenyl)-methyl]-cyclohexylmethyl}-4-(2-methylsulfanylethyl)-4,5-dihydro-1H-imidazole-4-carboxylic acid methyl ester
1-benzyl-5-{4-[dimethylamino-(3-fluorophenyl)-methyl]-cyclohexylmethyl}-4-methyl-4,5-dihydro-1H-imidazole-4-carboxylic acid methyl ester
1-benzyl-5-{4-[dimethylamino-(4-fluorophenyl)-methyl]-cyclohexylmethyl}-4-propyl-4,5-dihydro-1H-imidazole-4-carboxylic acid methyl ester
1-benzyl-5-{4-[dimethylamino-(4-fluorophenyl)-methyl]-cyclohexylmethyl}-4-(2-methylsulfanylethyl)-4,5-dihydro-1H-imidazole-4-carboxylic acid methyl ester
1-benzyl-5-{4-[dimethylamino-(4-fluorophenyl)-methyl]-cyclohexylmethyl}-4-methyl-4,5-dihydro-1H-imidazole-4-carboxylic acid methyl ester
1-benzyl-5-[4-(1-dimethylamino-3-phenylpropyl)-cyclohexylmethyl]-4-propyl-4,5-dihydro-1H-imidazole-4-carboxylic acid methyl ester
1-benzyl-5-[4-(1-dimethylamino-3-phenylpropyl)-cyclohexylmethyl]-4-(2-methylsulfanylethyl)-4,5-dihydro-1H-imidazole-4-carboxylic acid methyl ester
1-benzyl-5-[4-(1-dimethylamino-3-phenylpropyl)-cyclohexylmethyl]-4-methyl-4,5-dihydro-1H-imidazole-4-carboxylic acid methyl ester
1-cyclohexyl-5-{4-[dimethylamino-(3-fluorophenyl)-methyl]-cyclohexylmethyl}-4-(2-methoxycarbonylethyl)-4,5-dihydro-1H-imidazole-4-carboxylic acid methyl ester
4-sec-butyl-1-cyclohexyl-5-{4-[dimethylamino-(3-fluorophenyl)-methyl]-cyclohexylmethyl}-4,5-dihydro-1H-imidazole-4-carboxylic acid methyl ester
1-cyclohexyl-5-{4-[dimethylamino-(3-fluorophenyl)-methyl]-cyclohexylmethyl}-4-methylsulfanylmethyl-4,5-dihydro-1H-imidazole-4-carboxylic acid methyl ester
4-benzylsulfanylmethyl-1-cyclohexyl-5-{4-[dimethylamino-(3-fluorophenyl)-methyl]-cyclohexylmethyl}-4,5-dihydro-1H-imidazole-4-carboxylic acid methyl ester
1-cyclohexyl-5-{4-[dimethylamino-(4-fluorophenyl)-methyl]-cyclohexylmethyl}-4-(2-methoxycarbonylethyl)-4,5-dihydro-1H-imidazole-4-carboxylic acid methyl ester
4-sec-butyl-1-cyclohexyl-5-{4-[dimethylamino-(4-fluorophenyl)-methyl]-cyclohexylmethyl}-4,5-dihydro-1H-imidazole-4-carboxylic acid methyl ester
1-cyclohexyl-5-{4-[dimethylamino-(4-fluorophenyl)-methyl]-cyclohexylmethyl}-4-methylsulfanylmethyl-4,5-dihydro-1H-imidazole-4-carboxylic acid methyl ester
1-cyclohexyl-5-[4-(1-dimethylamino-3-phenylpropyl)-cyclohexylmethyl]-4-(2-methoxycarbonylethyl)-4,5-dihydro-1H-imidazole-4-carboxylic acid methyl ester
1-cyclohexyl-5-[4-(1-dimethylamino-3-phenylpropyl)-cyclohexylmethyl]-4-methylsulfanylmethyl-4,5-dihydro-1H-imidazole-4-carboxylic acid methyl ester
4-benzylsulfanylmethyl-1-cyclohexyl-5-[4-(1-dimethylamino-3-phenylpropyl)-cyclohexylmethyl]-4,5-dihydro-1H-imidazole-4-carboxylic acid methyl ester
5-{4-[dimethylamino-(3-fluorophenyl)-methyl]-cyclohexylmethyl}-4-(2-methoxycarbonylethyl)-1-propyl-4,5-dihydro-1H-imidazole-4-carboxylic acid methyl ester
5-{4-[dimethylamino-(3-fluorophenyl)-methyl]-cyclohexylmethyl}-4-methylsulfanylmethyl-1-propyl-4,5-dihydro-1H-imidazole-4-carboxylic acid methyl ester
5-{4-[dimethylamino-(4-fluorophenyl)-methyl]-cyclohexylmethyl}-4-(2-methoxycarbonylethyl)-1-propyl-4,5-dihydro-1H-imidazole-4-carboxylic acid methyl ester
5-{4-[dimethylamino-(4-fluorophenyl)-methyl]-cyclohexylmethyl}-4-methylsulfanylmethyl-1-propyl-4,5-dihydro-1H-imidazole-4-carboxylic acid methyl ester
4-benzylsulfanylmethyl-5-{4-[dimethylamino-(4-fluorophenyl)-methyl]-cyclohexylmethyl}-1-propyl-4,5-dihydro-1H-imidazole-4-carboxylic acid methyl ester
methyl-4-sec-butyl-5-((4-(4-chlorophenyl)(dimethylamino)methyl)cyclohexyl)methyl)-1-cyclohexyl-4,5-dihydro-1H-imidazole-4-carboxylate
in the form of the racemate; in the form of the enantiomers, diastereomers, mixtures of the enantiomers or diastereomers or in the form of an individual enantiomer or diastereomer; in the form of the bases and/or salts of physiologically compatible acids.

11. Process for the preparation of an imidazoline derivative according to the invention, wherein amines of the general Formula A are reacted with aldehydes of the general Formula B and isonitrile esters of the general Formula C in an organic solvent, for example ethanol or methanol, at a temperature of 20°-100°C according to the following synthesis reaction scheme, to form substituted imidazoline derivatives of the general Formula I

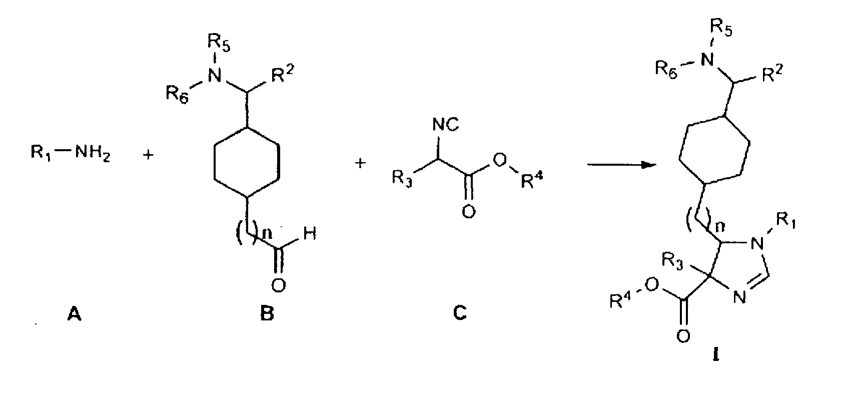

12. Substituted aldehyde of the general Formula B 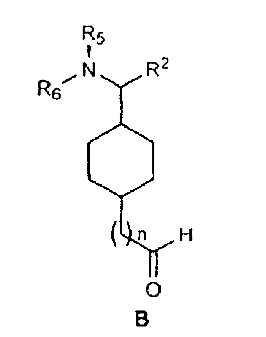 wherein the radicals R², R⁵, R⁶ as well as n have the meanings given in claims 1, 4, 5 and 9.

13. Substituted aldehydes according to claim 12, from the group
4-[dimethylamino-(4-fluorophenyl)-methyl]-cyclohexanecarbaldehyde
4-(1-dimethylamino-3-phenylpropyl)-cyclohexanecarbaldehyde
4-[dimethylamino-(3-fluorophenyl)-methyl]-cyclohexanecarbaldehyde
4-[(4-chlorophenyl)-dimethylaminomethyl]-cyclohexanecarbaldehyde
{4-[(4-chlorophenyl)-dimethylaminomethyl]-cyclohexyl}-acetaldehyde
{4-[dimethylamino-(3-fluorophenyl)-methyl]-cyclohexyl}-acetaldehyde
4-(dimethylamino-thiophen-2-yl-methyl)-cyclohexanecarbaldehyde
{4-[dimethylamino-(4-fluorophenyl)-methyl]-cyclohexyl}-acetaldehyde
[4-(dimethylamino-thiophen-2-yl-methyl)-cyclohexyl]-acetaldehyde
[4-(1-dimethylamino-3-phenylpropyl)-cyclohexyl]-acetaldehyde
in the form of the racemate; in the form of the enantiomers, diastereomers, mixtures of enantiomers or diastereomers or in the form of an individual enantiomer or diastereomer; in the form of the bases and/or salts of physiologically compatible acids.

14. Medicament containing at least one substituted imidazoline derivative according to claim 1 or a substituted aldehyde according to claim 12, optionally in the form of the racemate; in the form of the enantiomers, diastereomers, mixtures of the enantiomers or diastereomers or in the form of an individual enantiomer or diastereomer; in the form of the bases and/or salts of physiologically compatible acids and optionally containing suitable additives and/or auxiliary substances and/or optionally further active substances.

15. Use of a substituted imidazoline derivative according to claim 1 or a substituted aldehyde according to claim 12, optionally in the form of the racemate; in the form of the enantiomers, diastereomers, mixtures of the enantiomers or diastereomers or in the form of an individual enantiomer or diastereomer; in the form of the bases and/or salts of physiological compatible acids, for the preparation of a medicament for the treatment of pain, in particular acute, neuropathic or chronic pain.

16. Use of a substituted imidazoline derivative according to claim 1 or a substituted aldehyde according to claim 12, optionally in the form of the racemate; in the form of the enantiomers, diastereomers, mixtures of the enantiomers or diastereomers or in the form of an individual enantiomer or diastereomer; in the form of the bases and/or salts of physiological compatible acids, for the preparation of a medicament for the treatment of depression, urinary incontinence, diarrhea, pruritus, alcohol and drug misuse, drug dependency, loss of drive and/or anxiety.

## Revendications

1. Dérivés d'imidazoline substitués de formule générale I 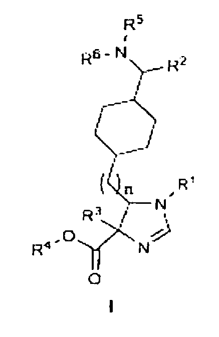 dans laquelle
n a la valeur 0, 1 ou 2
R¹ représente un reste alkyle en C₁ à C₈, saturé ou non saturé, ramifié ou non ramifié, non substitué ou substitué une ou plusieurs fois ; un reste cycloalkyle en C₃ à C₈ ou un reste cycloalkyle correspondant, dont un ou deux atomes de carbone sont remplacés par un hétéroatome S, N ou O, saturé ou non saturé, non substitué ou substitué une ou plusieurs fois ;
l'expression "substitué une ou plusieurs fois" désignant dans chaque cas le remplacement d'un ou plusieurs atomes d'hydrogène par F, Cl, Br, I, -CN, NH₂, NH-(alkyle en C₁ à C₆), NH-(alkyle en C₁ à C₆)-OH, N (alkyle en C1 à C₆)₂, N((alkyle en C₁ à C₆)-OH)₂, NO₂, SH, S-(alkyle en C₁ à C₆), S-benzyle, O-(alkyle en C₁ à C₆), OH, O-(alkyle en C₁ à C₆)-OH, =O, alkyle en C₁ à C₆, benzyle, O-benzyle, C(=O)(alkyle en C₁ à C₆), CO₂H, CO₂-(alkyle en C₁ à C₆) et la substitution multiple étant présente, avec des substituants identiques ou différents sur des atomes différents ou identiques ;
un reste aryle ou hétéroaryle lié par l'intermédiaire d'une chaîne alkyle en C₁ à C₃, chacun non substitué ou substitué une ou plusieurs fois,
l'expression "substitué une ou plusieurs fois" désignant dans chaque cas le remplacement d'un ou plusieurs atomes d'hydrogène du système cyclique par F, Cl, Br, I, -CN, NH₂, NH-(alkyle en C₁ à C₆), NH-(alkyle en C₁ à C₆)-OH, N(alkyle en C₁ à C₆)₂, N((alkyle en C₁ à C₆)-OH)₂, NO₂, SH, S-(alkyle en C₁ à C₆), OH, O-(alkyle en C₁ à C₆), O-(alkyle en C₁ à C₆)-OH, C(=O)(alkyle en C₁ à C₆), CO₂H, CO₂-(alkyle en C₁ à C₆), CF₃, alkyle en C₁ à C₆ et la substitution multiple étant présente, avec les mêmes substituants ou avec des substituants différents sur un ou divers atomes,
R² représente un reste aryle ou hétéroaryle, chacun non substitué ou substitué une ou plusieurs fois comme défini ci-dessus pour le reste aryle ou hétéroaryle ; un reste aryle lié par l'intermédiaire d'une chaîne alkyle en C₁ à C₃, dans chaque cas non substitué ou substitué une plusieurs fois comme défini ci-dessus pour le reste aryle,
R³ représente un reste alkyle en C₁ à C₈, saturé ou non saturé, ramifié ou non ramifié, non substitué ou substitué une ou plusieurs fois comme défini ci-dessus pour le reste alkyle en C₁ à C₈ ; un reste aryle lié par l'intermédiaire d'une chaîne alkyle en C₁ à C₃, dans chaque cas non substitué ou substitué une ou plusieurs fois comme défini ci-dessus pour le reste aryle,
R⁴ représente H, un reste alkyle en C₁ à C₄ ou un reste aryle lié par l'intermédiaire d'une chaîne alkyle en C₁ à C₃,
R⁵ et R⁶ représentent indépendamment l'un de l'autre H ; un reste alkyle en C₁ à C₆, dans chaque cas saturé ou non saturé, ramifié ou non ramifié, R⁵ et R⁶ ne pouvant pas représenter en même temps H,
ou bien les restes R⁵ et R⁶ forment ensemble un groupe CH₂CH₂OCH₂CH₂ ou (CH₂)₃₋₆,
sous forme du racémate ; des énantiomères, des diastéréoisomères, de mélanges des énantiomères ou des diastéréoisomères ou sous forme d'un énantiomère individuel ou d'un diastéréoisomère individuel ; des bases et/ou des sels d'acides physiologiquement acceptables.

2. Dérivés d'imidazoline substitués suivant la revendication 1, dans lesquels R¹ représente un reste alkyle en C₁ à C₈, ramifié ou non ramifié, non substitué ou substitué une ou plusieurs fois avec F, Cl, -CN, SH, S-(alkyle en C₁ à C₆), S-benzyle, OH, O-benzyle, O-(alkyle en C₁ à C₆), CO₂H, CO₂-(alkyle en C₁ à C₆) ; un reste cycloalkyle en C₃ à C₈, dans chaque cas non substitué ou substitué une ou plusieurs fois avec F, Cl, -CN, SH, S-(alkyle en C₁ à C₆), S-benzyle, OH, O-benzyle, O-(alkyle en C₁ à C₆), CO₂H, CO₂-(alkyle en C₁ à C₆) ; un reste phényle lié par l'intermédiaire d'une chaîne alkyle en C₁ à C₃, dans chaque cas non substitué ou substitué une ou plusieurs fois avec F, Cl, CN, NO₂, SH, S-(alkyle en C₁ à C₆), OH, O-(alkyle en C₁ à C₆), CO₂H, CO₂-(alkyle en C₁ à C₆), CF₃, alkyle en C₁ à C₆.

3. Dérivés d'imidazoline substitués suivant la revendication 1, dans lesquels R¹ représente un reste cyclohexyle, n-propyle, n-butyle, phénéthyle ou benzyle.

4. Dérivés d'imidazoline substitués suivant l'une des revendications 1 à 3, dans lesquels R² représente un reste phényle ou thiényle, chacun non substitué ou substitué une ou plusieurs fois avec F, Cl, CN, NO₂, SH, S-(alkyle en C₁ à C₆), OH, O-(alkyle en C₁ à C₆), CO₂H, CO₂-(alkyle en C₁ à C₆), CF₃, alkyle en C₁ à C₆ ; un reste phényle lié par l'intermédiaire d'une chaîne alkyle en C₁ à C₃, dans chaque cas non substitué ou substitué une ou plusieurs fois avec F, Cl, CN, NO₂, SH, S-(alkyle en C₁ à C₆), OH, O-(alkyle en C₁ à C₆), CO₂H, CO₂-(alkyle en C₁ à C₆), CF₃, alkyle en C₁ à C₆.

5. Dérivés d'imidazoline substitués suivant l'une des revendications 1 à 3, dans lesquels R² représente un reste phényle, non substitué ou substitué une fois avec Cl ou F, un reste thiényle ou phénéthyle.

6. Dérivés d'imidazoline substitués suivant l'une des revendications 1 à 5, dans lesquels R³ représente un reste alkyle en C₁ à C₈, ramifié ou non ramifié, non substitué ou substitué une ou plusieurs fois avec F, Cl, -CN, SH, S-(alkyle en C₁ à C₆), S-benzyle, OH, O-benzyle, O-(alkyle en C₁ à C₆), CO₂H, CO₂-(alkyle en C₁ à C₆) ; un reste phényle lié par l'intermédiaire d'une chaîne alkyle en C₁ à C₃, dans chaque cas non substitué ou substitué une ou plusieurs fois avec F, Cl, CN, NO₂, SH, S-(alkyle en C₁ à C₆), OH, O-(alkyle en C₁ à C₆), CO₂H, CO₂-(alkyle en C₁ à C₆), CF₃, alkyle en C₁ à C₆.

7. Dérivés d'imidazoline substitués suivant l'une des revendications 1 à 5, dans lesquels R³ représente un reste sec.-butyle, isobutyle, n-butyle, n-propyle, CH₃, CH₂CH₂COOCH₃, CH₂-S-benzyle, CH₂CH₂-S-CH₃, CH₂-S-CH₃ ou 4-Cl-benzyle.

8. Dérivés d'imidazoline substitués suivant l'une des revendications 1 à 7, dans lesquels R⁴ représente un reste CH₃.

9. Dérivés d'imidazoline substitués suivant l'une des revendications 1 à 8, dans lesquels R⁵ et R⁶ représentent un reste CH₃.

10. Dérivés d'imidazoline substitués suivant l'une des revendications 1 à 9, choisis dans le groupe consistant en :
l'ester méthylique d'acide 1-cyclohexyl-5-{4-[diméthyl-amino-(3-fluorophényl)-méthyl]-cyclohexyl}-4-isobutyl-4,5-dihydro-1H-imidazole-4-carboxylique,
l'ester méthylique d'acide 4-(4-chlorobenzyl)-1-cyclohexyl-5-{4-[diméthylamino-(3-fluorophényl)-méthyl]-cyclohexyl}-4,5-dihydro-1H-imidazole-4-carboxylique,
l'ester méthylique d'acide 4-butyl-1-cyclohexyl-5-{4-[diméthylamino-(3-fluorophényl)-méthyl]-cyclohexyl}-4,5-dihydro-1H-imidazole-4-carboxylique,
l'ester méthylique d'acide 5-{4-[(4-chlorophényl)-diméthylamino-méthyl]-cyclohexyl}-1-cyclohexyl-4-isobutyl-4,5-dihydro-1H-imidazole-4-carboxylique,
l'ester méthylique d'acide 4-(4-chlorobenzyl)-5-{4-[(4-chlorophényl)-diméthylamino-méthyl]-cyclohexyl}-1-cyclohexyl-4,5-dihydro-1H-imidazole-4-carboxylique,
l'ester méthylique d'acide 4-butyl-5-{4-[(4-chlorophényl)-diméthylamino-méthyl]-cyclohexyl}-1-cyclohexyl-4,5-dihydro-1H-imidazole-4-carboxylique,
l'ester méthylique d'acide 5-{4-[(4-chlorophényl)-diméthylamino-méthyl]-cyclohexylméthyl}-1-cyclohexyl-4-isobutyl-4,5-dihydro-1H-imidazole-4-carboxylique,
l'ester méthylique d'acide 4-(4-chlorobenzyl)-5-{4-[(4-chlorophényl)-diméthylamino-méthyl]-cyclohexylméthyl}-1-cyclohexyl-4,5-dihydro-1H-imidazole-4-carboxylique,
l'ester méthylique d'acide 4-butyl-5-{4-[(4-chlorophényl)-diméthylamino-méthyl]-cyclohexylméthyl}-1-cyclohexyl-4,5-dihydro-1H-imidazole-4-carboxylique,
l'ester méthylique d'acide 4-(4-chlorobenzyl)-1-cyclohexyl-5-[4-(diméthylamino-thiophène-2-yl-méthyl)-cyclohexylméthyl]-4,5-dihydro-1H-imidazole-4-carboxylique,
l'ester méthylique d'acide 5-{4-[diméthylamino-(3-fluorophényl)-méthyl]-cyclohexyl}-4-isobutyl-1-propyl-4,5-dihydro-1H-imidazole-4-carboxylique,
l'ester méthylique d'acide 4-(4-chlorobenzyl)-5-{4-[diméthylamino-(3-fluorophényl)-méthyl]-cyclohexyl}-1-propyl-4,5-dihydro-1H-imidazole-4-carboxylique,
l'ester méthylique d'acide 4-butyl-5-{4-[4-diméthylamino-(3-fluorophényl)-méthyl]-cyclohexyl}-1-propyl-4,5-dihydro-1H-imidazole-4-carboxylique,
l'ester méthylique d'acide 5-{4-[(4-chlorophényl)-diméthylamino-méthyl]-cyclohexyl}-4-isobutyl-1-propyl-4,5-dihydro-1H-imidazole-4-carboxylique,
l'ester méthylique d'acide 4-(4-chlorobenzyl)-5-{4-[(4-chlorophényl)-diméthylamino-méthyl]-cyclohexyl}-1-propyl-4,5-dihydro-1H-imidazole-4-carboxylique,
l'ester méthylique d'acide 5-{4-[(4-chlorophényl)-diméthylamino-méthyl]-cyclohexylméthyl}-4-isobutyl-1-propyl-4,5-dihydro-1H-imidazole-4-carboxylique,
l'ester méthylique d'acide 4-(4-chlorobenzyl)-5-{4-[(4-chlorophényl)-diméthylamino-méthyl]-cyclohexylméthyl}-1-propyl-4,5-dihydro-1H-imidazole-4-carboxylique,
l'ester méthylique d'acide 4-butyl-5-{4-[(4-chlorophényl)-diméthylamino-méthyl]-cyclohexylméthyl}-1-propyl-4,5-dihydro-1H-imidazole-4-carboxylique,
l'ester méthylique d'acide 1-butyl-5-{4-[diméthylamino-(3-fluorophényl)-méthyl]-cyclohexyl}-4-isobutyl-4,5-dihydro-1H-imidazole-4-carboxylique,
l'ester méthylique d'acide 1-butyl-4-(4-chlorobenzyl)-5-{4-[diméthylamino-(3-fluorophényl)-méthyl]-cyclohexyl}-4,5-dihydro-1H-imidazole-4-carboxylique,
l'ester méthylique d'acide 1-butyl-5-{4-[(4-chlorophényl)-diméthylamino-méthyl]-cyclohexyl}-4-isobutyl-4,5-dihydro-1H-imidazole-4-carboxylique,
l'ester méthylique d'acide 1-butyl-4-(4-chlorobenzyl)-5-{4-[(4-chlorophényl)-diméthylamino-méthyl]-cyclohexyl}-4,5-dihydro-1H-imidazole-4-carboxylique,
l'ester méthylique d'acide 1,4-dibutyl-5-{4-[(4-chlorophényl)-diméthylamino-méthyl]-cyclohexyl}-4,5-dihydro-1H-imidazole-4-carboxylique,
l'ester méthylique d'acide 1-butyl-5-{4-[(4-chlorophényl)-diméthylamino-méthyl]-cyclohexylméthyl}-4-isobutyl-4,5-dihydro-1H-imidazole-4-carboxylique,
l'ester méthylique d'acide 1-butyl-4-(4-chlorobenzyl)-5-{4-[(4-chlorophényl)-diméthylamino-méthyl]-cyclohexylméthyl}-4,5-dihydro-1H-imidazole-4-carboxylique,
l'ester méthylique d'acide 1,4-dibutyl-5-{4-[(4-chlorophényl)-diméthylamino-méthyl]-cyclohexylméthyl}-4,5-dihydro-1H-imidazole-4-carboxylique,
l'ester méthylique d'acide 5-{4-[diméthylamino-(3-fluorophényl)-méthyl]-cyclohexyl}-4-isobutyl-1-phénéthyl-4,5-dihydro-1H-imidazole-4-carboxylique,
l'ester méthylique d'acide 4-(4-chlorobenzyl)-5-{4-[diméthylamino-(3-fluorophényl)-méthyl]-cyclohexyl}-1-phénéthyl-4,5-dihydro-1H-imidazole-4-carboxylique,
l'ester méthylique d'acide 4-butyl-5-{4-[diméthylamino-(3-fluorophényl)-méthyl]-cyclohexyl}-1-phénéthyl-4,5-dihydro-1H-imidazole-4-carboxylique,
l'ester méthylique d'acide 5-{4-[(4-chlorophényl)-diméthylamino-méthyl]-cyclohexyl}-4-isobutyl-1-phénéthyl-4,5-dihydro-1H-imidazole-4-carboxylique,
l'ester méthylique d'acide 4-(4-chlorobenzyl)-5-{4-[(4-chlorophényl)-diméthylamino-méthyl]-cyclohexyl}-1-phénéthyl-4,5-dihydro-1H-imidazole-4-carboxylique,
l'ester méthylique d'acide 4-butyl-5-{4-[(4-chlorophényl)-diméthylamino-méthyl]-cyclohexyl}-1-phénéthyl-4,5-dihydro-1H-imidazole-4-carboxylique,
l'ester méthylique d'acide 5-{4-[(4-chlorophényl)-diméthylamino-méthyl]-cyclohexylméthyl}-4-isobutyl-1-phénéthyl-4,5-dihydro-1H-imidazole-4-carboxylique,
l'ester méthylique d'acide 4-(4-chlorobenzyl)-5-{4-[(4-chlorophényl)-diméthylamino-méthyl]-cyclohexylméthyl}-1-phénéthyl-4,5-dihydro-1H-imidazole-4-carboxylique,
l'ester méthylique d'acide 4-butyl-5-{4-[(4-chlorophényl)-diméthylamino-méthyl]-cyclohexylméthyl}-1-phénéthyl-4,5-dihydro-1H-imidazole-4-carboxylique,
l'ester méthylique d'acide 1-benzyl-5-{4-[diméthylamino-(3-fluorophényl)-méthyl]-cyclohexyl}-4-isobutyl-4,5-dihydro-1H-imidazole-4-carboxylique,
l'ester méthylique d'acide 1-benzyl-4-(4-chlorobenzyl)-5-{4-[diméthylamino-(3-fluorophényl)-méthyl]-cyclohexyl}-4,5-dihydro-1H-imidazole-4-carboxylique,
l'ester méthylique d'acide 1-benzyl-5-{4-[(4-chlorophényl)-diméthylamino-méthyl]-cyclohexyl}-4-isobutyl-4,5-dihydro-1H-imidazole-4-carboxylique,
l'ester méthylique d'acide 1-benzyl-4-(4-chlorobenzyl)-5-{4-[(4-chlorophényl)-diméthylamino-méthyl]-cyclohexyl}-4,5-dihydro-1H-imidazole-4-carboxylique,
l'ester méthylique d'acide 1-benzyl-5-{4-[(4-chlorophényl)-diméthylamino-méthyl]-cyclohexylméthyl}-4-isobutyl-4,5-dihydro-1H-imidazole-4-carboxylique,
l'ester méthylique d'acide 5-{4-[(4-chlorophényl)-diméthylamino-méthyl]-cyclohexyl}-1-cyclohexyl-4-(2-méthoxy-carbonyl-éthyl)-4,5-dihydro-1H-imidazole-4-carboxylique,
l'ester méthylique d'acide 4-benzylsulfanylméthyl-5-{4-[(4-chlorophényl)-diméthylamino-méthyl]-cyclohexyl}-1-cyclohexyl-4,5-dihydro-1H-imidazole-4-carboxylique,
l'ester méthylique d'acide 5-{4-[(4-chlorophényl)-diméthylamino-méthyl]-cyclohexylméthyl}-1-cyclohexyl-4-(2-méthoxycarbonyl-éthyl)-4,5-dihydro-1H-imidazole-4-carboxylique,
l'ester méthylique d'acide 1-benzyl-5-{4-[diméthylamino-(3-fluorophényl)-méthyl]-cyclohexylméthyl}-4-propyl-4,5-dihydro-1H-imidazole-4-carboxylique,
l'ester méthylique d'acide 5-{4-[(4-chlorophényl)-diméthylamino-méthyl]-cyclohexylméthyl}-1-cyclohexyl-4-méthyl-sulfanylméthyl-4,5-dihydro-1H-imidazole-4-carboxylique,
l'ester méthylique d'acide 4-benzylsulfanylméthyl-5-{4-[(4-chlorophényl)-diméthylamino-méthyl]-cyclohexylméthyl}-1-cyclohexyl-4,5-dihydro-1H-imidazole-4-carboxylique,
l'ester méthylique d'acide 5-{4-[diméthylamino-(3-fluorophényl)-méthyl]-cyclohexyl}-4-méthylsulfanylméthyl-1-propyl-4,5-dihydro-1H-imidazole-4-carboxylique,
l'ester méthylique d'acide 4-benzylsulfanylméthyl-5-{4-[diméthylamino-(3-fluorophényl)-méthyl]-cyclohexyl}-1-propyl-4,5-dihydro-1H-imidazole-4-carboxylique,
l'ester méthylique d'acide 5-{4-[(4-chlorophényl)-diméthylamino-méthyl]-cyclohexyl}-4-(2-méthoxycarbonyl-éthyl)-1-propyl-4,5-dihydro-1H-imidazole-4-carboxylique,
l'ester méthylique d'acide 4-benzylsulfanylméthyl-5-{4-[(4-chlorophényl)-diméthylamino-méthyl]-cyclohexyl}-1-propyl-4,5-dihydro-1H-imidazole-4-carboxylique,
l'ester méthylique d'acide 5-{4-[(4-chlorophényl)-diméthylamino-méthyl]-cyclohexylméthyl}-4-(2-méthoxycarbonyl-éthyl)-1-propyl-4,5-dihydro-1H-imidazole-4-carboxylique,
l'ester méthylique d'acide 4-sec.-butyl-5-{4-[(4-chloro-phényl)-diméthylamino-méthyl]-cyclohexylméthyl}-1-propyl-4,5-dihydro-1H-imidazole-4-carboxylique,
l'ester méthylique d'acide 5-{4-[(4-chlorophényl)-diméthylamino-méthyl]-cyclohexylméthyl}-4-méthylsulfanylméthyl-1-propyl-4,5-dihydro-1H-imidazole-4-carboxylique,
l'ester méthylique d'acide 4-benzylsulfanylméthyl-5-{4-[(4-chlorophényl)-diméthylamino-méthyl]-cyclohexylméthyl}-1-propyl-4,5-dihydro-1H-imidazole-4-carboxylique,
l'ester méthylique d'acide 1-butyl-5-{4-[diméthylamino-(3-fluorophényl)-méthyl]-cyclohexyl}-4-(2-méthoxycarbonyl-éthyl)-4,5-dihydro-1H-imidazole-4-carboxylique,
l'ester méthylique d'acide 1-butyl-5-{4-[diméthylamino-(3-fluorophényl)-méthyl]-cyclohexyl}-4-méthylsulfanylméthyl-4,5-dihydro-1H-imidazole-4-carboxylique,
l'ester méthylique d'acide 4-benzylsulfanylméthyl-1-butyl-5-{4-[diméthylamino-(3-fluorophényl)-méthyl]-cyclohexyl}-4,5-dihydro-1H-imidazole-4-carboxylique,
l'ester méthylique d'acide 1-butyl-5-{4-[(4-chlorophényl)-diméthylamino-méthyl]-cyclohexyl}-4-(2-méthoxycarbonyl-éthyl)-4,5-dihydro-1H-imidazole-4-carboxylique,
l'ester méthylique d'acide 4-benzylsulfanylméthyl-1-butyl-5-{4-[(4-chlorophényl)-diméthylamino-méthyl]-cyclohexyl}-4,5-dihydro-1H-imidazole-4-carboxylique,
l'ester méthylique d'acide 1-butyl-5-{4-[(4-chlorophényl)-diméthylamino-méthyl]-cyclohexylméthyl}-4-(2-méthoxycarbonyl-éthyl)-4,5-dihydro-1H-imidazole-4-carboxylique,
l'ester méthylique d'acide 5-[4-(1-diméthylamino-3-phényl-propyl)-cyclohexylméthyl]-4-(2-méthoxycarbonyl-éthyl]-1-propyl-4,5-dihydro-1H-imidazole-4-carboxylique,
l'ester méthylique d'acide 1-butyl-5-{4-[diméthylamino-(3-fluorophényl)-méthyl]-cyclohexylméthyl}-4-(2-méthoxycarbonyl-éthyl)-4,5-dihydro-1H-imidazole-4-carboxylique,
l'ester méthylique d'acide 1-butyl-4-sec.-butyl-5-{4-[diméthylamino-(3-fluorophényl)-méthyl]-cyclohexylméthyl}-4,5-dihydro-1H-imidazole-4-carboxylique,
l'ester méthylique d'acide 1-butyl-5-{4-[diméthylamino-(3-fluorophényl)-méthyl]-cyclohexylméthyl}-4-méthylsulfanyl-méthyl-4,5-dihydro-1H-imidazole-4-carboxylique,
l'ester méthylique d'acide 4-benzylsulfanylméthyl-1-butyl-5-{4-[diméthylamino-(3-fluorophényl)-méthyl]-cyclohexylméthyl}-4,5-dihydro-1H-imidazole-4-carboxylique,
l'ester méthylique d'acide 1-butyl-5-{4-[diméthylamino-(4-fluorophényl)-méthyl]-cyclohexylméthyl}-4-(2-méthoxycarbonyl-éthyl)-4,5-dihydro-1H-imidazole-4-carboxylique,
l'ester méthylique d'acide 1-butyl-5-{4-[diméthylamino-(4-fluorophényl)-méthyl]-cyclohexylméthyl}-4-méthylsulfanylméthyl-4,5-dihydro-1H-imidazole-4-carboxylique,
l'ester méthylique d'acide 1-butyl-5-[4-(1-diméthylamino-3-phénylpropyl)-cyclohexylméthyl]-4-(2-méthoxycarbonyl-éthyl)-4,5-dihydro-1H-imidazole-4-carboxylique,
l'ester méthylique d'acide 4-benzylsulfanylméthyl-1-butyl-5-[4-(1-diméthylamino-3-phénylpropyl)-cyclohexylméthyl]-4,5-dihydro-1H-imidazole-4-carboxylique,
l'ester méthylique d'acide 5-{4-[diméthylamino-(3-fluorophényl)-méthyl]-cyclohexylméthyl}-4-(2-méthoxycarbonyl-éthyl)-1-phénéthyl-4,5-dihydro-1H-imidazole-4-carboxylique,
l'ester méthylique d'acide 4-sec.-butyl-5-{4-[diméthylamino-(3-fluorophényl)-méthyl]-cyclohexylméthyl}-1-phénéthyl-4,5-dihydro-1H-imidazole-4-carboxylique,
l'ester méthylique d'acide 4-benzylsulfanylméthyl-5-{4-[diméthylamino-(3-fluorophényl)-méthyl]-cyclohexylméthyl}-1-phénéthyl-4,5-dihydro-1H-imidazole-4-carboxylique,
l'ester méthylique d'acide 5-{4-[diméthylamino-(4-fluorophényl)-méthyl]-cyclohexylméthyl}-4-(2-méthoxycarbonyl-éthyl)-1-phénéthyl-4,5-dihydro-1H-imidazole-4-carboxylique,
l'ester méthylique d'acide 5-[4-(1-diméthylamino-3-phénylpropyl)-cyclohexylméthyl]-4-(2-méthoxycarbonyl-éthyl)-1-phénéthyl-4,5-dihydro-1H-imidazole-4-carboxylique,
l'ester méthylique d'acide 1-benzyl-5-{4-[diméthylamino-(3-fluorophényl)-méthyl]-cyclohexylméthyl}-4-(2-méthoxycarbonyl-éthyl)-4,5-dihydro-1H-imidazole-4-carboxylique,
l'ester méthylique d'acide 1-benzyl-4-benzylsulfanylméthyl-5-{4-[diméthylamino-(3-fluorophényl)-méthyl]-cyclohexylméthyl}-4,5-dihydro-1H-imidazole-4-carboxylique,
l'ester méthylique d'acide 1-benzyl-5-{4-[diméthylamino-(4-fluorophényl)-méthyl]-cyclohexylméthyl}-4-(2-méthoxycarbonyl-éthyl)-4,5-dihydro-1H-imidazole-4-carboxylique,
l'ester méthylique d'acide 1-butyl-4-sec.-butyl-5-{4-[(4-chlorophényl)-diméthylamino-méthyl]-cyclohexylméthyl}-4,5-dihydro-1H-imidazole-4-carboxylique,
l'ester méthylique d'acide 1-butyl-5-{4-[(4-chlorophényl)-diméthylamino-méthyl]-cyclohexylméthyl}-4-méthylsulfanylméthyl-4,5-dihydro-1H-imidazole-4-carboxylique,
l'ester méthylique d'acide 4-benzylsulfanylméthyl-1-butyl-5-{4-[(4-chlorophényl)-diméthylamino-méthyl]-cyclohexylméthyl}-4,5-dihydro-1H-imidazole-4-carboxylique,
l'ester méthylique d'acide 5-{4-[diméthylamino-(3-fluorophényl)-méthyl]-cyclohexyl}-4-(2-méthoxycarbonyl-éthyl)-1-phénéthyl-4,5-dihydro-1H-imidazole-4-carboxylique,
l'ester méthylique d'acide 4-benzylsulfanylméthyl-5-{4-[diméthylamino-(3-fluorophényl)-méthyl]-cyclohexyl}-1-phénéthyl-4,5-dihydro-1H-imidazole-4-carboxylique,
l'ester méthylique d'acide 4-benzylsulfanylméthyl-5-{4-[(4-chlorophényl)-diméthylamino-méthyl]-cyclohexyl}-1-phénéthyl-4,5-dihydro-1H-imidazole-4-carboxylique,
l'ester méthylique d'acide 5-{4-[(4-chlorophényl)-diméthylamino-méthyl]-cyclohexylméthyl}-4-(2-méthoxycarbonyl-éthyl)-1-phénéthyl-4,5-dihydro-1H-imidazole-4-carboxylique,
l'ester méthylique d'acide 1-benzyl-5-{4-[diméthylamino-(3-fluorophényl)-méthyl]-cyclohexyl}-4-(2-méthoxycarbonyl-éthyl)-4,5-dihydro-1H-imidazole-4-carboxylique,
l'ester méthylique d'acide 1-benzyl-5-{4-[diméthylamino-(3-fluorophényl)-méthyl]-cyclohexyl}-4-méthylsulfanylméthyl-4,5-dihydro-1H-imidazole-4-carboxylique,
l'ester méthylique d'acide 1-benzyl-4-benzylsulfanylméthyl-5-{4-[diméthylamino-(3-fluorophényl)-méthyl]-cyclohexyl}-4,5-dihydro-1H-imidazole-4-carboxylique,
l'ester méthylique d'acide 1-benzyl-4-benzylsulfanylméthyl-5-{4-[(4-chlorophényl)-diméthylamino-méthyl]-cyclohexyl}-4,5-dihydro-1H-imidazole-4-carboxylique,
l'ester méthylique d'acide 1-benzyl-5-{4-[(4-chlorophényl)-diméthylamino-méthyl]-cyclohexylméthyl}-4-(2-méthoxycarbonyl-éthyl)-4,5-dihydro-1H-imidazole-4-carboxylique,
l'ester méthylique d'acide 1-benzyl-5-{4-[(4-chlorophényl)-diméthylamino-méthyl]-cyclohexylméthyl}-4-méthylsulfanylméthyl-4,5-dihydro-1H-imidazole-4-carboxylique,
l'ester méthylique d'acide 1-benzyl-4-benzylsulfanylméthyl-5-{4-[(4-chlorophényl)-diméthylaminométhyl]-cyclohexylméthyl}-4,5-dihydro-1H-imidazole-4-carboxylique,
l'ester méthylique d'acide 1-cyclohexyl-5-{4-[diméthylamino-(3-fluorophényl)-méthyl]-cyclohexylméthyl}-4-propyl-4,5-dihydro-1H-imidazole-4-carboxylique,
l'ester méthylique d'acide 1-cyclohexyl-5-{4-[diméthylamino-(3-fluorophényl)-méthyl]-cyclohexylméthyl}-4-(2-méthylsulfanyl-éthyl)-4,5-dihydro-1H-imidazole-4-carboxylique,
l'ester méthylique d'acide 1-cyclohexyl-5-{4-[diméthylamino-(3-fluorophényl)-méthyl]-cyclohexylméthyl}-4-méthyl-4,5-dihydro-1H-imidazole-4-carboxylique,
l'ester méthylique d'acide 1-cyclohexyl-5-{4-[diméthylamino-(4-fluorophényl)-méthyl]-cyclohexylméthyl}-4-propyl-4,5-dihydro-1H-imidazole-4-carboxylique,
l'ester méthylique d'acide 1-cyclohexyl-5-{4-[diméthylamino-(4-fluorophényl)-méthyl]-cyclohexylméthyl}-4-(2-méthylsulfanyl-éthyl)-4,5-dihydro-1H-imidazole-4-carboxylique,
l'ester méthylique d'acide 1-cyclohexyl-5-{4-[diméthylamino-(4-fluorophényl)-méthyl]-cyclohexylméthyl}-4-méthyl-4,5-dihydro-1H-imidazole-4-carboxylique,
l'ester méthylique d'acide 1-cyclohexyl-5-[4-(1-diméthylamino-3-phényl-propyl)-cyclohexylméthyl]-4-propyl-4,5-dihydro-1H-imidazole-4-carboxylique,
l'ester méthylique d'acide 1-cyclohexyl-5-[4-(1-diméthyl-amino-3-phényl-propyl)-cyclohexylméthyl]-4-(2-méthylsulfanyl-éthyl)-4,5-dihydro-1H-imidazole-4-carboxylique,
l'ester méthylique d'acide 1-cyclohexyl-5-[4-(1-diméthyl-amino-3-phényl-propyl)-cyclohexylméthyl]-4-méthyl-4,5-dihydro-1H-imidazole-4-carboxylique,
l'ester méthylique d'acide 5-{4-[diméthylamino-(3-fluorophényl)-méthyl]-cyclohexylméthyl}-1-4-dipropyl-4,5-dihydro-1H-imidazole-4-carboxylique,
l'ester méthylique d'acide 5-{4-[diméthylamino-(3-fluorophényl)-méthyl]-cyclohexylméthyl}-4-(2-méthylsulfanyl-éthyl)-1-propyl-4,5-dihydro-1H-imidazole-4-carboxylique,
l'ester méthylique d'acide 1-benzyl-4-(4-chlorobenzyl)-5-{4-[(4-chlorophényl)-diméthylamino-méthyl]-cyclohexylméthyl}-4,5-dihydro-1H-imidazole-4-carboxylique,
l'ester méthylique d'acide 1-benzyl-4-butyl-5-{4-[(4-chlorophényl)diméthylamino-méthyl]-cyclohexylméthyl}-4,5-dihydro-1H-imidazole-4-carboxylique,
l'ester méthylique d'acide 1-cyclohexyl-5-{4-[diméthyl-amino-(3-fluorophényl)-méthyl]-cyclohexyl}-4-(2-méthoxycarbonyl-éthyl)-4,5-dihydro-1H-imidazole-4-carboxylique,
l'ester méthylique d'acide 4-benzylsulfanylméthyl-1-cyclohexyl-5-{4-[diméthylamino-(3-fluorophényl)-méthyl]-cyclohexyl}-4,5-dihydro-1H-imidazole-4-carboxylique,
l'ester méthylique d'acide 5-{4-[diméthylamino-(3-fluorophényl)-méthyl]-cyclohexylméthyl}-4-méthyl-1-propyl-4,5-dihydro-1H-imidazole-4-carboxylique,
l'ester méthylique d'acide 5-{4-[diméthylamino-(4-fluorophényl)-méthyl]-cyclohexylméthyl}-1,4-dipropyl-4,5-dihydro-1H-imidazole-4-carboxylique,
l'ester méthylique d'acide 5-{4-[diméthylamino-(4-fluorophényl)-méthyl]-cyclohexylméthyl}-4-(2-méthylsulfanyl-éthyl)-1-propyl-4,5-dihydro-1H-imidazole-4-carboxylique,
l'ester méthylique d'acide 5-[4-(1-diméthylamino-3-phényl-propyl)-cyclohexylméthyl]-1,4-dipropyl-4,5-dihydro-1H-imidazole-4-carboxylique,
l'ester méthylique d'acide 5-[4-(1-diméthylamino-3-phényl-propyl)-cyclohexylméthyl]-4-(2-méthylsulfanyl-éthyl)-1-propyl-4,5-dihydro-1H-imidazole-4-carboxylique,
l'ester méthylique d'acide 1-butyl-5-{4-[diméthylamino-(3-fluorophényl)-méthyl]-cyclohexylméthyl}-4-propyl-4,5-dihydro-1H-imidazole-4-carboxylique,
l'ester méthylique d'acide 1-butyl-5-{4-[diméthylamino-(3-fluorophényl)-méthyl]-cyclohexylméthyl}-4-(2-méthylsulfanyl-éthyl)-4,5-dihydro-1H-imidazole-4-carboxylique,
l'ester méthylique d'acide 1-butyl-5-{4-[diméthylamino-(3-fluorophényl)-méthyl]-cyclohexylméthyl}-4-méthyl-4,5-dihydro-1H-imidazole-4-carboxylique,
l'ester méthylique d'acide 1-butyl-5-{4-[diméthylamino-(4-fluorophényl)-méthyl]-cyclohexylméthyl}-4-propyl-4,5-dihydro-1H-imidazole-4-carboxylique,
l'ester méthylique d'acide 1-butyl-5-{4-[diméthylamino-(4-fluorophényl)-méthyl]-cyclohexylméthyl}-4-(2-méthylsulfanyl-éthyl)-4,5-dihydro-1H-imidazole-4-carboxylique,
l'ester méthylique d'acide 1-butyl-5-[4-(1-diméthylamino-3-phényl-propyl)-cyclohexylméthyl]-4-propyl-4,5-dihydro-1H-imidazole-4-carboxylique,
l'ester méthylique d'acide 1-butyl-5-[4-(1-diméthylamino-3-phényl-propyl)-cyclohexylméthyl}-4-(2-méthylsulfanyl-éthyl)-4,5-dihydro-1H-imidazole-4-carboxylique,
l'ester méthylique d'acide 5-{4-[diméthylamino-(3-fluorophényl)-méthyl]-cyclohexylméthyl}-1-phénéthyl-4-propyl-4,5-dihydro-1H-imidazole-4-carboxylique,
l'ester méthylique d'acide 5-{4-[diméthylamino-(3-fluorophényl)-méthyl]-cyclohexylméthyl}-4-(2-méthylsulfanyl-éthyl)-1-phénéthyl-4,5-dihydro-1H-imidazole-4-carboxylique,
l'ester méthylique d'acide 5-{4-[diméthylamino-(3-fluorophényl)-méthyl]-cyclohexylméthyl}-4-méthyl-1-phénéthyl-4,5-dihydro-1H-imidazole-4-carboxylique,
l'ester méthylique d'acide 5-{4-[diméthylamino-(4-fluorophényl)-méthyl]-cyclohexylméthyl}-1-phénéthyl-4-propyl-4,5-dihydro-1H-imidazole-4-carboxylique,
l'ester méthylique d'acide 5-{4-[diméthylamino-(4-fluorophényl)-méthyl]-cyclohexylméthyl}-4-(2-méthylsulfanyl-éthyl)-1-phénéthyl-4,5-dihydro-1H-imidazole-4-carboxylique,
l'ester méthylique d'acide 5-{4-[diméthylamino-(4-fluorophényl)-méthyl]-cyclohexylméthyl}-4-méthyl-1-phénéthyl-4,5-dihydro-1H-imidazole-4-carboxylique,
l'ester méthylique d'acide 5-[4-(1-diméthylamino-3-phényl-propyl)-cyclohexylméthyl]-1-phénéthyl-4-propyl-4,5-dihydro-1H-imidazole-4-carboxylique,
l'ester méthylique d'acide 5-[4-(1-diméthylamino-3-phényl-propyl)-cyclohexylméthyl]-4-(2-méthylsulfanyl-éthyl)-1-phénéthyl-4,5-dihydro-1H-imidazole-4-carboxylique,
l'ester méthylique d'acide 5-[4-(1-diméthylamino-3-phényl-propyl)-cyclohexylméthyl]-4-méthyl-1-phénéthyl-4,5-dihydro-1H-imidazole-4-carboxylique,
l'ester méthylique d'acide 1-benzyl-5-{4-[diméthylamino-(3-fluorophényl)-méthyl]-cyclohexylméthyl}-4-propyl-4,5-dihydro-1H-imidazole-4-carboxylique,
l'ester méthylique d'acide 1-benzyl-5-{4-[diméthylamino-(3-fluorophényl)-méthyl]-cyclohexylméthyl}-4-(2-méthylsulfanyl-éthyl)-4,5-dihydro-1H-imidazole-4-carboxylique,
l'ester méthylique d'acide 1-benzyl-5-{4-[diméthylamino-(3-fluorophényl)-méthyl]-cyclohexylméthyl}-4-méthyl-4,5-dihydro-1H-imidazole-4-carboxylique,
l'ester méthylique d'acide 1-benzyl-5-{4-[diméthylamino-(4-fluorophényl)-méthyl]-cyclohexylméthyl}-4-propyl-4,5-dihydro-1H-imidazole-4-carboxylique,
l'ester méthylique d'acide 1-benzyl-5-{4-[diméthylamino-(4-fluorophényl)-méthyl]-cyclohexylméthyl}-4-(2-méthylsulfanyl-éthyl)-4,5-dihydro-1H-imidazole-4-carboxylique,
l'ester méthylique d'acide 1-benzyl-5-{4-[diméthylamino-(4-fluorophényl)-méthyl]-cyclohexylméthyl}-4-méthyl-4,5-dihydro-1H-imidazole-4-carboxylique,
l'ester méthylique d'acide 1-benzyl-5-[4-(1-diméthylamino-3-phényl-propyl)-cyclohexylméthyl]-4-propyl-4,5-dihydro-1H-imidazole-4-carboxylique,
l'ester méthylique d'acide 1-benzyl-5-[4-(1-diméthylamino-3-phényl-propyl)-cyclohexylméthyl]-4-(2-méthylsulfanyl-éthyl)-4,5-dihydro-1H-imidazole-4-carboxylique,
l'ester méthylique d'acide 1-benzyl-5-[4-(1-diméthylamino-3-phényl-propyl-cyclohexylméthyl]-4-méthyl-4,5-dihydro-1H-imidazole-4-carboxylique,
l'ester méthylique d'acide 1-cyclohexyl-5-{4-[diméthylamino-(3-fluorophényl)-méthyl]-cyclohexylméthyl}-4-(2-méthoxycarbonyl-éthyl)-4,5-dihydro-1H-imidazole-4-carboxylique,
l'ester méthylique d'acide 4-sec.-butyl-1-cyclohexyl-5-{4-[diméthylamino-(3-fluorophényl)-méthyl]-cyclohexylméthyl}-4,5-dihydro-1H-imidazole-4-carboxylique,
l'ester méthylique d'acide 1-cyclohexyl-5-{4-[diméthylamino-(3-fluorophényl)-méthyl]-cyclohexylméthyl}-4-méthylsulfanylméthyl-4,5-dihydro-1H-imidazole-4-carboxylique,
l'ester méthylique d'acide 4-benzylsulfanylméthyl-1-cyclohexyl-5-{4-[diméthylamino-(3-fluorophényl)-méthyl]-cyclohexylméthyl}-4,5-dihydro-1H-imidazole-4-carboxylique,
l'ester méthylique d'acide 1-cyclohexyl-5-{4-[diméthylamino-(4-fluorophényl)-méthyl]-cyclohexylméthyl}-4-(2-méthoxycarbonyl-éthyl)-4,5-dihydro-1H-imidazole-4-carboxylique,
l'ester méthylique d'acide 4-sec.-butyl-1-cyclohexyl-5-{4-[diméthylamino-(4-fluorophényl)-méthyl]-cyclohexylméthyl}-4,5-dihydro-1H-imidazole-4-carboxylique,
l'ester méthylique d'acide 1-cyclohexyl-5-{4-[diméthylamino-(4-fluorophényl)-méthyl]-cyclohexylméthyl}-4-méthylsulfanylméthyl-4,5-dihydro-1H-imidazole-4-carboxylique,
l'ester méthylique d'acide 1-cyclohexyl-5-[4-(1-diméthyl-amino-3-phényl-propyl)-cyclohexylméthyl]-4-(2-méthoxycarbonyl-éthyl)-4,5-dihydro-1H-imidazole-4-carboxylique,
l'ester méthylique d'acide 1-cyclohexyl-5-[4-(1-diméthyl-amino-3-phényl-propyl)-cyclohexylméthyl]-4-méthylsulfanylméthyl-4,5-dihydro-1H-imidazole-4-carboxylique,
l'ester méthylique d'acide 4-benzylsulfanylméthyl-1-cyclohexyl-5-[4-(1-diméthylamino-3-phényl-propyl)-cyclohexyl-méthyl]-4,5-dihydro-1H-imidazole-4-carboxylique,
l'ester méthylique d'acide 5-{4-[diméthylamino-(3-fluorophényl)-méthyl]-cyclohexylméthyl}-4-(2-méthoxycarbonyl-éthyl)-1-propyl-4,5-dihydro-1H-imidazole-4-carboxylique,
l'ester méthylique d'acide 5-{4-[diméthylamino-(3-fluorophényl)-méthyl]-cyclohexylméthyl}-4-méthylsulfanylméthyl-1-propyl-4,5-dihydro-1H-imidazole-4-carboxylique,
l'ester méthylique d'acide 5-{4-[diméthylamino-(4-fluorophényl)-méthyl]-cyclohexylméthyl}-4-(2-méthoxycarbonyl-éthyl)-1-propyl-4,5-dihydro-1H-imidazole-4-carboxylique,
l'ester méthylique d'acide 5-{4-[diméthylamino-(4-fluorophényl)-méthyl]-cyclohexylméthyl}-4-méthylsulfanylméthyl-1-propyl-4,5-dihydro-1H-imidazole-4-carboxylique,
l'ester méthylique d'acide 4-benzylsulfanylméthyl-5-{4-[diméthylamino-(4-fluorophényl)-méthyl]-cyclohexylméthyl}-1-propyl-4,5-dihydro-1H-imidazole-4-carboxylique,
le 4-sec.-butyl-5-((4-((4-chlorophényl)(diméthylamino)-méthyl)cyclohexyl)méthyl)-1-cyclohexyl-4,5-dihydro-1H-imidazole-4-carboxylate de méthyle,
sous forme du racémate ; des énantiomères, des diastéréoisomères, de mélanges des énantiomères ou des diastéréoisomères ou d'un énantiomère ou diastéréoisomère individuel ; des bases et/ou de sels d'acides physiologiquement acceptables.

11. Procédé de production d'un dérivé d'imidazoline conforme à l'invention, dans lequel on fait réagir des amines de formule générale A avec des aldéhydes de formule générale B et des esters d'isonitrile de formule générale C dans un solvant organique, par exemple l'éthanol ou le méthanol, à une température de 20 à 100°C d'après le schéma de synthèse suivant pour obtenir des dérivés d'imidazoline substitués de formule générale I.

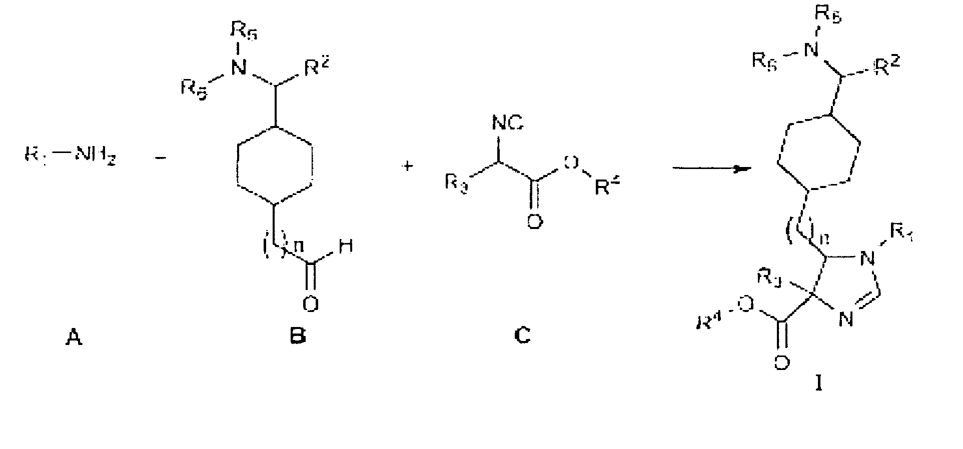

12. Aldéhyde substitué de formule générale B 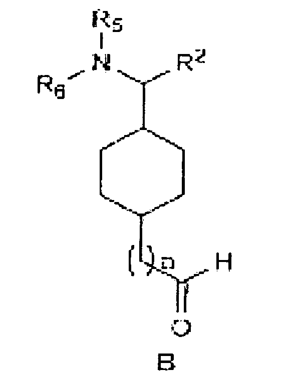 dans laquelle les restes R², R⁵, R⁶ ainsi que n ont la définition indiquée dans les revendications 1, 4, 5 et 9.

13. Aldéhydes substitués suivant la revendication 12, choisis dans le groupe
4-[diméthylamino-(4-fluorophényl)-méthyl]-cyclohexane-carbaldéhyde,
4-(1-diméthylamino-3-phényl-propyl)-cyclohexanecarbaldéhyde,
4-[diméthylamino-(3-fluorophényl)-méthyl]-cyclohexane-carbaldéhyde,
4-[(4-chlorophényl)-diméthylamino-méthyl]-cyclohexane-carbaldéhyde,
{4-[(4-chlorophényl)-diméthylamino-méthyl]-cyclohexyl}-acétaldéhyde,
{4-[diméthylamino-(3-fluorophényl)-méthyl]-cyclohexyl}-acétaldéhyde,
4-(diméthylamino-thiophène-2-yl-méthyl)cyclohexane-carbaldéhyde,
{4-[diméthylamino-(4-fluorophényl)-méthyl]-cyclohexyl}-acétaldéhyde,
[4-(diméthylamino-thiophène-2-yl-méthyl)cyclohexyl]-acétaldéhyde,
[4-(diméthylamino-3-phényl-propyl)-cyclohexyl]acétaldéhyde,
sous forme du racémate ; des énantiomères, des diastéréoisomères, de mélanges des énantiomères ou des diastéréoisomères ou d'un énantiomère ou diastéréoisomère individuel ; des bases et/ou de sels d'acides physiologiquement acceptables.

14. Médicament contenant au moins un dérivé d'imidazoline substitué suivant la revendication 1 ou un aldéhyde substitué suivant la revendication 12 le cas échéant sous forme du racémate ; des énantiomères, des diastéréoisomères, de mélanges des énantiomères ou des diastéréoisomères ou d'un énantiomère ou diastéréoisomère individuel ; des bases et/ou de sels d'acides physiologiquement acceptables, contenant de même le cas échéant des additifs et/ou des substances auxiliaires convenables et/ou éventuellement d'autres substances actives.

15. Utilisation d'un dérivé d'imidazoline substitué suivant la revendication 1 ou d'un aldéhyde substitué suivant la revendication 12, éventuellement sous forme du racémate ; des énantiomères, des diastéréoisomères, de mélanges des énantiomères ou diastéréoisomères ou d'un énantiomère ou diastéréoisomère individuel ; des bases et/ou de sels d'acides physiologiquement acceptables, pour la préparation d'un médicament destiné au traitement de la douleur, en particulier d'une douleur aiguë, neuropathique ou chronique.

16. Utilisation d'un dérivé d'imidazoline substitué suivant la revendication 1 ou d'un aldéhyde substitué suivant la revendication 12, éventuellement sous forme du racémate ; des énantiomères, des diastéréoisomères, de mélanges des énantiomères ou diastéréoisomères ou d'un énantiomère ou diastéréoisomère individuel ; des bases et/ou de sels d'acides physiologiquement acceptables, pour la préparation d'un médicament destiné au traitement de dépressions, de l'incontinence urinaire, de la diarrhée, du prurit, de l'abus d'alcool et de stupéfiants, de la pharmacodépendance, du manque de tonus et/ou en vue de l'anxiolyse.
